# EUROPEAN PATENT SPECIFICATION

(11) **EP 4 536 077 B1**
(45) Date of publication and mention of the grant of the patent: **18.11.2026**
(21) Application number: 23738977.0
(22) Date of filing: 09.06.2023
(51) Int. Cl.: A61B 5/145

(54) **APPARATUSES, SYSTEMS, AND METHODS OF CONTROLLING SENSOR DEPLOYMENT**
VORRICHTUNGEN, SYSTEME UND VERFAHREN ZUR STEUERUNG DES SENSOREINSATZES
APPAREILS, SYSTÈMES ET PROCÉDÉS DE COMMANDE DE DÉPLOIEMENT DE CAPTEUR

(30) Priority: 10.06.2022 US 202263351297 P
(43) Date of publication of application: 16.04.2025
(73) Proprietor: Dexcom, Inc., San Diego, CA 92121 (US)
(72) Inventor: COLLIGNON, Sean, San Diego, California 92121 (US); FALL, Scott, San Diego, California 92121 (US); HARPER, Eric G., San Diego, California 92121 (US); SHELVER, Christopher J., San Diego, California 92121 (US); HOANG, Nam Q., San Diego, California 92121 (US); SEIDEL, Jared Colin, San Diego, California 92121 (US); DURHAM, John, San Diego, California 92121 (US); BARRY, John C., San Diego, California 92121 (US); ROBINSON, Morgan Alexander, San Diego, California 92121 (US); WINDMILLER, Joshua, San Diego, California 92121 (US); ABDULLAYEV, Elshad, San Diego, California 92121 (US); PARNELL, Shane, Richard, San Diego, California 92121 (US); WANG, Shanger, San Diego, California 92121 (US)
(74) Representative: Hoffmann Eitle
(86) International application number: PCT/US2023/024974
(87) International publication number: WO 2023/239929

(56) References cited:
- WO-A1-2019/005627
- US-A1- 2009 187 091
- US-A1- 2012 253 145
- US-A1- 2014 031 655
- US-A1- 2016 338 734
- US-A1- 2017 303 831
- US-A1- 2019 223 767

## Description

### RELATED APPLICATION

This application claims the benefit of U.S. Provisional Patent Application No. 63/351,297, filed June 10, 2022.

### BACKGROUND OF THE INVENTION

### Field of the Invention

Medical device systems and methods. More particularly, apparatuses, systems, and methods are provided for controlling the deployment of a transcutaneous analyte sensor to the skin of a host.

### Description of the Related Technology

Diabetes mellitus is a disorder in which the pancreas cannot create sufficient insulin (Type 1 or insulin dependent) and/or in which insulin is not effective (Type 2 or non-insulin dependent). In the diabetic state, the victim suffers from high blood sugar, which can cause an array of physiological derangements associated with the deterioration of small blood vessels, for example, kidney failure, skin ulcers, or bleeding into the vitreous of the eye. A hypoglycemic reaction (low blood sugar) can be induced by an inadvertent overdose of insulin, or after a normal dose of insulin or glucose-lowering agent accompanied by extraordinary exercise or insufficient food intake.

Conventionally, a person with diabetes carries a self-monitoring blood glucose (SMBG) monitor, which typically requires uncomfortable finger pricking methods. Due to the lack of comfort and convenience, a person with diabetes normally only measures his or her glucose levels two to four times per day. Unfortunately, such time intervals are spread so far apart that the person with diabetes likely finds out too late of a hyperglycemic or hypoglycemic condition, sometimes incurring dangerous side effects. Glucose levels may be alternatively monitored continuously by a measurement system including an on-skin sensor assembly. The sensor assembly may have a wireless transmitter which transmits measurement data to a receiver which can process and display information based on the measurements.

The process of applying the sensor to the person is important for such a system to be effective and user friendly. The application process should result in the on-skin sensor assembly being attached to the person in a state where it is capable of sensing the analyte (e.g., glucose) level information, communicating the sensed data to the transmitter, and transmitting the analyte level information to the receiver.

Exemplary systems are disclosed in, e.g., U.S. Patent Publication No. 2014/0088389, U.S. Patent Publication No. 2013/0267813, and U.S. Patent Publication No. 2018/0368771, owned by the assignee of the present application.

US 2014/031655 A1 relates to a sensor insertion assembly including an introducer including a shaft portion comprising a channel, the shaft portion including a holding member disposed along a length of the channel, the holding member configured to substantially releasably retain a sensor.

This Background is provided to introduce a brief context for the Summary and Detailed Description that follow. This Background is not intended to be an aid in determining the scope of the claimed subject matter nor be viewed as limiting the claimed subject matter to implementations that solve any or all of the disadvantages or problems presented above.

### SUMMARY

The present systems and methods relate to apparatuses, systems, and methods for medical devices. More particularly, apparatuses, systems, and methods are provided for deploying a transcutaneous analyte sensor to the skin of a host. The apparatuses, systems, and methods are configured for reducing friction between a sensor and an insertion element and/or for controlling sensor deployment. The various examples of the present apparatuses, systems, and methods may have several features, no single one of which is solely responsible for their desirable attributes. Without limiting the scope of the present examples as expressed by the claims that follow, their more prominent features now will be discussed briefly. After considering this discussion, and particularly after reading the section entitled "Detailed Description," one will understand how the features of the present examples provide the advantages described herein.

What is claimed is a medical device system comprising the features of claim 1. Optional features of the claimed medical device system are set forth in the dependent claims.

In this specification, the following non-SI units are used, which may be converted to the respective SI or metric units according to the following conversion:
1 lbf (pound of force) = 4.448 N (Newton)
1 in (inch) = 2.54 cm (centimeter).

In a first aspect, a medical device system comprising: a housing configured to be worn on skin of a host and including a distal surface for facing towards the skin and a proximal surface facing opposite the distal surface, the housing including an opening for an insertion element to be retracted proximally through from the skin; an analyte sensor having a first portion coupled to the housing and a second portion configured to extend distally from the housing and be guided by the insertion element into the skin of the host; and a stopper body configured to impede the analyte sensor from retracting proximally through the opening upon the insertion element retracting proximally through the opening.

Implementations of the embodiments may include one or more of the following. The analyte sensor may include a bend positioned between the first portion and the second portion, the bend being axially aligned with the opening. The second portion may be straight and is axially aligned with the opening. The stopper body may be configured to contact the analyte sensor to impede the analyte sensor from retracting proximally upon the insertion element retracting proximally through the opening. The stopper body may be positioned proximate the opening. The stopper body may comprise a tab extending into the opening. The first portion of the analyte sensor may be positioned within a cavity and the tab extends from the cavity into the opening. The stopper body may be integral with the housing. The stopper body may comprise a plug positioned within the opening. The plug may comprise a gasket. The plug may have a chamfer. The plug may be pierceable by the insertion element. The stopper body may be positioned proximal of the analyte sensor. The system may further comprise the insertion element, wherein the insertion element includes a channel for receiving the analyte sensor. The insertion element may comprise a needle. A needle hub may be positioned at a proximal portion of the needle, and wherein the stopper body is positioned between the needle hub and the analyte sensor. The insertion element may be positioned within the opening of the housing and extends parallel with the second portion of the analyte sensor. The stopper body may surround the insertion element. The stopper body may be in contact with the insertion element. The analyte sensor may comprise a transcutaneous analyte sensor.

In a second aspect, a medical device system comprising: a housing configured to be worn on skin of a host and including a distal surface for facing towards the skin and a proximal surface facing opposite the distal surface; an elongate analyte sensor coupled to the housing and configured to extend distally from the housing and be positioned in the skin of the host; an elongate insertion element including a shaft configured to extend along a portion of the elongate analyte sensor and configured to guide the elongate analyte sensor into the skin of the host; and a spacer body configured to be positioned between the portion of the elongate analyte sensor and the shaft and space the portion of the elongate analyte sensor from the shaft

Implementations of the embodiments may include one or more of the following. The spacer body may be removable from between the portion of the elongate analyte sensor and the shaft. The spacer body may be manually removable from between the portion of the elongate analyte sensor and the shaft. The system may further comprise a tether coupled to the spacer body and configured to be pulled to remove the spacer body from between the portion of the elongate analyte sensor and the shaft. The tether and the spacer body may be formed from a single piece of material. The tether may comprise a pull tab for a user to pull. The spacer body may include a sheath surrounding the elongate insertion element. The sheath may include a channel that the elongate analyte sensor is positioned within. A cover may cover the distal surface of the housing and coupled to the spacer body. The system may further comprise an applicator housing configured to retain the housing and including a proximal end and a distal opening for the housing to be deployed to the skin from, and wherein the cover comprises a cap positioned at the distal opening. The system may further comprise a tether coupled to the spacer body and coupled to the cap. Removal of the cap may pull the tether coupled to the spacer body to remove the spacer body from between the portion of the elongate analyte sensor and the shaft. The system may further comprise an adhesive patch positioned at the distal surface of the housing, and wherein the cover comprises a liner cover for the adhesive patch. The spacer body may comprise a thermally expandable metal. The elongate insertion element may have a first coefficient of thermal expansion and the spacer body has a second coefficient of thermal expansion that is different than the first coefficient of thermal expansion. The spacer body may be compressible. The elongate insertion element includes a channel for receiving the portion of the elongate analyte sensor. The spacer body may be removable and configured to be removed to seat the portion of the elongate analyte sensor into the channel. The elongate insertion element may comprise a needle. The system may further comprise a needle hub positioned at a proximal portion of the needle, wherein the spacer body is positioned on the needle hub.

In a third aspect, a medical device system comprising: a housing configured to be worn on skin of a host and including a distal surface for facing towards the skin and a proximal surface facing opposite the distal surface; an elongate analyte sensor coupled to the housing and configured to extend distally from the housing and be positioned in the skin of the host; an elongate insertion element including a shaft configured to extend along a portion of the elongate analyte sensor and be inserted into the skin to guide the elongate analyte sensor into the skin of the host and configured to be retracted from the skin; and a displacement mechanism configured to displace the portion of the elongate analyte sensor relative to the elongate insertion element prior to retraction of the shaft from the skin to reduce stiction between the elongate analyte sensor and the shaft.

Implementations of the embodiments may include one or more of the following. The displacement mechanism may be configured to slide the portion of the elongate analyte sensor relative to the shaft prior to retraction of the shaft from the skin to reduce stiction between the elongate analyte sensor and the shaft. The system may further comprise a hub positioned at a proximal portion of the elongate insertion element, and wherein the displacement mechanism includes a compressible body positioned between the hub and the proximal surface of the housing. The displacement mechanism may include a compressible body protruding distally from the distal surface of the housing. The displacement mechanism may be configured to vibrate one or more of the elongate insertion element or the portion of the elongate analyte sensor prior to retraction of the shaft from the skin to reduce stiction between the elongate analyte sensor and the shaft. The system may include an insertion assembly for inserting the shaft of the elongate insertion element into the skin, wherein the insertion assembly includes the displacement mechanism. The displacement mechanism may include a cover covering the distal surface of the housing. The system may further comprise an applicator housing configured to retain the housing and including a proximal end and a distal opening for the housing to be deployed to the skin from, and wherein the cover comprises a cap positioned at the distal opening. The cap may include a cam surface for applying a force to the housing to displace the portion of the elongate analyte sensor relative to the elongate insertion element. The system may further comprise an adhesive patch positioned at the distal surface of the housing, and wherein the cover comprises a liner cover for the adhesive patch.

In a fourth aspect, a medical device system comprising: a housing configured to be worn on skin of a host and including a distal surface for facing towards the skin and a proximal surface facing opposite the distal surface; an elongate analyte sensor coupled to the housing and configured to extend distally from the housing and be positioned in the skin of the host; and an elongate insertion element including a shaft configured to extend along a portion of the elongate analyte sensor and be inserted into the skin to guide the elongate analyte sensor into the skin of the host, the shaft including a surface configured to reduce friction with the portion of the elongate analyte sensor.

Implementations of the embodiments may include one or more of the following. The surface may be configured to reduce stiction with the portion of the elongate analyte sensor. The surface may include a surface texture. The surface may include a surface roughness of 35 root mean square (RMS) microinches or greater. The surface may include one or more of bumps, holes, or grooves. According to the present invention, the surface includes a coating configured to reduce friction with the portion of the elongate analyte sensor. The coating may comprise a lubricant. The coating may comprise one or more of a spray coating, brush coating, electrostatically applied coating, or more preferably a plating, a dip coating, or a deposition. The coating may comprise a polymer. The coating may comprise a thermal oxide. The coating may comprise an inert material. The coating may be bonded to the shaft. The coating may have a thickness upon the shaft of less than 1.5 micrometers. The coating may be cured, i.e. via addition curing, condensation curing, thermal curing, etc. The coating may include silicone. The silicone may comprise an aminofunctional dimethylsiloxane copolymer. The surface may be configured to reduce hydrogen bonding with the portion of the elongate analyte sensor. The elongate insertion element may comprise a needle. The elongate insertion element may include a channel for receiving the portion of the elongate analyte sensor. The channel may have a C-shaped cross-section.

In a fifth aspect, a medical device system comprising: a housing configured to be worn on skin of a host and including a distal surface for facing towards the skin and a proximal surface facing opposite the distal surface; an elongate analyte sensor coupled to the housing and configured to extend distally from the housing and be positioned in the skin of the host; and an elongate insertion element including a shaft configured to extend along a portion of the elongate analyte sensor and be inserted into the skin to guide the elongate analyte sensor into the skin of the host, the shaft having a V-shaped or a W-shaped cross-sectional channel for receiving the portion of the elongate analyte sensor.

Implementations of the embodiments may include one or more of the following. The V-shaped cross-sectional channel may have an angle of between 60 degrees and 120 degrees. The V-shaped cross-sectional channel may have an angle of 90 degrees. The W-shaped cross-sectional channel may be formed by an elongate protrusion added to a central portion of an elongate insertion element having a C-shaped cross-sectional channel. An outer surface of the elongate analyte sensor may have a circular shaped cross-section.

In a sixth aspect, a medical device system comprising: a housing configured to be worn on skin of a host and including a distal surface for facing towards the skin and a proximal surface facing opposite the distal surface; an elongate insertion element including a shaft configured to be inserted into the skin; and an elongate analyte sensor coupled to the housing and configured to extend along the elongate insertion element and be guided into the skin by the elongate insertion element, the elongate analyte sensor including a surface configured to reduce friction with the elongate insertion element.

Implementations of the embodiments may include one or more of the following. The surface may be configured to reduce stiction with the elongate analyte sensor. The surface may be configured to reduce hydrogen bonding with the elongate insertion element. The elongate insertion element may comprise a needle. The elongate insertion element may include a channel for receiving a portion of the elongate analyte sensor.

In a seventh aspect, a medical device system comprising: a housing configured to be worn on skin of a host and including a distal surface for facing towards the skin and a proximal surface facing opposite the distal surface; an elongate insertion element including a shaft configured to be inserted into the skin; and an elongate analyte sensor coupled to the housing and configured to extend along the elongate insertion element and be guided into the skin by the elongate insertion element, the elongate analyte sensor having a cross-section with an oval shape.

Implementations of the embodiments may include one or more of the following. An outer surface of the elongate analyte sensor may be configured to reduce stiction with the elongate insertion element. The elongate insertion element may comprise a needle. The elongate insertion element may include a channel for receiving a portion of the elongate analyte sensor. The channel may have a C-shaped cross-section.

In an eighth aspect, a medical device system comprising: a housing configured to be worn on skin of a host and including a distal surface for facing towards the skin and a proximal surface facing opposite the distal surface; and an analyte sensor having a first portion coupled to the housing and a second portion configured to extend distally from the housing and be inserted into the skin of the host, the analyte sensor including a bend having at least two kinks that angle the second portion from the first portion.

Implementations of the embodiments may include one or more of the following. The second portion may extend perpendicular from the distal surface of the housing. The first portion may extend parallel with the distal surface of the housing. The at least two kinks may angle the second portion to be perpendicular from the first portion. The at least two kinks may include a first kink and a second kink, the first kink having an angle of less than ninety degrees and the second kink having an angle of less than ninety degrees.

In a ninth aspect, a medical device system comprising: a housing configured to be worn on skin of a host and including a distal surface for facing towards the skin and a proximal surface facing opposite the distal surface; an analyte sensor coupled to the housing and configured to extend distally from the housing and be positioned in the skin of the host; an insertion element configured to extend along the analyte sensor and guide the analyte sensor into the skin of the host; an insertion assembly configured to drive the insertion element into the skin of the host; and a force channeling component configured to channel a force from the insertion assembly proximate the insertion element.

Implementations of the embodiments may include one or more of the following. The insertion assembly may include a plate configured to be positioned proximal of the proximal surface, and the force channeling component comprises one or more protrusions on the plate configured to channel the force proximate the insertion element. The one or more protrusions may be configured to apply a force to the proximal surface of the housing proximate the insertion element. The housing may include an opening for the insertion element to be retracted proximally through from the skin, and the force channeling component is configured to contact the proximal surface of the housing proximate the opening. The insertion element may comprise a needle.

In a tenth aspect, a medical device system comprising: a housing configured to be worn on skin of a host and including a distal surface for facing towards the skin and a proximal surface facing opposite the distal surface; an elongate analyte sensor coupled to the housing and configured to extend distally from the housing and be positioned in the skin of the host, the elongate analyte sensor having a flexural modulus of greater than 8 giga Pascals; and an elongate insertion element including a shaft configured to extend along a portion of the elongate analyte sensor and be inserted into the skin to guide the portion of the elongate analyte sensor into the skin of the host.

Implementations of the embodiments may include one or more of the following. The flexural modulus may be greater than 8.4 giga Pascals. The elongate analyte sensor may include a first portion coupled to the housing and a second portion extending distally from the distal surface of the housing, the second portion having the flexural modulus of greater than 8 giga Pascals. The shaft may include a channel configured to receive the portion of the elongate analyte sensor. The elongate insertion element may comprise a needle.

In an eleventh aspect, a medical device system comprising: a housing configured to be worn on skin of a host and including a distal surface for facing towards the skin and a proximal surface facing opposite the distal surface; an elongate analyte sensor coupled to the housing and configured to extend distally from the housing and be positioned in the skin of the host; and an elongate insertion element including a shaft having a channel that a portion of the elongate analyte sensor is positioned in, the shaft configured to be inserted into the skin to guide the portion of the elongate analyte sensor into the skin, and the shaft having a diametrical clearance from the portion of the elongate analyte sensor of at least 0.07 millimeters.

Implementations of the embodiments may include one or more of the following. The diametrical clearance may be at least 0.10 millimeters. The elongate insertion element may comprise a needle. The channel may have a C-shaped cross-section. The elongate analyte sensor may have a first portion coupled to the housing and a second portion extending distally from the distal surface of the housing and positioned within the channel.

In a twelfth aspect, a medical device system comprising: a housing configured to be worn on skin of a host and including a distal surface for facing towards the skin and a proximal surface facing opposite the distal surface; an elongate analyte sensor having a first portion coupled to the housing and a second portion configured to extend distally from the housing and be positioned in the skin of the host, the second portion having a diameter; and an elongate insertion element including a shaft having an opening for a channel that the second portion of the elongate analyte sensor is positioned in, the shaft configured to be inserted into the skin to guide the second portion into the skin, and the channel at the opening having a width, and wherein a ratio of the diameter to the width is less than 0.9.

Implementations of the embodiments may include one or more of the following. The ratio of the diameter to the width may be less than 0.8. The ratio of the diameter to the width may be less than 0.7. The channel may have a C-shaped cross-section. The elongate insertion element may comprise a needle.

In a thirteenth aspect, a method comprising: reducing friction between an analyte sensor and an insertion element during or following a sterilization process being performed to the analyte sensor and the insertion element and prior to retraction of the insertion element from skin of a host, wherein the insertion element is configured to guide the analyte sensor into the skin of the host and be retracted from the skin of the host.

Implementations of the embodiments may include one or more of the following. The method may further comprise vibrating the analyte sensor and the insertion element to reduce the friction between the analyte sensor and the insertion element. The method may further comprise increasing an ambient temperature or decreasing the ambient temperature to reduce the friction between the analyte sensor and the insertion element. The method may further comprise decreasing the ambient humidity to reduce the friction between the analyte sensor and the insertion element. The method may further comprise packaging the analyte sensor and the insertion element with desiccant. The friction may comprise stiction. The insertion element may comprise an elongate insertion element including a shaft configured to be inserted into the skin, and the analyte sensor comprises an elongate analyte sensor having a portion extending along the shaft of the elongate insertion element. The shaft may include a channel and the portion of the elongate analyte sensor is positioned within the channel. The method may further comprise reducing hydrogen bonds between the analyte sensor and the insertion element. The insertion element may comprise a needle. The analyte sensor may be coupled to a housing that is configured to be worn on skin of a host, the housing including an opening that the insertion element passes through. The analyte sensor may include a first portion that is coupled to the housing and a second portion that extends distally from a distal surface of the housing. The housing, the analyte sensor, and the insertion element may be positioned within an applicator housing. The sterilization process may include applying a sterilizing gas to the analyte sensor and the insertion element. The sterilization process may include an ethylene oxide sterilization process.

In a founeenth aspect, a method comprising: coating at least a portion of a shaft of an elongate insertion element with a material, the elongate insertion element being for guiding an elongate analyte sensor into skin of a host upon insertion into the skin with the elongate analyte sensor extending along a portion of the shaft, the material being configured to reduce friction between the elongate insertion element and the elongate analyte sensor.

Implementations of the embodiments may include one or more of the following. The method may include positioning the elongate insertion element adjacent to the elongate analyte sensor. The method may include positioning the elongate analyte sensor within a channel of the elongate insertion element. The elongate analyte sensor may extend distally from a housing that is configured to be worn on the skin of the host. The material may be configured to reduce stiction with the elongate analyte sensor. The coating may comprise one or more of a plating, a dip coating, or a deposition. The coating may be bonded to the shaft. The coating may have a thickness upon the shaft of less than 1.5 micrometers. The method may include curing the coating upon the shaft. The coating may include silicone. The silicone may comprise an aminofunctional dimethylsiloxane copolymer. The method may include positioning the shaft within a solution of the material. The solution may include a solvent. The material may produce a friction coefficient for the portion of the shaft that is more than ten times lower than a friction coefficient of a surface of the portion of the shaft coated with the material. The elongate insertion element may comprise a needle.

In a fifteenth aspect, a medical device system comprising: a housing configured to be worn on skin of a host and including a distal surface for facing towards the skin and a proximal surface facing opposite the distal surface; an elongate analyte sensor coupled to the housing and configured to extend distally from the housing and be positioned in the skin of the host; and one or more elongate insertion elements each including a shaft configured to extend along a portion of the elongate analyte sensor, each of the one or more elongate insertion elements configured to guide the elongate analyte sensor into the skin of the host with the elongate analyte sensor positioned external to the shaft of the respective elongate insertion element.

Implementations of the embodiments may include one or more of the following. The elongate analyte sensor may include a central axis, and each of the one or more elongate insertion elements includes a respective central axis, the central axis of the elongate analyte sensor configured to be parallel and laterally spaced apart from the respective central axes of the one or more elongate insertion elements. At least a portion of each of the one or more elongate insertion elements may include a convex outer surface configured to extend parallel and adjacent to an outer surface of the elongate analyte sensor. Each of the one or more elongate insertion elements may include an outer surface having a longitudinally extending segment configured to extend parallel and adjacent to an outer surface of the elongate analyte sensor. Each of the one or more elongate insertion elements may lack a channel for retaining an elongate analyte sensor. Each of the one or more elongate insertion elements may include an outer surface configured to contact an outer surface of the elongate analyte sensor in a deployment configuration. The one or more elongate insertion elements may include at least two of the elongate insertion elements. The at least two elongate insertion elements may each include an outer surface configured to contact an outer surface of the elongate analyte sensor in a deployment configuration. The at least two elongate insertion elements may be configured to be positioned on opposite sides of the elongate analyte sensor in a deployment configuration. The elongate analyte sensor may be configured to be positioned between the at least two elongate insertion elements in a deployment configuration. Each of the at least two elongate insertion elements may include a proximal end portion and a tip, and further comprising a needle hub coupled to the respective proximal end portions of the at least two elongate insertion elements. The shaft of the respective at least two elongate insertion elements may extend from the proximal end portion to the respective tip, and the tips of the at least two elongate insertion elements are unconnected to each other. The at least two elongate insertion elements may include a first elongate insertion element and a second elongate insertion element, the first elongate insertion element having a first outer surface and the second elongate insertion element having a second outer surface that extends parallel with the first outer surface and is laterally spaced from the first outer surface. The at least two elongate insertion elements may include a first elongate insertion element and a second elongate insertion element, the first elongate insertion element having a first outer surface and the second elongate insertion element having a second outer surface that extends parallel with the first outer surface and is in contact with the first outer surface. The one or more elongate insertion elements may include at least three of the elongate insertion elements. The elongate analyte sensor may be a first elongate analyte sensor, and further comprising a second elongate analyte sensor coupled to the housing and configured to extend distally from the housing and be positioned in the skin of the host; and the one or more elongate insertion elements each include a shaft configured to extend along a portion of the second elongate analyte sensor, each of the one or more elongate insertion elements configured to guide the second elongate analyte sensor into the skin of the host with the second elongate analyte sensor positioned external to the shaft of the respective elongate insertion element. At least one of the one or more elongate insertion elements may have an oval cross section. The elongate analyte sensor may include a distal tip, and the one or more elongate insertion elements include a distal tip configured to extend radially over at least a portion of the distal tip of the elongate analyte sensor. The distal tip of the one or more elongate insertion elements may have a diameter that is greater than a diameter of the respective shaft of the one or more elongate insertion elements. The system may include a rotation mechanism for rotating the distal tip of the one or more elongate insertion elements to uncover the portion of the distal tip of the elongate analyte sensor.

Any of the features of an embodiment of any of the aspects, including but not limited to any embodiments of any of the first through fifteenth aspects referred to above, is applicable to all other aspects and embodiments identified herein, including but not limited to any embodiments of any of the first through fifteenth aspects referred to above. Moreover, any of the features of an embodiment of the various aspects, including but not limited to any embodiments of any of the first through fifteenth aspects referred to above, is independently combinable, partly or wholly with other embodiments described herein in any way, e.g., one, two, or three or more embodiments may be combinable in whole or in part. Further, any of the features of an embodiment of the various aspects, including but not limited to any embodiments of any of the first through fifteenth aspects referred to above, may be made optional to other aspects or embodiments. Any aspect or embodiment of a method can be performed by a system or apparatus of another aspect or embodiment, and any aspect or embodiment of a system or apparatus can be configured to perform a method of another aspect or embodiment, including but not limited to any embodiments of any of the first through fifteenth aspects referred to above.

This Summary is provided to introduce a selection of concepts in a simplified form. The concepts are further described in the Detailed Description section. Elements or steps other than those described in this Summary are possible, and no element or step is necessarily required. This Summary is not intended to identify key features or essential features of the claimed subject matter, nor is it intended for use as an aid in determining the scope of the claimed subject matter. The claimed subject matter is not limited to implementations that solve any or all disadvantages noted in any part of this disclosure.

### BRIEF DESCRIPTION OF THE DRAWINGS

These and other features, aspects, and advantages are described below with reference to the drawings, which are intended to illustrate, but not to limit, the disclosure. In the drawings, like reference characters denote corresponding features consistently throughout similar examples.
FIG. 1 illustrates a schematic view of a continuous analyte sensor system.
FIG. 2A illustrates a top perspective assembly view of an on-skin sensor assembly.
FIG. 2B illustrates a bottom perspective view of the on-skin sensor assembly of FIG. 2A in an assembled state.
FIG. 2C illustrates a top perspective view of the on-skin sensor assembly of FIG. 2A in an assembled state.
FIG. 3 illustrates a perspective assembly view of an on-skin sensor assembly.
FIG. 4 illustrates a perspective view of an on-skin sensor assembly.
FIG. 5 illustrates a perspective view of an applicator system for an on-skin sensor assembly of an analyte sensor system.
FIG. 6 illustrates an exploded perspective view of the applicator system of FIG. 5.
FIGS. 7-9 illustrate several cross-sectional views of the applicator system of FIGS. 5 and 6, taken along the section line A-A' of FIG. 5, during operation.
FIGS. 10-12 illustrate several cross-sectional views of the applicator system of FIGS. 5 and 6, taken along the section line B-B' of FIG. 5, during operation.
FIGS. 13 and 14 illustrate magnified views of some features of the applicator system of FIGS. 5 and 6.
FIGS. 15 and 16 illustrate magnified views of some features of the applicator system of FIGS. 5 and 6.
FIG. 17 illustrates a perspective partial cutaway view of the needle carrier assembly, hub, and on-skin sensor assembly of the applicator system of FIGS. 5 and 6.
FIG. 18 illustrates a cross-sectional view of the hub and on-skin sensor assembly of the applicator system of FIGS. 5 and 6.
FIG. 19 illustrates a top view of a portion of the needle carrier assembly and hub of FIGS. 5 and 6.
FIGS. 20A and 20B illustrate perspective views of locking features for needles for use in an applicator for an analyte sensor system.
FIGS. 21-23 illustrate several cross-sectional views, and various features and operating positions, of yet another applicator for an on-skin sensor assembly of an analyte sensor system.
FIG. 24 illustrates a perspective view of various features of the applicator system of FIGS. 21-23.
FIG. 25 illustrates a cross-sectional view of a system, according to some examples.
FIGS. 26A-26B illustrate cross-sectional schematic views of an analyte sensor with and without an insertion element.
FIG. 27A illustrates a cross-sectional view of a housing of an on-skin sensor assembly.
FIG. 27B illustrates a cross-sectional view of the housing shown in FIG. 27A with the insertion element retracted.
FIG. 28 illustrates a top cross-sectional view of an analyte sensor within a channel of an insertion element.
FIG. 29 illustrates a cross-sectional view of a system prior to application to the skin of a host.
FIG. 30A illustrates a cross-sectional view of an analyte sensor within a channel of an insertion element.
FIG. 30B illustrates a cross-sectional view of the analyte sensor and insertion element shown in FIG. 30A, with a spacer body expanded.
FIG. 30C illustrates a cross-sectional view of the analyte sensor and insertion element shown in FIG. 30B, with the spacer body decreased in size.
FIG. 31 illustrates a side view of a spacer body positioned between an analyte sensor and an insertion element.
FIG. 32 illustrates a front view of the spacer body shown in FIG. 31 and a schematic cross-sectional view of a cap.
FIG. 33 illustrates a side view of a spacer body positioned between an analyte sensor and an insertion element, with the spacer body coupled to a liner removal component.
FIG. 34A illustrates a top perspective cross-sectional view of a spacer body comprising a sheath.
FIG. 34B illustrates a bottom perspective view of the spacer body shown in FIG. 34A.
FIG. 34C illustrates a bottom view of the spacer body shown in FIG. 34B.
FIG. 35A illustrates a cross-sectional view of a spacer body and a housing of an on-skin sensor assembly.
FIG. 35B illustrates a cross-sectional view of a spacer body and a housing of an on-skin sensor assembly.
FIG. 36 illustrates a cross-sectional view of a housing of an on-skin sensor assembly within an applicator housing.
FIG. 37A illustrates a perspective view of a stopper body including a flat face.
FIG. 37B illustrates a perspective view of a stopper body including a projected face.
FIG. 38 illustrates a perspective view of a stopper body positioned between a hub and an analyte sensor.
FIG. 39 illustrates a bottom view of the stopper body that is shown in FIG. 36.
FIG. 40 illustrates a cross-sectional view of a housing of an on-skin sensor assembly.
FIG. 41 illustrates a cross-sectional view of a housing of an on-skin sensor assembly.
FIG. 42 illustrates a cross-sectional view of a housing of an on-skin sensor assembly.
FIG. 43 illustrates a cross-sectional view of a housing of an on-skin sensor assembly.
FIG. 44 illustrates a cross-sectional view of a housing of an on-skin sensor assembly.
FIG. 45 illustrates a side view of a displacement mechanism between a hub and a housing of an on-skin sensor assembly.
FIG. 46 illustrates a top view of the displacement mechanism shown in FIG. 45.
FIG. 47 illustrates a side view representation of an analyte sensor displacing relative to an insertion element.
FIG. 48 illustrates a cross-sectional view of a housing of an on-skin sensor assembly including a displacement mechanism.
FIG. 49 illustrates a cross-sectional view of the housing of the on-skin sensor assembly shown in FIG. 48.
FIG. 50 illustrates a cross-sectional view of a displacement mechanism.
FIG. 51 illustrates a cross-sectional view of a liner removal component including a displacement mechanism.
FIG. 52 illustrates a perspective view of a cap including a displacement mechanism.
FIG. 53 illustrates a cross-sectional view of the cap shown in FIG. 52 and an on-skin sensor assembly.
FIG. 54 illustrates a cross-sectional view of the cap shown in FIG. 53 rotated from the position shown in FIG. 53.
FIG. 55 illustrates a bottom perspective view of a force channeling component of an insertion assembly.
FIG. 56 illustrates a cross-sectional view of an analyte sensor within a channel of an insertion element.
FIG. 57 illustrates a rotated, top cross-sectional view of the analyte sensor within the channel of the insertion element along line C-C' of FIG. 56.
FIG. 58 illustrates a cross-sectional view of a housing of an on-skin sensor assembly.
FIG. 59 illustrates a cross-sectional view of an analyte sensor within a channel of an insertion element.
FIG. 60 illustrates a cross-sectional view of an analyte sensor within a channel of an insertion element.
FIG. 61 illustrates a front view of a channel of an insertion element including a surface texture.
FIG. 62 illustrates a front view of a channel of an insertion element including grooves.
FIG. 63 illustrates a front view of a channel of an insertion element including holes.
FIG. 64 illustrates a top cross-sectional view of an analyte sensor within a channel of an insertion element.
FIG. 65 illustrates a top cross-sectional view of an analyte sensor within a channel of an insertion element.
FIG. 66 illustrates a top cross-sectional view of an analyte sensor within a channel of an insertion element.
FIG. 67 illustrates a side cross-sectional schematic view of an insertion element partially withdrawn from a chemical bath.
FIG. 68 illustrates a side view of an insertion element withdrawn from a chemical bath.
FIG. 69 illustrates a side cross sectional view of a coating upon an insertion element.
FIG. 70 illustrates a top cross sectional view of a coating upon an insertion element.
FIG. 71 illustrates a side perspective view of a plurality of insertion elements adjacent to an analyte sensor.
FIG. 72 illustrates a top cross sectional view of the plurality of insertion elements adjacent to an analyte sensor in the position shown in FIG. 71.
FIG. 73 illustrates a side perspective view of a plurality of insertion elements adjacent to an analyte sensor.
FIG. 74 illustrates a side perspective view of a plurality of insertion elements guiding an analyte sensor into skin of a host.
FIG. 75 illustrates a side perspective view of a plurality of insertion elements retracting from skin of a host.
FIG. 76 illustrates a top cross sectional view of a plurality of insertion elements adjacent to an analyte sensor.
FIG. 77 illustrates a top cross sectional view of a plurality of insertion elements adjacent to an analyte sensor.
FIG. 78 illustrates a top cross sectional view of a plurality of insertion elements adjacent to an analyte sensor.
FIG. 79 illustrates a top cross sectional view of a plurality of insertion elements adjacent to an analyte sensor.
FIG. 80 illustrates a top cross sectional view of a plurality of insertion elements adjacent to an analyte sensor.
FIG. 81 illustrates a top cross sectional view of a plurality of insertion elements adjacent to a plurality of analyte sensors.
FIG. 82 illustrates a top cross sectional view of a plurality of insertion elements adjacent to a plurality of analyte sensors.
FIG. 83 illustrates a top cross sectional view of a plurality of insertion elements adjacent to a plurality of analyte sensors.
FIG. 84 illustrates a top cross sectional view of a plurality of insertion elements adjacent to a plurality of analyte sensors.
FIG. 85 illustrates a top cross sectional view of a plurality of insertion elements adjacent to a plurality of analyte sensors.
FIG. 86 illustrates a side partial cross sectional view of an insertion element adjacent to an analyte sensor.
FIG. 87 illustrates a perspective view of the insertion element adjacent to the analyte sensor of FIG. 86.
FIG. 88 illustrates a side view of an insertion element guiding an analyte sensor into skin of a host.
FIG. 89 illustrates a side view of an insertion element retracting from skin of a host.

### DETAILED DESCRIPTION

The following description illustrates some examples of the disclosure in detail. Those of skill in the art will recognize that there are numerous variations and modifications of the disclosure that are encompassed by its scope. Accordingly, the description of a certain example should not be deemed to limit the scope of the present disclosure.

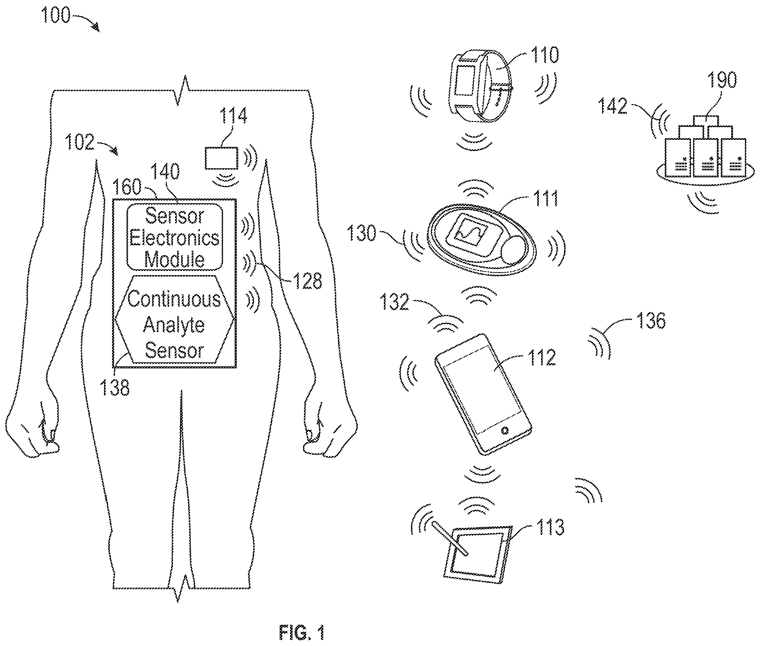

FIG. 1 is a diagram depicting an example medical device system according to examples herein. The medical device system in examples may comprise a continuous analyte monitoring system 100. The continuous analyte monitoring system 100 may include an analyte sensor system 102 comprising an on-skin sensor assembly 160 configured to be fastened to the skin of a host via a base (not shown).

In examples, other forms of medical device systems may be utilized, including other forms of monitoring systems, medicament delivery systems, or other therapeutic systems. In examples, an on-skin wearable medical device may be utilized that may comprise an on-skin sensor assembly, or a medicament delivery medical device, among other forms of on-skin wearable medical devices.

As shown in FIG. 1, the analyte sensor system 102 may be operatively connected to a host and a plurality of display devices 110-114 according to certain aspects of the present disclosure. Example display devices 110-114 may include computers such as smartphones, smartwatches, tablet computers, laptop computers, and desktop computers. In some examples, display devices 110-114 may be Apple Watches, iPhones, and iPads made by Apple Inc., or iOS, Windows, or Android operating system devices. It should be noted that display device 114 alternatively or in addition to being a display device, may be a medicament delivery device that can act cooperatively with analyte sensor system 102 to deliver medicaments to the host. Analyte sensor system 102 may include a sensor electronics module 140 and a continuous analyte sensor 138 associated with sensor electronics module 140. Sensor electronics module 140 may be in direct wireless communication with one or more of the plurality of display devices 110-114 via wireless communications signals. As will be discussed in greater detail below, display devices 110-114 may also communicate amongst each other and/or through each other to analyte sensor system 102. For ease of reference, wireless communications signals from analyte sensor system 102 to display devices 110-114 can be referred to as "uplink" signals 128. Wireless communications signals from, e.g., display devices 110-114 to analyte sensor system 102 can be referred to as "downlink" signals 130. Wireless communication signals between two or more of display devices 110-114 may be referred to as "crosslink" signals 132. Additionally, wireless communication signals can include data transmitted by one or more of display devices 110-113 via "long-range" uplink signals 136 (e.g., cellular signals) to one or more remote servers 190 or network entities, such as cloud-based servers or databases, and receive long-range downlink signals 142 transmitted by remote servers 190.

In examples shown by FIG. 1, one of the plurality of display devices may be a custom display device 111 specially designed for displaying certain types of displayable sensor information associated with analyte values received from the sensor electronics module 140 (e.g., a numerical value and an arrow, in some examples). In some examples, one of the plurality of display devices may be a handheld device 112, such as a mobile phone based on the Android, iOS operating systems or other operating system, a palm-top computer and the like, where handheld device 112 may have a relatively larger display and be configured to display a graphical representation of the continuous sensor data (e.g., including current and historic data). Other display devices can include other hand-held devices, such as a tablet 113, a smart watch 110, a medicament delivery device 114, a blood glucose meter, and/or a desktop or laptop computer.

It should be understood that in the case of display device 114, which may be a medicament delivery device in addition to or instead of a display device, the alerts and/or sensor information provided by continuous analyte sensor 138 vis-à-vis sensor electronics module 140, can be used to initiate and/or regulate the delivery of the medicament to host.

During use, a sensing portion of sensor 138 may be disposed under the host's skin and a contact portion of sensor 138 can be electrically connected to sensor electronics module 140. Electronics module 140 can be engaged with a housing (e.g., a base) which is attached to a patch that may engage the skin of the host. The patch may be an adhesive patch in examples. In some examples, electronics module 140 is integrally formed with the housing. Furthermore, electronics module 140 may be disposable and directly coupled to the patch.

Continuous analyte sensor system 100 can include a sensor configuration that provides an output signal indicative of a concentration of an analyte. The output signal including (e.g., sensor data, such as a raw data stream, filtered data, smoothed data, and/or otherwise transformed sensor data) is sent to the receiver.

In some examples, analyte sensor system 102 includes a transcutaneous glucose sensor, such as is described in U.S. Patent Publication No. 2011/0027127. In some examples, sensor system 102 includes a continuous glucose sensor and comprises a transcutaneous sensor (e.g., as described in U.S. Pat. No. 6,565,509, as described in U.S. Pat. No. 6,579,690, and/or as described in U.S. Pat. No. 6,484,046).

Various signal processing techniques and glucose monitoring system examples suitable for use with the examples described herein are described in U.S. Patent Publication No. 2005/0203360 and U.S. Patent Publication No. 2009/0192745. The sensor can extend through a housing, which can maintain sensor 138 on, in or under the skin and/or can provide for electrical connection of sensor 138 to sensor electronics in sensor electronics module 140.

In some examples, description of a base, a housing, a wearable, and/or a transmitter of on-skin sensor assembly 160 may be interchangeable. In other examples, a base and a housing of on-skin sensor assembly 160 may be different in the sense that they may be separate components from sensor electronics module 140, e.g., from a transmitter or receiver.

In several examples, sensor 138 is in a form of a wire. A distal end of the wire can be formed, e.g., having a conical shape (to facilitate inserting the wire into the tissue of the host). Sensor 138 may comprise an elongate analyte sensor, and may include an elongate conductive body, such as an elongate conductive core (e.g., a metal wire) or an elongate conductive core coated with one, two, three, four, five, or more layers of material, each of which may or may not be conductive. The elongate analyte sensor may be long and thin, yet flexible and strong. For example, in some examples, the smallest dimension of the elongate conductive body is less than 0.1 inches, less than 0.075 inches, less than 0.05 inches, less than 0.025 inches, less than 0.01 inches, less than 0.004 inches, less than 0.002 inches, less than 0.001 inches, and/or less than 0.0005 inches.

Sensor 138 may have a circular shaped cross section. In some examples, the cross section of the elongated conductive body can be ovoid, rectangular, triangular, polyhedral, star-shaped, C-shaped, T-shaped, X-shaped, Y-shaped, irregular, or the like. In some examples, a conductive wire electrode is employed as a core. In other examples, sensor 138 may be disposed on a substantially planar substrate. To such an electrode, one or two additional conducting layers may be added (e.g., with intervening insulating layers provided for electrical isolation). The conductive layers can be comprised of any suitable material. In certain examples, it may be desirable to employ a conductive layer comprising conductive particles (i.e., particles of a conductive material) in a polymer or other binder.

In some examples, the materials used to form the elongate conductive body (e.g., stainless steel, titanium, tantalum, platinum, platinum-iridium, iridium, certain polymers, and/or the like) can be strong and hard, and therefore can be resistant to breakage. For example, in several examples, the ultimate tensile strength of the elongated conductive body is greater than 80 kPsi and less than 140 kPsi, and/or the Young's modulus of the elongate conductive body is greater than 160 GPa and less than 220 GPa. The yield strength of the elongate conductive body can be greater than 58 kPsi and less than 2200 kPsi.

Electronics module 140 can be releasably or permanently coupled to sensor 138. Electronics module 140 can include electronic circuitry associated with measuring and processing the continuous analyte sensor data. Electronics module 140 can be configured to perform algorithms associated with processing and calibration of the sensor data. For example, electronics module 140 can provide various aspects of the functionality of a sensor electronics module as described in U.S. Patent Publication No. 2009/0240120 and U.S. Patent Publication No. 2012/0078071. Electronics module 140 may include hardware, firmware, and/or software that enable measurement of levels of the analyte via a glucose sensor, such as sensor 138.

For example, electronics module 140 can include a potentiostat, a power source for providing power to sensor 138, signal processing components, data storage components, and a communication module (e.g., a telemetry module) for one-way or two-way data communication between electronics module 140 and one or more receivers, repeaters, and/or display devices, such as devices 110-114. Electronic components can be affixed to a printed circuit board (PCB), or the like, and can take a variety of forms. The electronic components can take the form of an integrated circuit (IC), such as an Application-Specific Integrated Circuit (ASIC), a microcontroller, and/or a processor. The electronics module 140 may include sensor electronics that are configured to process sensor information, such as storing data, analyzing data streams, calibrating analyte sensor data, estimating analyte values, comparing estimated analyte values with time-corresponding measured analyte values, analyzing a variation of estimated analyte values, and the like. Examples of systems and methods for processing sensor analyte data are described in more detail in U.S. Pat. No. 7,310,544, U.S. Pat. No. 6,931,327, U.S. Patent Publication No. 2005/0043598, U.S. Patent Publication No. 2007/0032706, U.S. Patent Publication No. 2007/0016381, U.S. Patent Publication No. 2008/0033254, U.S. Patent Publication No. 2005/0203360, U.S. Patent Publication No. 2005/0154271, U.S. Patent Publication No. 2005/0192557, U.S. Patent Publication No. 2006/0222566, U.S. Patent Publication No. 2007/0203966 and U.S. Patent Publication No. 2007/0208245. Electronics module 140 may communicate with the devices 110-114, and/or any number of additional devices, via any suitable communication protocol. Example communication methods or protocols include radio frequency; Bluetooth; universal serial bus; any of the wireless local area network (WLAN) communication standards, including the IEEE 802.11, 802.15, 802.20, 802.22 and other 802 communication protocols; ZigBee; wireless (e.g., cellular) telecommunication; paging network communication; magnetic induction; satellite data communication; a proprietary communication protocol, open source communication protocol, and/or any suitable wireless communication method.

Additional sensor information is described in U.S. Patent No. 7,497,827 and U.S. Patent No. 8,828,201.

Any sensor shown or described herein can be an analyte sensor; a glucose sensor; and/or any other suitable sensor. A sensor described in the context of any example can be any sensor described herein. Sensors shown or described herein can be configured to sense, measure, detect, and/or interact with any analyte.

As used herein, the term "analyte" is a broad term, and is to be given its ordinary and customary meaning to a person of ordinary skill in the art (and is not to be limited to a special or customized meaning), and refers without limitation to a substance or chemical constituent in a biological fluid (for example, blood, interstitial fluid, cerebral spinal fluid, lymph fluid, urine, sweat, saliva, etc.) that can be analyzed. Analytes can include naturally occurring substances, artificial substances, metabolites, or reaction products.

In some examples, the analyte for measurement by the sensing regions, devices, systems, and methods is glucose. However, other analytes are contemplated as well, including, but not limited to ketone bodies; acetyl-CoA; acarboxyprothrombin; acylcarnitine; adenine phosphoribosyl transferase; adenosine deaminase; albumin; alpha-fetoprotein; amino acid profiles (arginine (Krebs cycle), histidine/urocanic acid, homocysteine, phenylalanine/tyrosine, tryptophan); andrenostenedione; antipyrine; arabinitol enantiomers; arginase; benzoylecgonine (cocaine); biotinidase; biopterin; c-reactive protein; carnitine; carnosinase; CD4; ceruloplasmin; chenodeoxycholic acid; chloroquine; cholesterol; cholinesterase; cortisol; testosterone; choline; creatine kinase; creatine kinase MM isoenzyme; cyclosporin A; d-penicillamine; de-ethylchloroquine; dehydroepiandrosterone sulfate; DNA (acetylator polymorphism, alcohol dehydrogenase, alpha 1-antitrypsin, cystic fibrosis, Duchenne/Becker muscular dystrophy, glucose-6-phosphate dehydrogenase, hemoglobin A, hemoglobin S, hemoglobin C, hemoglobin D, hemoglobin E, hemoglobin F, D-Punjab, beta-thalassemia, hepatitis B virus, HCMV, HIV-1, HTLV-1, Leber hereditary optic neuropathy, MCAD, RNA, PKU, Plasmodium vivax, sexual differentiation, 21-deoxycortisol); desbutylhalofantrine; dihydropteridine reductase; diptheria/tetanus antitoxin; erythrocyte arginase; erythrocyte protoporphyrin; esterase D; fatty acids/acylglycines; triglycerides; glycerol; free β-human chorionic gonadotropin; free erythrocyte porphyrin; free thyroxine (FT4); free tri-iodothyronine (FT3); fumarylacetoacetase; galactose/gal-1-phosphate; galactose-1-phosphate uridyltransferase; gentamicin; glucose-6-phosphate dehydrogenase; glutathione; glutathione perioxidase; glycocholic acid; glycosylated hemoglobin; halofantrine; hemoglobin variants; hexosaminidase A; human erythrocyte carbonic anhydrase I; 17-alpha-hydroxyprogesterone; hypoxanthine phosphoribosyl transferase; immunoreactive trypsin; lactate; lead; lipoproteins ((a), B/A-1, β); lysozyme; mefloquine; netilmicin; phenobarbitone; phenytoin; phytanic/pristanic acid; progesterone; prolactin; prolidase; purine nucleoside phosphorylase; quinine; reverse tri-iodothyronine (rT3); selenium; serum pancreatic lipase; sissomicin; somatomedin C; specific antibodies (adenovirus, anti-nuclear antibody, anti-zeta antibody, arbovirus, Aujeszky's disease virus, dengue virus, Dracunculus medinensis, Echinococcus granulosus, Entamoeba histolytica, enterovirus, Giardia duodenalisa, Helicobacter pylori, hepatitis B virus, herpes virus, HIV-1, IgE (atopic disease), influenza virus, Leishmania donovani, leptospira, measles/mumps/rubella, Mycobacterium leprae, Mycoplasma pneumoniae, Myoglobin, Onchocerca volvulus, parainfluenza virus, Plasmodium falciparum, poliovirus, Pseudomonas aeruginosa, respiratory syncytial virus, rickettsia (scrub typhus), Schistosoma mansoni, Toxoplasma gondii, Trepenoma pallidium, Trypanosoma cruzi/rangeli, vesicular stomatis virus, Wuchereria bancrofti, yellow fever virus); specific antigens (hepatitis B virus, HIV-1); acetone (e.g., succinylacetone); acetoacetic acid; sulfadoxine; theophylline; thyrotropin (TSH); thyroxine (T4); thyroxine-binding globulin; trace elements; transferrin; UDP-galactose-4-epimerase; urea; uroporphyrinogen I synthase; vitamin A; white blood cells; and zinc protoporphyrin. Salts, sugar, protein, fat, vitamins, and hormones naturally occurring in blood or interstitial fluids can also constitute analytes in certain examples. The analyte can be naturally present in the biological fluid or endogenous, for example, a metabolic product, a hormone, an antigen, an antibody, and the like. Alternatively, the analyte can be introduced into the body or exogenous, for example, a contrast agent for imaging, a radioisotope, a chemical agent, a fluorocarbon-based synthetic blood, or a drug or pharmaceutical composition, including but not limited to insulin; glucagon; ethanol; cannabis (marijuana, tetrahydrocannabinol, hashish); inhalants (nitrous oxide, amyl nitrite, butyl nitrite, chlorohydrocarbons, hydrocarbons); cocaine (crack cocaine); stimulants (amphetamines, methamphetamines, Ritalin, Cylert, Preludin, Didrex, PreState, Voranil, Sandrex, Plegine); depressants (barbiturates, methaqualone, tranquilizers such as Valium, Librium, Miltown, Serax, Equanil, Tranxene); hallucinogens (phencyclidine, lysergic acid, mescaline, peyote, psilocybin); narcotics (heroin, codeine, morphine, opium, meperidine, Percocet, Percodan, Tussionex, Fentanyl, Darvon, Talwin, Lomotil); designer drugs (analogs of fentanyl, meperidine, amphetamines, methamphetamines, and phencyclidine, for example, Ecstasy); anabolic steroids; and nicotine. The metabolic products of drugs and pharmaceutical compositions are also contemplated analytes. Analytes such as neurochemicals and other chemicals generated within the body can also be analyzed, such as, for example, ascorbic acid, uric acid, dopamine, noradrenaline, 3-methoxytyramine (3MT), 3,4-dihydroxyphenylacetic acid (DOPAC), homovanillic acid (HVA), 5-hydroxytryptamine (SHT), 5-hydroxyindoleacetic acid (FHIAA), and intermediaries in the Citric Acid Cycle.

Any of the features described in the context of at least FIG. 1 can be applicable to all aspects and examples identified herein. Moreover, any of the features of an example is independently combinable, partly or wholly with other examples described herein in any way, e.g., one, two, or three or more examples may be combinable in whole or in part. Further, any of the features of an example may be made optional to other aspects or examples. Any aspect or example of a method can be performed by a system or apparatus of another aspect or example, and any aspect or example of a system can be configured to perform a method of another aspect or example.

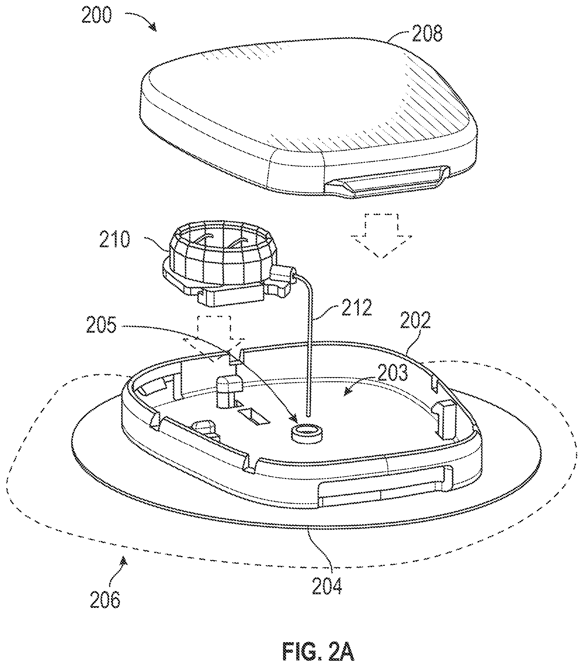

FIG. 2A illustrates a perspective view of an exemplary on-skin wearable medical device, in the form of an on-skin sensor assembly 200, which is configured to be deployed to skin. The on-skin sensor assembly 200 may include a housing or base 202. The housing or base 202 may be configured to be worn on skin of a host and may include a distal surface for facing towards the skin and a proximal surface 203 facing opposite the distal surface. The housing or base 202 may include an opening 205 for an insertion element to be retracted proximally through from the skin. A patch 204 such as an adhesive patch can couple the base 202 to the skin 206 of the host. The patch 204 may be positioned on the distal surface of the housing or base 202. In some examples, the adhesive patch 204 may include an engaging surface for engaging the skin and including an adhesive suitable for skin adhesion, for example a pressure sensitive adhesive (e.g., acrylic, rubber-based, or other suitable type) bonded to a carrier substrate (e.g., spun lace polyester, polyurethane film, or other suitable type) for skin attachment, though any suitable type of adhesive is also contemplated. An on-skin sensor assembly 200 may comprise an electronics unit 208 (e.g., a transmitter) which may further comprise a glucose sensor module 210 coupled to an analyte sensor such as a transcutaneous analyte sensor (e.g., a glucose sensor) 212 and to base 202.

The applicator system can engage the adhesive patch 204 to skin 206. The glucose sensor module 210 may be secured to base 202 (e.g., via retention elements such as snap fits and/or interference features, adhesive, welding, etc.) to ensure analyte sensor 212 (e.g., glucose sensor) is coupled to base 202. In alternative examples, the sensor module 210 and base 202 are preassembled or manufactured as a single component.

After on-skin sensor assembly 200 is deployed to a user's skin, a user (or an applicator) can couple electronics unit 208 (e.g., a transmitter) to on-skin sensor assembly 200 via retention elements such as snap fits and/or interference features. Electronics unit 208 can measure and/or analyze glucose indicators sensed by transcutaneous analyte sensor (e.g., a glucose sensor) 212. Electronics unit 208 can transmit information (e.g., measurements, analyte data, glucose data) to a remotely located device (e.g., 110-114 shown in FIG. 1).

On-skin sensor assembly 200 may be attached to the host with use of an applicator adapted to provide convenient and secure application. Such an applicator may also be used for attaching electronics unit 208 to base 202, inserting sensor 212 through the host's skin, and/or connecting sensor 212 to electronics unit 208. Once electronics unit 208 is engaged with the base and sensor 212 has been inserted into the skin (and is connected to the electronics unit 208), the sensor assembly can detach from the applicator.

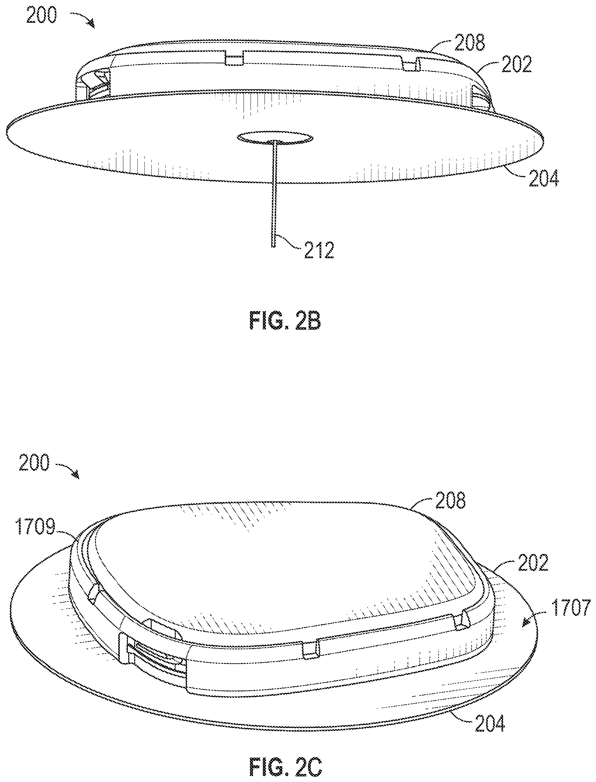

FIG. 2B illustrates a perspective view of electronics unit 208 coupled to base 202 via retention elements such as snap fits and/or interference features. In some examples, electronics unit 208 and base 202 are coupled by adhesive, welding, or other bonding techniques. Patch 204, on a distal surface of base 202, is configured to couple sensor assembly 200 to the skin.

FIG. 2C illustrates a perspective view of on-skin sensor assembly 200. On-skin sensor assembly 200 may be disposable or reusable. FIG. 2C further illustrates electronics unit 208 coupled to a base 202, and adhesive patch 204 configured to be attached to on-skin sensor assembly 200, which, when combined, may be held within the applicator.

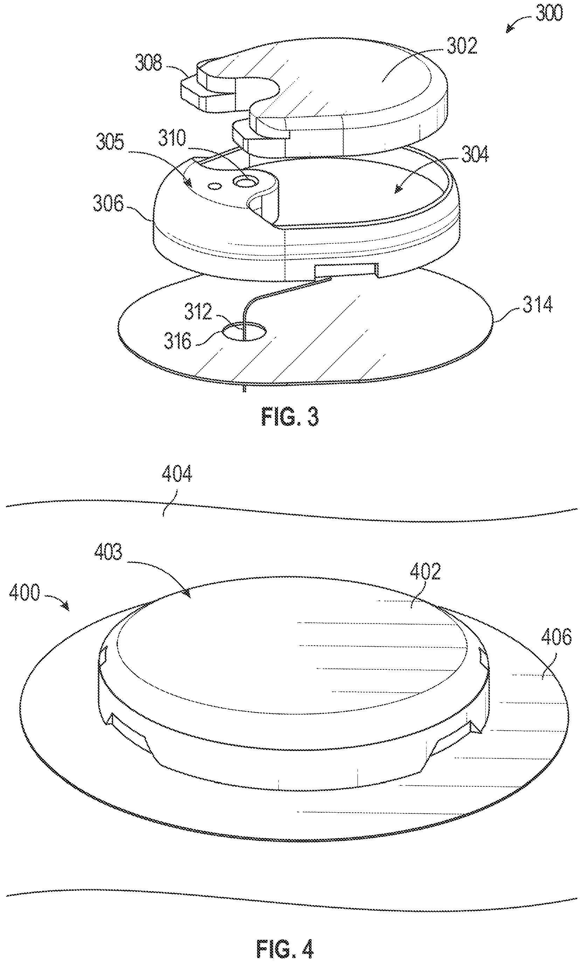

FIG. 3 illustrates an example of an on-skin wearable medical device in the form of an on-skin sensor assembly 300 with an electronics unit 302 configured to insert into a cavity 304 of the base or housing 306. The base or housing 306 may be configured to be worn on skin of a host and may include a distal surface for facing towards the skin and a proximal surface 305 facing opposite the distal surface. The electronics unit 302 may include one or more tabs 308 that couple to a portion of the housing 306 and allow the electronics unit 302 to be retained by the housing 306. The housing 306 may include an opening 310 for an insertion element to be retracted proximally through from the skin. The opening 310 may allow the insertion element (such as a needle) to pass through to deploy the transcutaneous analyte sensor 312 to the skin. The patch 314 may further include an aperture 316 that may allow the sensor 312 and the insertion element to pass through. The electronics unit 302 may couple to the housing 306 prior to or following deployment of the sensor 312 to the host's skin.

FIG. 4 illustrate an example of an on-skin wearable medical device in the form of an on-skin sensor assembly 400, in which the electronics unit is integral with the housing 402. The housing 402 may be configured to be worn on skin of a host and may include a distal surface for facing towards the skin and a proximal surface 403 facing opposite the distal surface. The on-skin sensor assembly 400 is shown on the skin 404, with the patch 406 engaging the skin 404.

The examples of FIGS. 2A-4 may each include an engaging surface for engaging the skin. The engaging surface may be positioned on the patch in examples, for example on a distal surface of the patch or may have another position in examples. The engaging surface may comprise an adhesive surface in examples configured to adhere to the skin. The adhesive can be configured for adhering to skin. Additional adhesive information is described in U.S. Patent No. 11,219,413, which was filed on August 25, 2015. The engaging surface in examples may be covered with a liner prior to deployment to the host's skin.

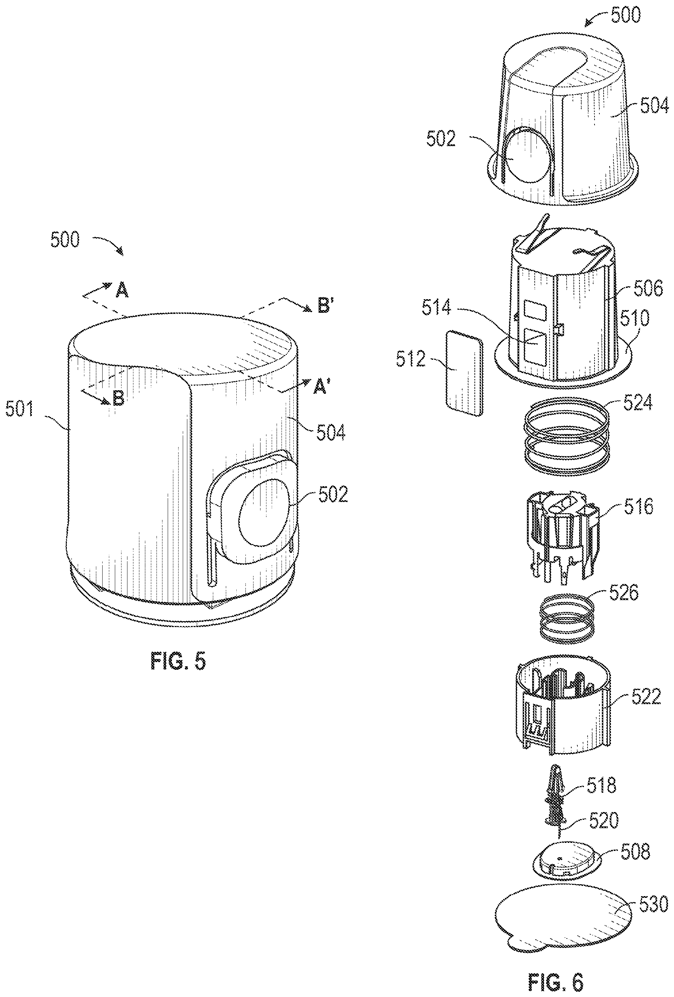

FIG. 5 illustrates a system for deploying an on-skin wearable medical device to skin. The system may comprise an applicator system in examples. The system may include an applicator for an on-skin sensor assembly of an analyte sensor system, according to some examples. In examples, other forms of systems may be utilized.

The applicator 500 may include an applicator housing 501, which may include an outer housing 504 and an inner housing 506, and other forms of housings in examples. The applicator housing 501 may be configured to retain the on-skin wearable medical device in examples. The applicator 500 may include a deployment mechanism that may be configured to deploy the on-skin wearable medical device to skin. The deployment mechanism, for example, may include one or more retention element(s) for retaining the on-skin wearable medical device and releasing the on-skin wearable medical device from the applicator housing 501 to the skin in examples. The deployment mechanism may include an insertion assembly for inserting at least a portion of the on-skin wearable medical device into the skin. The insertion assembly may drive a portion of the on-skin wearable medical device, such as the insertion element and the sensor, into the skin of the host. The deployment mechanism may include a retraction assembly for retracting the portion of the on-skin wearable medical device from the skin, such as an insertion element.

In examples, the applicator 500 may include an activation element 502 disposed on a side of applicator 500, for example, on a side of an outer housing 504 of applicator 500. In some examples, activation element 502 may be a button, a switch, a toggle, a slide, a trigger, a knob, a rotating member, a portion of applicator 500 that deforms and/or flexes or any other suitable mechanism for activating an insertion and/or retraction assembly of applicator 500. In some examples, activation element 502 may be disposed in any location, e.g., a top, upper side, lower side, or any other location of applicator 500. Applicator 500 may be large enough for a host to grasp with a hand and push, or otherwise activate, activation element 502 with, for example, a thumb, or with an index finger and/or a middle finger.

Applicator 500 may be configured with one or more safety features such that applicator 500 is prevented from activating until the safety feature is deactivated. In one example, the one or more safety features prevents applicator 500 from activating unless applicator 500 is pressed against the skin of a host with sufficient force. Moreover, as will be described in more detail in connection with one or more of FIGS. 6-20B below, applicator 500 may be further configured such that one or more components therein retract based at least in part on the one or more components pushing against the skin of the host with a force exceeding a predetermined threshold, rather than based on the one or more components translating beyond a predetermined and static distal position. In other words, applicator 500 may implement force-based retraction triggering rather than being limited to displacement-based retraction triggering.

FIG. 6 illustrates an exploded perspective view of applicator 500 of FIG. 5, according to some examples. Applicator 500 may include outer applicator housing 504 comprising activation element 502. The outer applicator housing 504 may be configured to be gripped by a user in examples. Outer applicator housing 504 may be configured to translate in a distal direction by a force applied by a host to applicator 500, specifically to inner housing 506, thereby aligning activation element 502 in a position that allows applicator 500 to fire. Further explanation of the alignment process will be explained below.

Applicator 500 further comprises inner housing 506, configured to house at least one or more mechanisms utilized to apply on-skin sensor assembly 508 to skin of a host. A distal surface 510 of a bottom opening of inner housing 506 may define a bottom surface of applicator 500. In some examples, upon pressing applicator 500 against skin of the host, skin may deform in a substantially convex shape at distal surface 510 such that at least a portion of a surface of skin disposed at the bottom opening of applicator inner housing 506 extends into the bottom opening of inner housing 506 beyond a plane defined by distal surface 510 in a proximal direction.

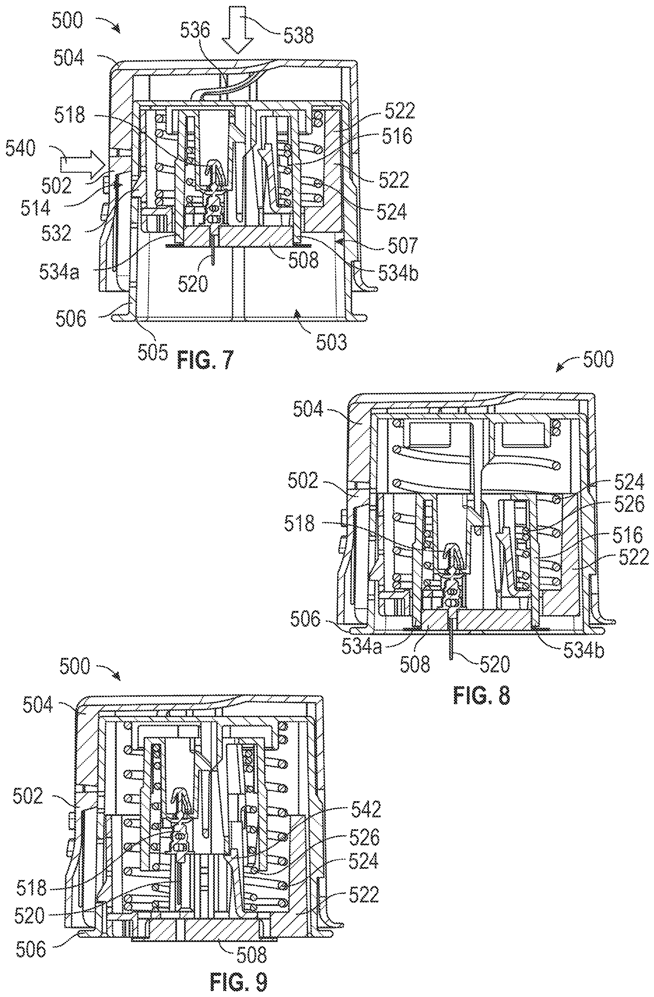

As shown in FIG. 7, the housing 501, and particularly the inner housing 506 may include an internal cavity 503 for retaining the on-skin wearable medical device. The internal cavity 503 may have a distal end portion 505 at the opening for on-skin wearable medical device to be deployed from. A proximal end portion 507 of the internal cavity 503 may include the on-skin wearable medical device coupled to the needle carrier assembly 516.

Referring back to FIG. 6, in some examples, a first barrier layer 512 may be disposed over one or more apertures in inner housing 506, for example, an aperture 514 through which at least a portion of activation element 502 may be configured to extend through during activation of applicator 500. In such examples, a portion of activation element 502 may be configured to pierce or deform first barrier layer 512 upon activation of applicator 500. First barrier layer 512 may comprise a gas permeable material such as Tyvek, or a non-gas permeable material such as metallic foil, polymer film, elastomer, or any other suitable material.

Applicator 500 may further comprise a needle carrier assembly 516, including a needle hub 518 configured to couple an insertion element 520 to needle carrier assembly 516. In some other examples, insertion element 520 may be directly coupled to needle carrier assembly 516. Insertion element 520 is configured to insert sensor of on-skin sensor assembly 508 into skin of the host. In some examples, the insertion element comprises a needle, for example, an open sided-needle, a needle with a deflected-tip, a curved needle, a polymer-coated needle, a hypodermic needle, or any other suitable type of needle or structure. In yet other examples, insertion element 520 may be integrally formed with sensor and may be sufficiently rigid to be inserted partially into skin of the host with minimal or no structural support.

Applicator 500 may further include a holder 522 releasably coupled to needle carrier assembly 516 and configured to guide needle carrier assembly 516 and on-skin sensor assembly 508 while coupled to needle carrier assembly 516, e.g., at least during translation from a proximal position to a distal insertion position. As will be described in more detail below, on-skin sensor assembly 508 may be stripped or released from holder 522 and/or needle carrier assembly 516 once on-skin sensor assembly 508 is disposed on skin of the host. For example, one or more retention elements may release the on-skin wearable medical device from the applicator housing 501.

Applicator 500 may further comprise an insertion assembly configured to translate insertion element 520, needle hub 518, needle carrier assembly 516, and on-skin sensor assembly 508 from a proximal position, in the distal direction, to a distal insertion position. Such an insertion assembly may include at least one spring for inserting at least a portion of the on-skin wearable device into the skin. The insertion assembly may include a first spring 524. First spring 524 may be a compression spring, or any suitable type of spring, and may have a first end in contact with or coupled to inner applicator housing 506 and a second end in contact with or coupled to holder 522. First spring 524 is configured to, upon activation of the insertion assembly, translate holder 522, needle carrier assembly 516, needle hub 518, insertion element 520 and on-skin sensor assembly 508, in the distal direction to the distal insertion position. Substantially at the distal insertion position, needle carrier assembly 516 may decouple from holder 522 and on-skin sensor assembly 508.

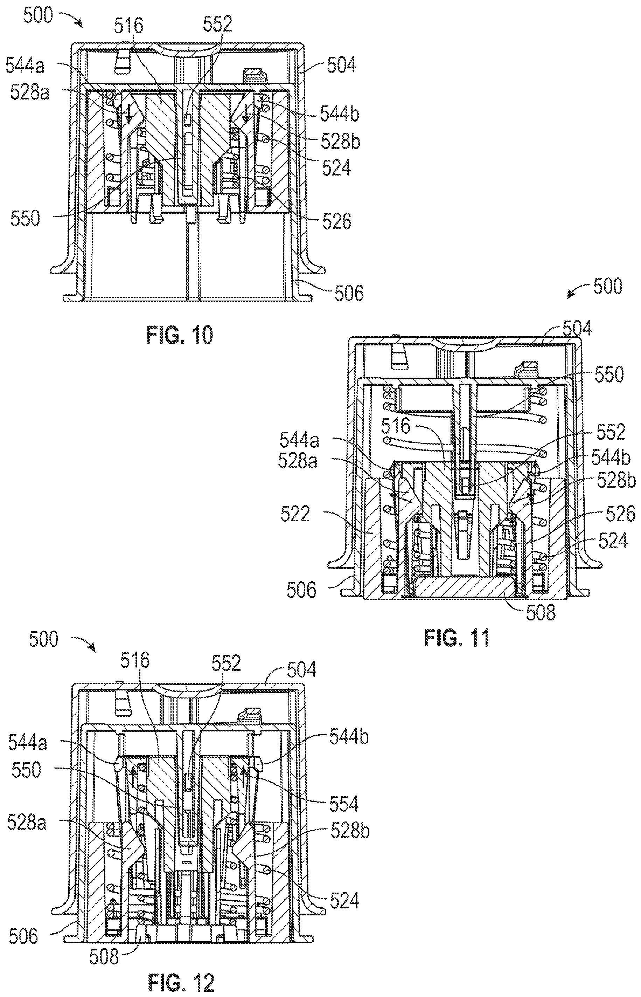

Applicator 500 may further comprise a retraction assembly for retracting the insertion element (e.g., needle) from the skin. The retraction assembly may be configured to translate needle carrier assembly 516, needle hub 518 and insertion element 520, in the proximal direction, from the distal insertion position to a proximal retracted position. In some examples the initial proximal position may be the same as the proximal retracted position. In other examples, the initial proximal position may be different from the proximal retracted position. Such a retraction assembly may include at least one spring. The retraction assembly may include a second spring 526. Second spring 526 may be a compression spring, or any suitable type of spring, and may have a first end contacting or coupled to holder 522 and a second end in contact with or coupled to at least one spring retention element (e.g., 528a, 528b in FIGS. 10-14), at least until retraction. Second spring 526 is configured to translate needle carrier assembly 516, needle hub 518, and insertion element 520 in the proximal direction from the distal insertion position to the proximal retracted position in response to on-skin sensor assembly 508 contacting skin of the host, and/or reaching a limit of travel with a force exceeding a predetermined threshold sufficient to cause first end of second spring 526 to overcome the at least one spring retention element (e.g., 528a, 528b in FIGS. 10-14). In some examples, a stop feature (not shown) may be disposed at a bottom of applicator 500, e.g., on a distal portion of inner housing 506. Such a stop feature may be configured to contact one or more of on-skin sensor assembly 508, needle carrier assembly 516, or holder 522 in the distal insertion position.

In some examples, a second barrier layer 530 may be disposed over the bottom opening of inner housing 506. Second barrier layer 530 may comprise a gas permeable material such as Tyvek, or a non-gas permeable material such as metallic foil, film. In some examples, second barrier layer 530 may be removed by the host prior to use of applicator 500. In examples comprising one or both of first and second barrier layers 512, 530, such layers may provide a sterile environment between applicator 500 and the outside environment and/or may allow ingress and egress of gas such as during sterilization.

A brief description of some aspects of the operation of applicator 500 follows with respect to FIGS. 7-9, which illustrate several cross-sectional views of applicator 500 of FIGS. 5 and 6 during operation, according to some examples. FIGS. 7-9 may correspond to applicator 500 cut along the section line A-A' shown in FIG. 5, for example.

FIG. 7 illustrates a state of applicator 500 prior to activation. Holder 522 comprises an insertion assembly retention element 532 configured to contact inner housing 506, thereby immobilizing holder 522, needle carrier assembly 516, needle hub 518, insertion element 520 and on-skin sensor assembly 508, in the pre-activated state.

Needle carrier assembly 516 comprises a plurality of wearable retention and/or alignment elements 534a, 534b configured to extend through holder 522 and releasably couple on-skin sensor assembly 508 to holder 522 and/or to needle carrier assembly 516. Wearable retention elements 534a, 534b may comprise, e.g., anus, deflection element, tabs, detents, snaps or any other features capable of a retaining function. In some examples, wearable retention elements 534a, 534b may extend around rather than through holder 522. Although two wearable retention elements are illustrated, any number of wearable retention elements are contemplated. In some examples, wearable retention element(s) 534a, 534b may comprise snap fits, friction fits, interference features, elastomeric grips and/or adhesives configured to couple on-skin sensor assembly 508 with needle carrier assembly 516 and/or holder 522.

Inner housing 506 may comprise a spring 536 configured to contact outer housing 504 and maintain a predetermined spacing between outer housing 504 and inner housing 506 in the pre-activation orientation of FIG. 7. Spring 536 may be a compression spring, leaf spring, flex arm spring, a piece of foam or rubber, etc. In some other examples, outer housing 504 may comprise spring 536 and spring 536 may be configured to contact inner housing 506, in a reverse fashion from that shown in FIG. 7.

Activation of applicator 500 may include a host pressing applicator 500 against their skin with sufficient force to translate outer housing 504 in a distal direction, as shown by arrow 538, toward and with respect to inner housing 506 until activation element 502 is aligned with aperture 514 of inner housing 506 and insertion assembly retention element 532 of holder 522. Insertion assembly retention element 532 may comprise, e.g., an arm, a deflection element, a tab, a detent a snap or any other feature capable of a retaining function. Once such an alignment is achieved, a host may initiate (e.g. pushing) activation element 502, as shown by arrow 540, thereby deflecting insertion assembly retention element 532 sufficiently to release holder 522 from inner housing 506. In some other examples, applicator 500 may be configured such that activation element 502 may be activated first, but that actual insertion is not triggered until outer housing 504 is translated sufficiently in the distal direction toward and with respect to inner housing 506. In yet other examples, activation element 502 may be biased toward a center of applicator 500 such that activation element 502 need not be explicitly activated by the host but, instead, activation element 502 may be configured to automatically initiate insertion upon outer housing 504 being translated sufficiently in the distal direction toward and with respect to inner housing 506.

Such configurations provide several benefits. First, translation of outer housing 504 with respect to inner housing 506 before activation provides a measure of drop protection such that if applicator 500 is accidentally dropped, it may not prematurely fire. Second, spring 536 provides a force bias that the host has to affirmatively overcome by pressing applicator 500 into their skin prior to firing, thereby reducing the probability of activating applicator 500 before it is properly positioned. Further, the host may decide to not fire applicator 500 and discontinue pressing applicator 500 against their skin, in which spring 536 will bias against outer housing 504 and allow outer housing 504 to return to its initial state.

Holder 522, needle carrier assembly 516, needle hub 518, insertion element 520, on-skin sensor assembly 508, first spring 524 and second spring 526 are all shown in pre-activation positions in FIG. 7.

FIG. 8 illustrates applicator 500 during insertion of on-skin sensor assembly 508 but before retraction of needle carrier assembly 516. First spring 524 drives holder 522, needle carrier assembly 516, needle hub 518, insertion element 520, and on-skin sensor assembly 508, in the distal direction toward the distal insertion position. FIG. 8 illustrates a position where on-skin sensor assembly 508 is in contact with skin of the host but where holder 522 is not yet fully driven, by first spring 524, into contact with on-skin sensor assembly 508 or skin of the host.

In some examples, masses of each of holder 522, needle carrier assembly 516, needle hub 518, insertion element 520, and on-skin sensor assembly 508 may be specifically designed to reduce or substantially eliminate a tendency of needle carrier assembly 516, needle hub 518, insertion element 520, and on-skin sensor assembly 508 to detach due to inertial forces from holder 522 while being driven in the distal direction during insertion. In some examples, a force exerted by first spring 524 may be selected to be sufficient for proper operation of applicator 500, while not so large as to further exacerbate such above-described inertially triggered detachment. In some examples, a spring (not shown) may be configured to exert a force against a portion of needle carrier assembly 516, for example in a distal direction, sufficient to prevent needle carrier assembly 516 from inertially triggered detaching from holder 522 during insertion.

FIG. 9 illustrates applicator 500 during activation, as needle carrier assembly 516, needle hub 518 and insertion element 520 are retracted in the proximal direction by second spring 526. In FIG. 9, first spring 524 has fully driven on-skin sensor assembly 508 to the skin of the host. In this position, second spring 526 is released from spring retention elements (e.g., 528a, 528b in FIGS. 10-14) and drives needle carrier assembly 516, needle hub 518, and insertion element 520 in the proximal direction from the distal insertion position. Upon needle carrier assembly 516 reaching the proximal retraction position, needle carrier retention element 542 of holder 522 engages with needle carrier assembly 516, thereby maintaining needle carrier assembly 516, needle hub 518 and insertion element 520 in a locked, retracted position limiting access to insertion element 520. Needle carrier retention element 542 may comprise, e.g., an arm, a deflection element, a tab, a detent, a snap or any other feature capable of a retaining function. In this retracted position, needle carrier assembly 516, needle hub 518, and insertion element 520 is prevented from travelling in a distal direction.

A further description of some aspects of the operation of applicator 500 follows with respect to FIGS. 10-12, which illustrate several cross-sectional views of applicator 500 of FIGS. 5 and 6 during operation, according to some examples. FIGS. 10-12 may correspond to applicator 500 cut along the section line B-B' shown in FIG. 5, for example. For ease of illustration, needle hub 518 and insertion element 520 are not shown in FIGS. 10-12.

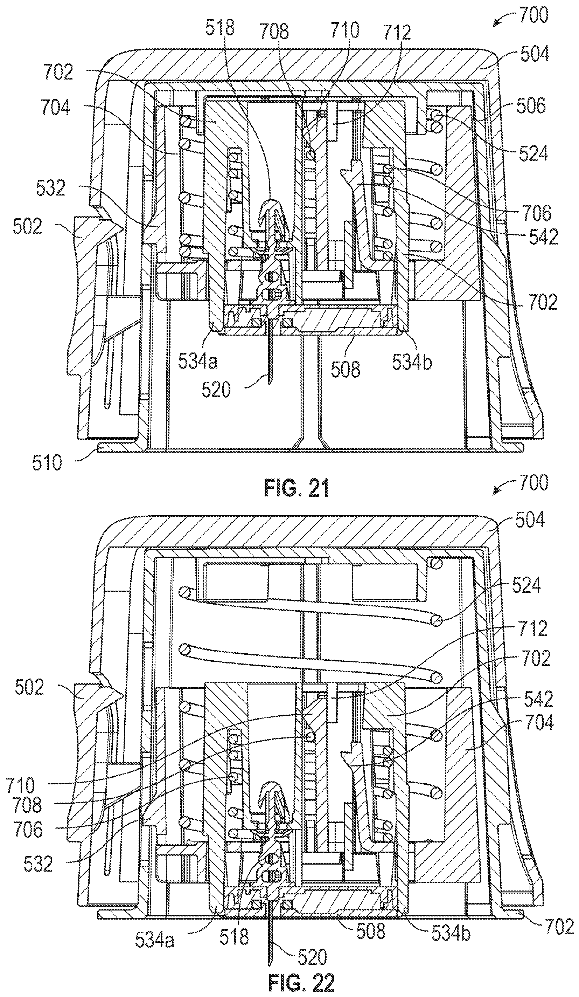

FIG. 10 illustrates a state of applicator 500 prior to activation. For ease of illustration, on-skin sensor assembly 508 is not illustrated in FIG. 10. Holder 522 comprises spring retention elements 528a, 528b configured to contact and retain a first end of second spring 526 in the pre-activated state, e.g., during insertion, while a second end of spring 526 is in contact with needle carrier assembly 516. Spring retention elements 528a, 528b may comprise, e.g., arms, deflection element, tabs, detents, snaps or any other features capable of a retaining function. Although two spring retention elements 528a, 528b are shown, at least one spring retention element is contemplated. In some examples, applicator 500 may include one spring retention element, as shown in FIGS. 21-24. In some examples, applicator 500 may include three spring retention elements. In some examples, applicator 500 may include four spring retention elements. In some examples, spring retention elements 528a, 528b are deflectable arms, rigid arms, deformable features, snaps, catches, or hooks. In some examples, spring retention elements 528a, 528b may be actively deflected by one or more features within applicator 500.

Needle carrier assembly 516 comprises backstop features 544a, 544b, configured to prevent lateral deflection of spring retention elements 528a, 528b in the proximal starting position, e.g., at least during insertion, thereby supporting retention of second spring 526 between spring retention elements 528a, 528b and holder 522 until retraction. Although two backstop features are illustrated, any number of backstop features are contemplated. The number of backstop features may equal the number of spring retention elements.

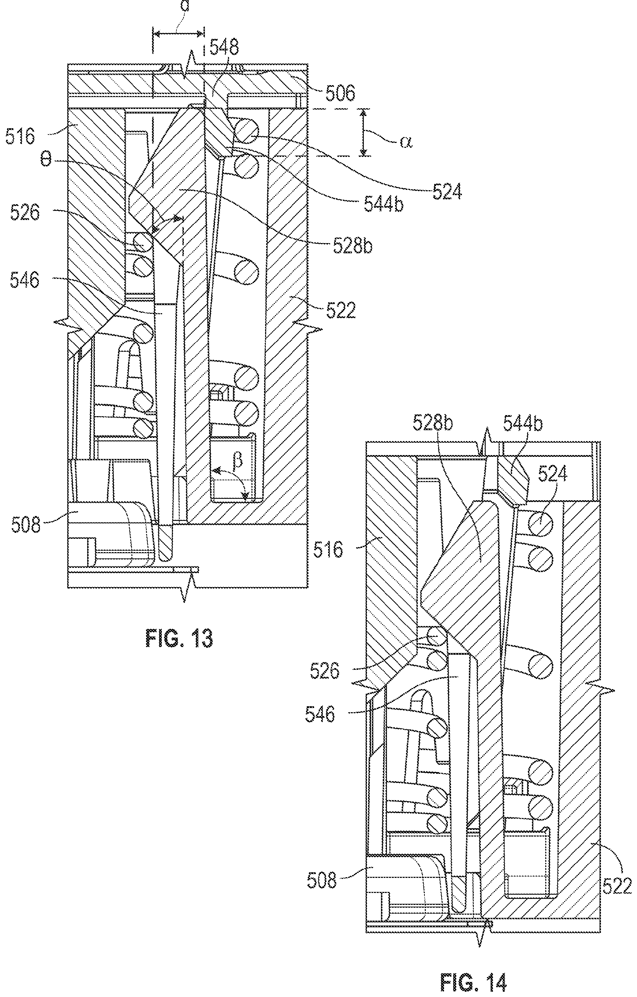

FIG. 13 illustrates a magnified view of spring retention element 528b and backstop feature 544b. In FIG. 13, first spring 524 is driving holder 522, needle carrier assembly 516 and on-skin sensor assembly 508, in the distal direction toward the distal inserted position. Backstop feature 544b is shown engaged to spring retention element 528b, preventing spring retention element 528b from deflecting laterally, thereby preventing second spring 526 from releasing. As shown in FIG. 13, a proximal end of spring retention element 528b may be offset from a distal end of backstop feature 544b by a distance a. In some examples, distance a is the length required for spring retention element 528b to traverse along backstop feature 544b such that spring retention element 528b clears past back-stop feature 544b. Backstop feature 544b may feature a ramp to guide spring retention element 528b. A distal end of needle carrier assembly 516 and a distal end of holder 522 may be offset from each other at least the same distance α to allow for spring retention element 528b to traverse distally past backstop feature 544b.

It may be appreciated that the frictional force between corresponding contacting surfaces of backstop feature 544b and spring retention element 528b may at least partly determine an amount of force to release spring retention element 528b from backstop feature 544b. This force may allow for lateral deflection of spring retention element 528b and thus allow the expansion of second spring 526. In some examples, the amount of force is at least 0.1 pounds. In some examples, the amount of force is at least 0.5 pounds. In some examples, the amount of force is at least 1 pound. In some examples, the amount of force is at least 2 pounds. In some examples, the amount of force is at least 3 pounds. In some examples, the amount of force is at least 4 pounds. In some examples, the amount of force is at least 5 pounds.

Although the figure shows backstop feature 544b preventing lateral deflection of spring retention element 528b in a radially outward direction, it is contemplated that an inverse structural relationship can be achieved. For instance, the ramped surface of spring retention element 528b can be reversed to face the opposite direction as shown in FIG. 13. Further, the ramped surface of spring retention element 528b may be biased in a radially inward direction by second spring 526 against backstop feature 544b. In such examples, backstop feature 544b may be located radially inward of spring retention element 528b.

Accordingly, in some examples, materials utilized to form holder 522 and needle carrier assembly 516 may be selected based on a desired amount of force to release spring retention element 528b for lateral deflection. Examples of such materials may include polycarbonate, ABS, PC/ABS, polypropylene, HIPS (High impact polystyrene), polybutylene terephthalate (PBT), polyoxymethylene (POM), acetal, polyacetal, polyformaldehyde, PTFE, high density polyethylene (HDPE), ultra-high-molecular-weight polyethylene (UHMWPE), nylon, polyethylene terephthalate (PET), thermoplastic elastomer (TPE), thermoplastic polyurethane (TPU), TPSiv, cyclo-olefin polymer (COP), cyclo-olefin copolymer (COC), and/or liquid-crystal polymer (LCP).

An angle θ of a portion of spring retention element 528b in contact with second spring 526 may also affect the amount of frictional force to laterally deflect spring retention element 528b and so to release second spring 526. Accordingly, the angle θ may be selected based on a desired amount of force to laterally deflect spring retention element 528b sufficiently to release second spring 526. In some examples, the angle θ is at least 1 degree with respect to a vertical axis of the spring retention element 528b. In some examples, the angle θ is at least 5 degrees. In some examples, the angle θ is at least 10 degrees. In some examples, the angle θ is at least 15 degrees. In some examples, the angle θ is at least 20 degrees. In some examples, the angle θ is about 30 to 45 degrees. In addition, the force profile of second spring 526 may affect a target amount of frictional force to laterally deflect spring retention element 528b. Accordingly, in some examples, the force profile of second spring 526 may be taken into account when selecting one or both of the materials for forming holder 522 and needle carrier assembly 516 and the angle θ of the portion of spring retention element 528b in contact with second spring 526.

An angle θ of spring retention element 528b with respect to a vertical axis may also affect the amount of frictional force to laterally deflect spring retention element 528b and so to release second spring 526. By contacting spring retention element 528b, second spring 526 may exert a force on spring retention element 528b at a distance d from a bottom of spring retention element 528b that causes a torque moment sufficient to induce a lateral deflection of spring retention element 528b.

FIG. 13 further illustrates needle carrier assembly 516 comprising a deflecting element 546 configured to contact spring retention element 528b and maintain spring retention element 528b in a laterally deflected orientation once second spring 526 has initially deflected spring retention element 528b and sufficiently driven needle carrier assembly 516 in the proximal direction, as will be shown in more detail in FIG. 14. Deflecting element 546 may prevent spring retention element 528b from contacting the windings of second spring 526 while second spring 526 is extending, smoothing the operation of applicator 500 and preventing energy released by second spring 526 and designed for driving needle carrier assembly 516 in the proximal direction from being absorbed by undesired contact with spring retention element 528b during the release of second spring 526.

In some examples, the angle θ of the portion of spring retention element 528b in contact with second spring 526 may be substantially 90° (e.g., flat) and deflecting element 546 may have a ramped or angled surface in contact with spring retention element 528b in the position illustrated in FIG. 13. In such examples, deflecting element 546, in addition to the above-described functionality, may be configured to initially deflect spring retention element 528b as first spring 524 drives holder 522 from the position illustrated in FIG. 13 to the position illustrated in FIG. 14.

In some examples, inner housing 506 may comprise a protrusion 548 extending from inner housing 506 in the distal direction. Protrusion 548 may be configured to contact at least one of spring retention elements 528a, 528b and backstop features 544a, 544b in the pre-activation state such that spring retention elements 528a, 528b are prevented from laterally deflecting until holder 522 and needle carrier assembly 516 have translated at least a predetermined minimum distance in the distal direction. Accordingly, protrusion 548 may provide a measure of drop protection such that applicator 500 may not prematurely fire in response to a concussive shock from being dropped before intentional activation.

Turning back to FIG. 10, inner housing 506 may further comprise an engagement element 550 configured to engage with a protrusion 552 of needle carrier assembly 516 upon needle carrier assembly 516 translating in the distal direction beyond a predetermined threshold, thereby preventing needle carrier assembly 516 from translating in the distal direction beyond the predetermined threshold. It is contemplated that this may ensure needle carrier assembly retraction in the event of an air firing or dry firing in which applicator 500 is somehow activated when not held against the skin of the host. In some examples, the predetermined threshold may correspond to the distal end of needle carrier assembly 516 extending beyond a point proximal to the distal end of inner housing 506, to a point substantially in line with the distal end of inner housing 506 or to a point distal of the distal end of inner housing 506. In some examples, engagement element 550 comprises a hook, a U-shaped structure, a loop, a protrusion, or any other structure capable of engaging with protrusion 552 as described above.

FIG. 11 illustrates applicator 500 after activation, at a beginning of a force retraction feature process at or near the distal insertion position where on-skin sensor assembly 508 may be in contact with the skin of the host. First spring 524 has driven holder 522, needle carrier assembly 516, needle hub 518, insertion element, and on-skin sensor assembly 508, in the distal direction toward the distal insertion position. During proper operation, holder 522 and on-skin sensor assembly 508 should be pressing against the skin of the host. However, FIG. 11 may also illustrate a dry fire condition, where applicator 500 is not properly pressed against the skin of the host before triggering applicator 500. Accordingly, upon first spring 524 driving holder 522 and needle carrier assembly 516 in the distal direction beyond the predetermined threshold, engagement element 550 contacts protrusion 552, which prevents needle carrier assembly 516 from traveling further in the distal direction, while holder 522 is driven sufficiently further in the distal direction such that backstop features 544a, 544b of needle carrier assembly 516 no longer contact spring retention elements 528a, 528b in the distal insertion position, thereby releasing the first end of second spring 526 and initiating retraction even when applicator 500 is dry fired. The insertion force provided by first spring 524 may be sufficient to additionally overcome the frictional force between corresponding contacting surfaces of backstop feature 544b and spring retention element 528b.

Turning to FIG. 14, first spring 524 has driven holder 522, needle carrier assembly 516 and on-skin sensor assembly 508 in the distal direction to the skin of the host. As first spring 524 drives holder 522, needle carrier assembly 516 and on-skin sensor assembly 508 against the skin of the host, the skin provides a counter force to the force generated by first spring 524. The skin may oppose the force of first spring 524 and bias against the distal end of on-skin sensor assembly 508. Because the distal end of holder 522 is offset from the distal end of on-skin sensor assembly 508 as shown in FIG. 13, the counter force provided by the skin is transferred to holder 522 as first spring 524 continues to drive holder 522 towards the skin while on-skin sensor assembly 508 is pressed against the skin. The counter force provided by the skin allows spring retention element 528b to displace past backstop feature 544b. Once spring retention element 528b has cleared distance a past backstop feature 544b, second spring 526 can laterally deflect spring retention element 528b, thereby releasing second spring 526, which drives needle carrier assembly 516 in the proximal direction. Alternatively, as described above in connection with FIG. 13, where the angle θ of the portion of spring retention element 528b in contact with second spring 526 is substantially 90° (e.g., flat), the ramped or angled surface of deflecting element 546 in contact with spring retention element 528b deflects spring retention element 528b sufficiently to release second spring 526, which drives needle carrier assembly 516 in the proximal direction.

In some examples, engagement element 550 may engage protrusion 552 even when applicator 500 is pressed against the skin of a user. In such examples, engagement element 550 engages protrusion 552 as first spring 524 drives holder 522, needle carrier assembly 516, and on-skin sensor assembly 508 against the skin of the host. As explained above, engagement element 550 prevents needle carrier assembly 516 from moving distally when engagement element 550 engages protrusion 552. This allows spring retention elements 528a, 528b to separate away from backstop features 544a, 544b and allow for release of second spring 526. The engagement of engagement element 550 and protrusion 552 may add additional force to the counter force provided by the skin, thus increasing the energy needed to overcome the frictional engagement of spring retention elements 528a, 528b and backstop features 544a, 544b. In some instances, the engagement of engagement element 550 and protrusion 552 provides an immediate impulse force that converts at least some of the initial energy of first spring 524 into energy needed to overcome the frictional engagement of spring retention elements 528a, 528b and backstop features 544a, 544b. It is contemplated that such examples may benefit users with soft skin or higher body fat percentage.

Turning back to FIG. 12, which illustrates applicator 500 during activation, needle carrier assembly 516 is retracted in the proximal direction by second spring 526, as indicated by arrow 554. In FIG. 12, with backstop features 544a, 544b no longer immobilizing spring retention elements 528a, 528b, first end of second spring 526 pushes against spring retention elements 528a, 528b with sufficient force to deflect spring retention elements 528a, 528b in the distal insertion position when on-skin sensor assembly 508 is in contact with skin of the host, allowing second spring 526 to clear spring retention elements 528a, 528b and drive needle carrier assembly 516 in the proximal direction, thereby maintaining needle carrier assembly 516, needle hub 518 (see FIGS. 7-9) and insertion element 520 (see FIGS. 7-9) in a locked, retracted position even in the event of a dry fire.

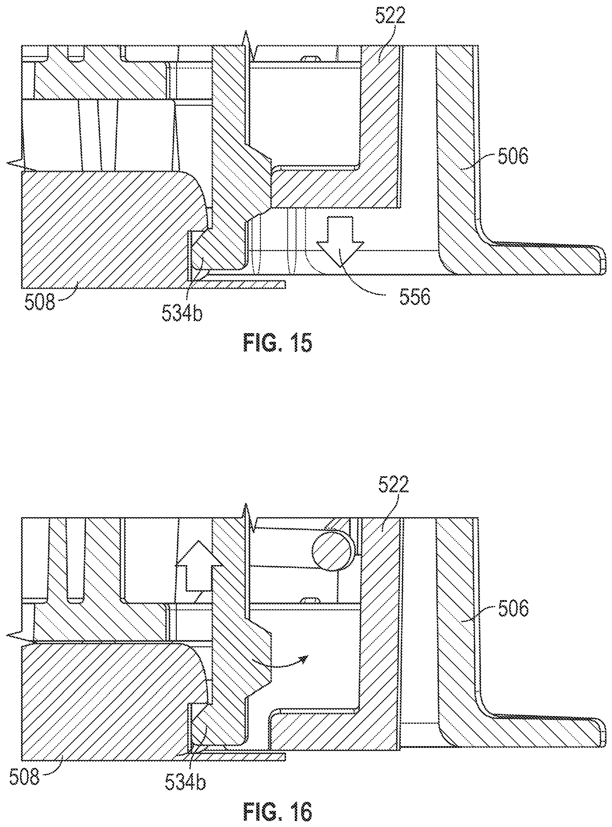

FIGS. 15 and 16 illustrate magnified views of some features of an applicator, such as applicator 500, according to some examples.

In FIG. 15, first spring 524 (see FIGS. 6-12) is driving holder 522, as well as the needle carrier assembly and on-skin sensor assembly 508 in the distal direction, illustrated by arrow 556, toward the distal insertion position. Retention element 534b of the needle carrier assembly is releasably coupled to on-skin sensor assembly 508. As illustrated, during insertion and near the distal inserted position, holder 522 is in contact with wearable retention element 534b, preventing wearable retention element 534b from deflecting laterally and thereby rigidly securing on-skin sensor assembly 508 to the needle carrier assembly.

In FIG. 16, second spring 526 (see FIGS. 6-12) is driving needle carrier assembly 516 in the proximal direction from the distal insertion position. Because holder 522 has been driven sufficiently in the distal direction, at the distal insertion position, holder 522 is no longer in contact with wearable retention element 534b. Accordingly, wearable retention element 534b is free to deflect laterally, thereby releasing on-skin sensor assembly 508 from wearable retention element 534b and thus from the needle carrier assembly 516. Needle carrier assembly 516 is now driven in the proximal direction by second spring 526, while on-skin sensor assembly 508 is secured to the skin of the host. Moreover, in some examples, because holder 522 is driven to the distal inserted position and substantially held in that position by first spring 524, holder 522 may press against one or both of on-skin sensor assembly 508 or an adhesive patch of on-skin sensor assembly 508, supporting one or both during attachment to the skin of the host.

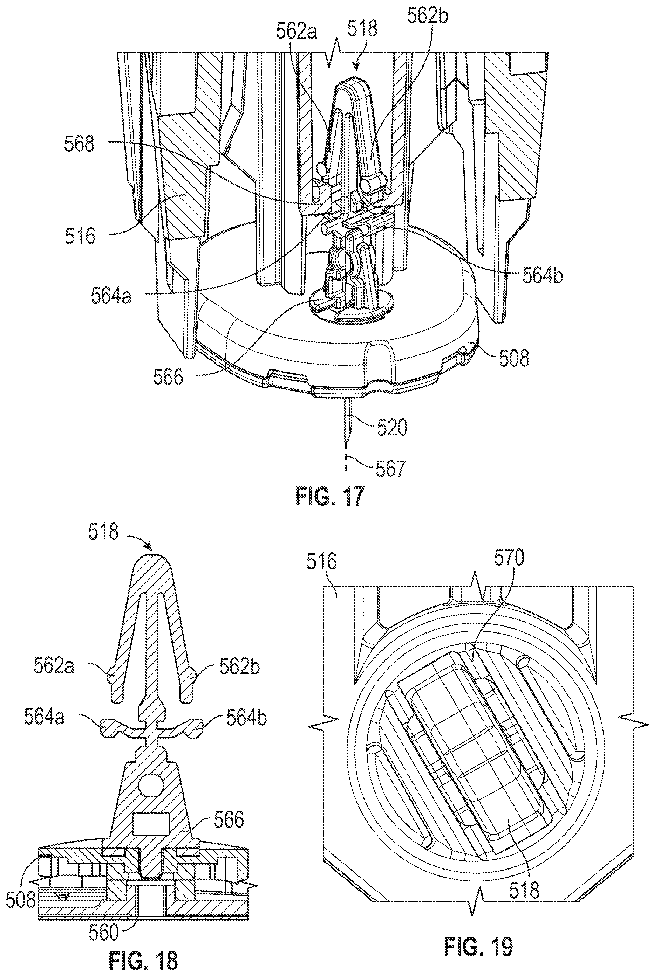

FIG. 17 illustrates a perspective partial cutaway view of needle carrier assembly 516, needle hub 518, and on-skin sensor assembly 508 of applicator 500 of FIGS. 5 and 6, according to some examples. FIG. 18 illustrates a cross-sectional view of needle hub 518 and on-skin sensor assembly 508, according to some examples. FIG. 19 illustrates a top view of a portion of needle carrier assembly 516 and needle hub 518, according to some examples. The following is a description of these features with reference to FIGS. 17-19.

On-skin sensor assembly 508 comprises sensor assembly opening 560. Needle hub 518 is configured to couple insertion element 520 to needle carrier assembly 516 and to substantially maintain a desired orientation of insertion element 520 during insertion of the sensor of on-skin sensor assembly 508 into the skin of the host.

Needle hub 518 comprises a plurality of upper arms 562a, 562b, a plurality of lower arms 564a, 564b, and a base 566. Although two upper arms and two lower arms are illustrated, any number of arms, including a single upper and lower arm, are contemplated. In some examples, upper arms 562a, 562b and lower arms 564a, 564b may be flexible such that, when needle hub 518 is coupled to needle carrier assembly 516, upper arms 562a, 562b and lower arms 564a, 564b secure needle hub 518 in a desired orientation with respect to needle carrier assembly 516. For example, upper arms 562a, 562b may be configured to flex radially inward, such that when disposed through a carrier aperture 568 in needle carrier assembly 516, upper arms 562a, 562b are in contact with an upper surface of needle carrier assembly 516 adjacent to carrier aperture 568 and lower arms 564a, 564b are in contact with a lower surface of needle carrier assembly 516 adjacent to carrier aperture 568. Such an arrangement allows a compliant fit between needle carrier assembly 516 and needle hub 518 where lower arms 564a, 564b deflect to allow upper arms 562a, 562b to expand after clearing surface of carrier aperture 568. The lower arms 564a, 564b can partially or fully relax to bias the needle hub in a distal direction and decrease the clearance between the needle hub and the needle carrier that would otherwise exist with a non-compliant fit. In addition, upper arms 562a, 562b and lower arms 564a, 564b also help to maintain contact between base 566 and a top surface of on-skin sensor assembly 508.

Base 566 comprises an anti-rotation feature. The anti-rotation feature may comprise a key having a shape complementary to at least a portion of sensor assembly opening 560 of on-skin sensor assembly 508 and may be configured to substantially prevent needle hub 518 from rotating about an axis 567 parallel to insertion element 520 with respect to on-skin sensor assembly 508, e.g., to prevent rotation of base 566 within sensor assembly opening 560. In addition, or the alternative, the upper surface of needle carrier assembly 516 adjacent to carrier aperture 568 may comprise a groove 570 configured to accept upper arms 562a, 562b when upper arms 562a, 562b are disposed through carrier aperture 568 in an orientation complementary to an orientation of groove 570, as illustrated in FIG. 19, thereby immobilizing needle hub 518 with respect to needle carrier assembly 516.

In some examples, base 566 further comprises a substantially flat surface configured to mate with a top surface or proximal surface of on-skin sensor assembly 508 and maintain insertion element 520 in a substantially perpendicular orientation to the top surface of on-skin sensor assembly 508, in some cases, when the anti-rotation feature of base 566 is engaged within an opening 560 of on-skin sensor assembly 508.

Based at least upon the above-described features of needle hub 518, on-skin sensor assembly 508, and/or needle carrier assembly 516, base 566 allows easy assembly during manufacture, including but not limited to proper alignment and preassembly of insertion element 520 onto on-skin sensor assembly 508, and/or the ability to easily engage an assembly of needle hub 518, insertion element 520, sensor and on-skin sensor assembly 508 to other portions of assembled applicator 500.

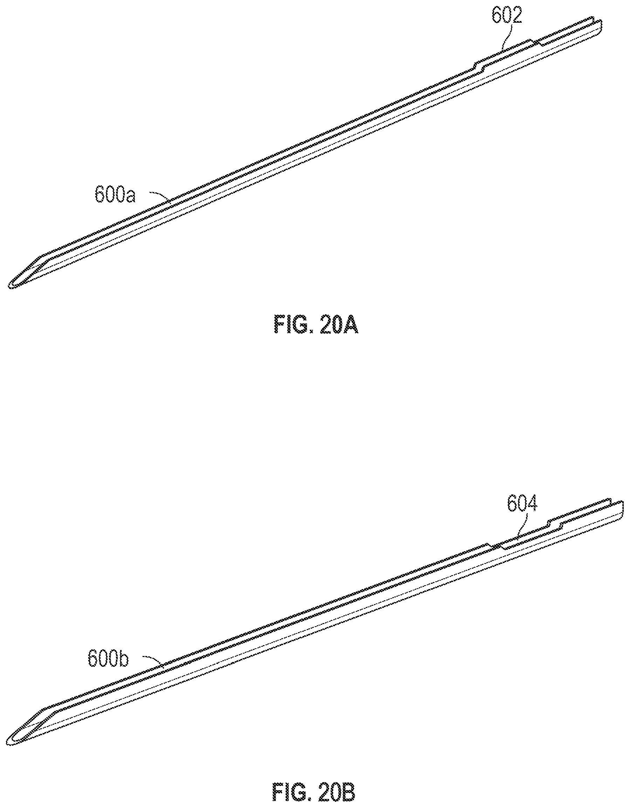

FIGS. 20A and 20B illustrate perspective views of locking features for insertion elements in the form of needles 600a, 600b for use in an applicator for an analyte sensor system, according to some examples. For example, needle 600a of FIG. 20 comprises a locking feature comprising a ridge 602 configured to mate with a complementary-shaped feature within needle hub 518, for example. In the alternative, needle 600b of FIG. 20B comprises a locking feature comprising a groove 604 configured to mate with a complementary-shaped feature within needle hub 518, for example.

In yet another alternative, any insertion element described in this disclosure may comprise a locking feature that heat stakes the selected insertion element to needle hub 518, for example. In yet another alternative, any insertion element described in this disclosure may comprise a locking feature comprising one or more friction-fit or snap-fit elements securing the selected insertion element to needle hub 518, for example. In yet another alternative, any insertion element described in this disclosure may comprise a locking feature comprising complementary clamshell elements on the selected insertion element and needle hub 518, for example, configured to mate with one another. In yet another alternative, any insertion element described in this disclosure may comprise a locking element comprising one or more inserted molded elements configured to couple the selected insertion element to needle hub 518, for example.

During manufacture, applicator 500 may be assembled in stages. For example, and not limitation, if present, first barrier layer 512 may be affixed to inner housing 506. Insertion element 520 may be coupled to needle hub 518, which may then be coupled to on-skin sensor assembly 508. Second spring 526 may be placed into holder 522 or needle carrier assembly 516 and then needle carrier assembly 516 may be disposed into holder 522 and attached to needle hub 518 and to on-skin sensor assembly 508 via wearable retention elements 534a, 534b. First spring 524 may be disposed in holder 522, which may then be installed into inner housing 506. Inner housing 506 may be inserted into and secured to outer housing 504. If present, second barrier layer 530 may be affixed to inner housing 506. If a separate element, activation element 502 may then be disposed into outer housing 504. Any labeling, sterilizing and/or packaging may then be applied to applicator 500.

FIGS. 21-23 illustrate several cross-sectional views, and various features and operating positions, of yet another applicator 700 for an on-skin sensor assembly of an analyte sensor system, according to some examples.

Applicator 700 may include outer applicator housing 504 comprising activation element 502. Outer applicator housing 504 may be configured to translate in a distal direction under force applied by a host of applicator 700, thereby aligning activation element 502 in a position that allows applicator 700 to fire, an alignment illustrated by FIG. 21. As previously described in connection with applicator 500, in some examples, activation element 502 may be disposed in any location, e.g., a top, upper side, lower side, or any other location of applicator 700.

Applicator 700 further comprises inner housing 506, configured to house one or more mechanisms utilized to apply on-skin sensor assembly 508 to skin of a host. Distal surface 510 of a bottom opening of inner housing 506 may define a bottom surface of applicator 700. In some examples, upon pressing applicator 700 against the skin of the host, the skin may deform in a substantially convex shape at distal surface 510 such that at least a portion of a surface of the skin disposed at the bottom opening of inner housing 506 extends into the bottom opening of inner housing 506, in a proximal direction, beyond a plane defined by distal surface 510.

Although not illustrated in FIGS. 21-23, inner housing 506 may comprise a spring 536 configured to contact outer housing 504 and maintain a predetermined spacing between outer housing 504 and inner housing 506 in the pre-activation orientation (see FIG. 7). Spring 536 may be a compression spring, leaf spring, flex arm spring, a piece of foam or rubber, etc. In some other examples, outer housing 504 may comprise spring 536 and spring 536 may be configured to contact inner housing 506.

Applicator 700 may further comprise a needle carrier assembly 702. Needle carrier assembly 702 comprises wearable retention and/or alignment elements 534a, 534b configured to pass through holder 704 and releasably couple on-skin sensor assembly 508 to holder 704 and/or to needle carrier assembly 702. Although two wearable retention and/or alignment elements are illustrated, any number of wearable retention and/or alignment elements are contemplated.

Applicator 700 further comprises needle hub 518 configured to couple insertion element 520 to needle carrier assembly 702. Insertion element 520 is configured to insert sensor of on-skin sensor assembly 508 into skin of the host. In some examples, insertion element 520 comprises a needle, for example, an open sided-needle, a needle with a deflected-tip, a curved needle, a polymer-coated needle, a hypodermic needle, or any other suitable type of needle or structure. In yet other examples, insertion element 520 may be integrally formed with sensor, in which insertion element 520 may be sufficiently rigid to be inserted partially into skin of the host with minimal or no structural support.

Applicator 700 may further include holder 704 releasably coupled to needle carrier assembly 702 and configured to guide on-skin sensor assembly 508 while coupled to needle carrier assembly 702, e.g., at least during translation from a proximal position to a distal insertion position. As previously described in connection with applicator 500, on-skin sensor assembly 508 may be stripped or released from holder 704 and/or needle carrier assembly 702 once on-skin sensor assembly 508 is disposed on the skin of the host.

Applicator 700 may further comprise an insertion assembly configured to translate insertion element 520, needle hub 518, and needle carrier assembly 702 from a proximal position, in the distal direction, to a distal insertion position. Such an insertion assembly may include first spring 524. First spring 524 may be a compression spring, or any suitable type of spring, and may have its first end in contact with or coupled to inner applicator housing 506 and its second end in contact with or coupled to holder 704. First spring 524 is configured to, upon activation of the insertion assembly, translate holder 704, needle carrier assembly 702, needle hub 518, insertion element 520 and on-skin sensor assembly 508, in the distal direction to the distal insertion position. Substantially at the distal insertion position, needle carrier assembly 702 may decouple from holder 704 and on-skin sensor assembly 508.

Applicator 700 may further comprise a retraction assembly configured to translate needle carrier assembly 702, needle hub 518 and insertion element 520, in the proximal direction, from the distal insertion position to a proximal retracted position. In some examples the initial proximal position may be the same as the proximal retracted position. In other examples, the initial proximal position may be different from the proximal retracted position. Such a retraction assembly may include a second spring 706. Second spring 706 may be a compression spring, or any suitable type of spring, and may have a first end contacting or coupled to holder 704 and a second end, comprising a tang 708 (e.g., a spring portion or spring end) disposed substantially along a diameter of second spring 706, in contact with or coupled to a spring retention element 710 of holder 704, at least until retraction. Spring retention element 710 may comprise, e.g., an arm, a deflection element, a tab, a detent, a snap or any other feature capable of a retaining function. Spring retention element 710 may have substantially the same form and function as spring retention elements 528a, 528b of applicator 500 except as described below. Second spring 706 is configured to translate needle carrier assembly 702, needle hub 518, and insertion element 520 in the proximal direction from the distal insertion position to the proximal retracted position. Tang 708 of second spring 706 is released from spring retention element 710 in the distal insertion position when spring retention element 710 is not backed up by backstop element 712 and in response to tang 708 of second spring 706 pushing against spring retention element 710 with a force exceeding a predetermined threshold sufficient to overcome and deflect spring retention element 710.

Needle carrier assembly 702 further comprises a backstop feature 712, configured to prevent lateral motion of spring retention element 710 of holder 704 in at least the proximal pre-activation position, thereby supporting retention of second spring 706 between spring retention element 710 and holder 704 until retraction. In the orientation shown in FIG. 21, second spring 706 is exerting a force against spring retention element 710 but backstop feature 712 prevents lateral deflection of retention element 710.

Holder 704 further comprises needle carrier retention element 542, which may comprise a deflectable arm, rigid arm, deformable feature, snap, catch, or hook. Upon needle carrier assembly 702 reaching the proximal retraction position after activation, needle carrier retention element 542 is configured to engage with needle carrier assembly 702, thereby maintaining needle carrier assembly 702, needle hub 518 and insertion element 520 in a locked, retracted position, limiting access to insertion element 520.

Although not illustrated in FIGS. 21-23, inner housing 506 of applicator 700 may further comprise engagement element 550 and needle carrier assembly 702 may further comprise protrusion 552 and may function substantially as previously described in connection with at least FIGS. 10-12.

Although not illustrated in FIGS. 21-23, inner housing 506 of applicator 700 may further comprise a protrusion extending from inner housing 506 in the distal direction, substantially as previously described protrusion 548. Similar to that previously described in connection with FIG. 13, this protrusion may be configured to contact at least one of spring retention element 710 and backstop feature 712 in the pre-activation state such that spring retention element 710 is prevented from laterally deflecting until holder 704 and needle carrier assembly 702 have translated at least a predetermined minimum distance in the distal direction. Accordingly, the protrusion may provide a measure of drop protection such that applicator 700 may not prematurely fire in response to a concussive shock from being dropped before activation.

Applicator 700 functions substantially similarly to applicator 500 with the exception that instead of utilizing spring retention elements 528a, 528b, which are disposed along an outside of second coil of spring 526 and are configured to contact and retain a coil of second spring 526, applicator 700 utilizes spring retention element 710, which is disposed along an inside of second spring 706 and is configured to contact and retain tang 708 of second spring 706 along a diameter of second spring 706. Disposing spring retention element 710 within and substantially along a center of second spring 706, as opposed to along an outside of second spring 706, further ensures that spring retention element 710 does not contact the coils of second spring 706 as second spring 706 extends during retraction, thereby smoothing the operation of applicator 700. In addition, the arrangement including spring retention element 710, as opposed to spring retention elements 528a, 528b mitigates the risk of, and difficulty ensuring that, multiple spring retention elements trigger or are overcome at substantially the same time.

FIG. 21 illustrates a state of applicator 700 prior to activation, according to some examples. Holder 704, needle carrier assembly 702, needle hub 518, insertion element 520, on-skin sensor assembly 508, first spring 524 and second spring 526 are all shown in pre-activation positions.

Retention element 532 of holder 704 is in contact with inner housing 506, thereby immobilizing holder 704, and therefore also needle carrier assembly 702, needle hub 518, insertion element 520 and on-skin sensor assembly 508, in the pre-activated state.

Backstop feature 712 of needle carrier assembly 702 is in contact with and prevents spring retention element 710 from deflecting laterally, thereby ensuring spring retention element 710 retains tang 708 of second spring 706 in the loaded or pre-activation position shown.

Activation of applicator 700 may include a host pressing applicator 700 against their skin with sufficient force to translate outer housing 504 in a distal direction toward and with respect to inner housing 506 until activation element 502 is aligned with insertion assembly retention element 532 of holder 704, as shown in FIG. 21. Once such an alignment is achieved, a host may initiate activation element 502, thereby deflecting insertion assembly retention element 532 sufficiently to release holder 704 from inner housing 506. In some other examples, applicator 700 may be configured such that activation element 502 may be activated first, but that actual insertion is not triggered until outer housing 504 is translated sufficiently in the distal direction toward and with respect to inner housing 506. In yet other examples, activation element 502 may be biased toward a center of applicator 700 such that activation element 502 need not be explicitly activated by the host but, instead, activation element 502 may be configured to automatically initiate insertion upon outer housing 504 being translated sufficiently in the distal direction toward and with respect to inner housing 506.

FIG. 22 illustrates applicator 700 after activation and during insertion, according to some examples. First spring 524 drives holder 704, and so needle carrier assembly 702, needle hub 518, insertion element 520, and on-skin sensor assembly 508, in the distal direction toward the distal insertion position. FIG. 22 illustrates on-skin sensor assembly 508 in contact with skin of the host but where holder 704 is not yet fully driven, by first spring 524, into contact with on-skin sensor assembly 508 or skin of the host

In some examples, masses of each of holder 704, needle carrier assembly 702, needle hub 518, insertion element 520, and on-skin sensor assembly 508 may be specifically designed to reduce or substantially eliminate a tendency of needle carrier assembly 702, needle hub 518, insertion element 520, and on-skin sensor assembly 508 to detach from holder 704 while being driven in the distal direction during insertion. In some examples, a force exerted by first spring 524 may further be selected to be sufficient for proper operation of applicator 700, while not so large as to further exacerbate such above-described inertially triggered detachment. In some examples, a spring (not shown) may be configured to exert a force against a portion of needle carrier assembly 702, for example in the distal direction, sufficient to prevent needle carrier assembly 702 from inertially triggered detaching from holder 704 during insertion.

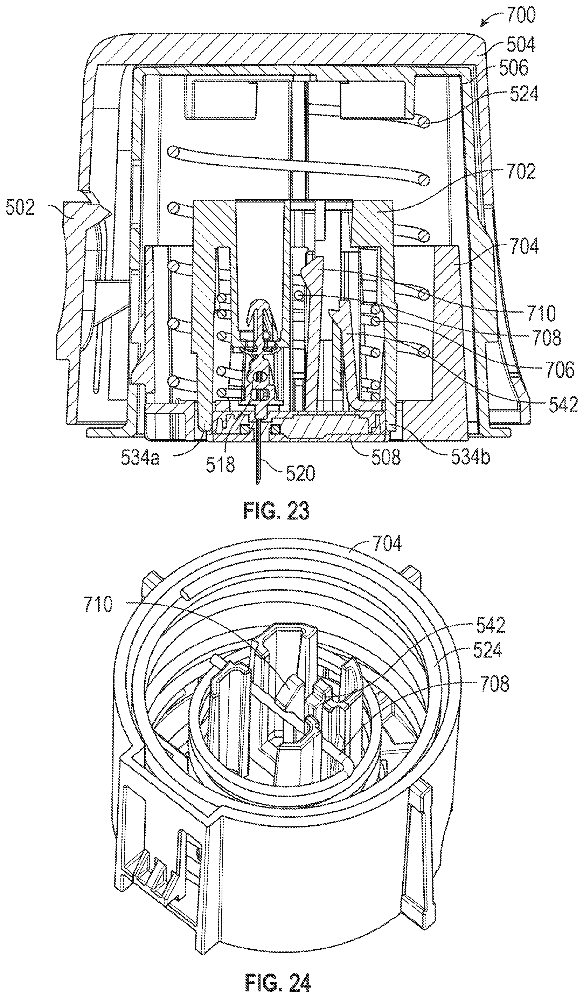

FIG. 23 illustrates the applicator 700 after activation and at or near the distal insertion position, according to some examples. First spring 524 has driven holder 704, needle carrier assembly 702 and on-skin sensor assembly 508 in the distal direction to the distal inserted position. Since first spring 524 has driven holder 704 a short distance farther in the distal direction than needle carrier assembly 702, backstop feature 712 is no longer in contact with spring retention element 710, allowing second spring 706 (e.g. tang 708) to laterally deflect spring retention element 710, thereby releasing second spring 706, which drives needle carrier assembly 702 in the proximal direction. Alternatively, similar to that described above in connection with applicator 500 in FIG. 13, where the angle θ of the portion of spring retention element 710 in contact with tang 708 of second spring 706 is substantially 90° (e.g., flat), spring retention element 710 may be biased to automatically deflect sufficiently to release second spring 706 once backstop feature 712 is no longer in contact with spring retention element 710, thereby freeing second spring 706 to drive needle carrier assembly 702 in the proximal direction. Although not shown in FIGS. 21-23, inner housing 506 may further comprise engagement element 550 configured to engage with a protrusion 552 of needle carrier assembly 702, and to function substantially as previously described in connection with at least FIGS. 10-12. In some examples, a stop feature (not shown) may be disposed at a bottom of applicator 700, e.g., on a distal portion of inner housing 506. Such a stop feature may be configured to contact one or more of on-skin sensor assembly 508, needle carrier assembly 702, or holder 704 in the distal insertion position.

Upon release of second spring 706, second spring 706 is configured to drive needle carrier assembly 702, needle hub 518 and insertion element 520, in the proximal direction. Although not shown in FIG. 23, as needle carrier assembly 702 travels to the proximal retracted position, needle carrier retention element 542 may engage with needle carrier assembly 702, thereby retention needle carrier assembly 702, needle hub 518 and insertion element 520, in a locked, retracted position limiting access to insertion element 520.

FIG. 24 illustrates a perspective view of holder 704, first spring 524 and second spring 706 of applicator 700, according to some examples. FIG. 24 illustrates spring retention element 710 and retention tang 708 of second spring 706 in an orientation within applicator 700 before retraction.

During manufacture, applicator 700 may be assembled in stages. For example, and not limitation, if present, as previously described in connection with applicator 500, first barrier layer 512 (see FIG. 6) may be affixed to inner housing 506. insertion element 520 may be coupled to needle hub 518, which may then be coupled to on-skin sensor assembly 508. Second spring may be placed into holder 704 or needle carrier assembly 702 and then needle carrier assembly 702 may be disposed into holder 704 and attached to needle hub 518 and to on-skin sensor assembly via wearable retention elements 534a, 534b. First spring 524 may be disposed in holder 704, which may then be installed into inner housing 506. Inner housing 506 may be inserted into and secured to outer housing 504. If present, as previously described in connection with applicator 500, second barrier layers 530 (see FIG. 6) may be affixed to inner housing 506. If a separate element, activation element 502 may then be disposed into outer housing 504. Any labeling, sterilizing and/or packaging may then be applied to applicator 700.

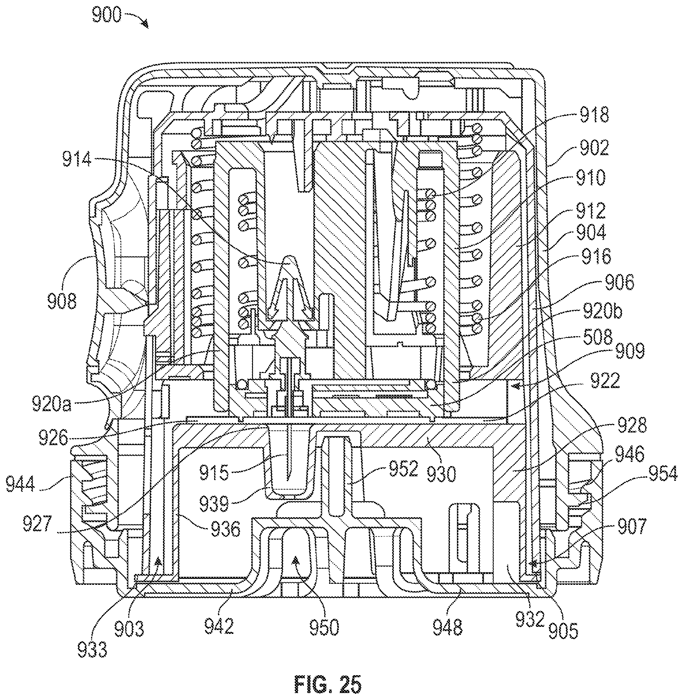

In examples, applicator systems may include a cap and/or a liner removal component. FIG. 25, for example, illustrates an example of an applicator 900 having an applicator housing 902 configured to retain the on-skin wearable medical device, and a deployment mechanism configured to deploy the on-skin wearable medical device to the skin. The applicator housing 902 may be configured similarly as in examples of applicators disclosed herein, including having an outer housing 904 and an inner housing 906 as disclosed in regard to the examples of FIGS. 5-24. The outer housing 904 for example, may be configured similarly as the outer housing 504 and the inner housing may be configured similarly as the inner housing 506. The applicator housing 902 may be configured to be gripped by a user in examples. Various other configurations of applicator housings may be utilized as desired.

The applicator housing 902 may include an internal cavity 903 for retaining the on-skin wearable medical device. The housing 902 may include an opening 905 at an end portion 907 of the internal cavity 903 for the on-skin wearable medical device to be deployed from. The internal cavity 903 may include a proximal end portion 909 that may include the on-skin wearable medical device coupled to a needle carrier assembly.

The deployment mechanism may be configured similarly as other forms of deployment mechanisms disclosed herein. The deployment mechanism may be configured similarly as the deployment mechanisms disclosed in regard to the examples of FIGS. 5-24. For example, the deployment mechanism may include one or more retention element(s) for retaining the on-skin wearable medical device and releasing the on-skin wearable medical device from the housing 902 to the skin in examples. The deployment mechanism may include an insertion assembly for inserting at least a portion of the on-skin wearable medical device into the skin. The insertion assembly may insert an insertion element (e.g., a needle) into the skin. The deployment mechanism may drive the insertion element to the skin upon the deployment mechanism deploying the on-skin wearable medical device to skin. The deployment mechanism may include a retraction assembly for retracting the insertion element from the skin. Other forms of deployment mechanisms may be utilized in examples as desired.

The applicator 900 may include an activation element 908 that may operate similarly as the activation element 502. The applicator 900 may include a needle carrier assembly 910 that may operate similarly as the needle carrier assembly 516. The applicator 900 may include a holder 912 that may operate similarly as the holder 522. The applicator 900 may include a hub (e.g., a needle hub 914) that may operate similarly as the needle hub 518. The applicator 900 may include an insertion element 915 (e.g., a needle) that may operate similarly as the insertion element 520. The applicator 900 may include springs 916, 918 that may operate similarly as the springs 524, 526 respectively. The applicator 900 may include retention elements 920a, b that may operate similarly as the retention elements 534a, 534b respectively. Additional components of the applicators shown in FIGS. 5-24 may be utilized with the applicator 900. The applicator 900 may operate in a similar manner and provide similar function as the applicators shown in FIGS. 5-24,

The applicator 900 may include a cap 942 that may be positioned at a distal portion of the applicator housing 902 and may cover the distal opening 905 of the internal cavity 903. The cap 942 may include a grip portion 944 on an exterior surface of the cap 942 and an engagement portion 946 on an interior surface of the cap 942. The cap 942 may include a central portion 948 that covers and spans the distal opening 905 of the internal cavity. The cap 942 may comprise an exterior lid for the applicator 900 upon transport and unpackaging of the applicator 900.

The central portion 948 of the cap 942 may include one or more openings 950 that may allow a sterilizing material such as sterilizing gas to pass through, to sterilize internal components of the applicator 900. The central portion 948 may include a central support 952 that may be configured to press against a liner removal component 928 to retain the liner removal component 928 in position. The central support 952 may be configured to rotate upon uncoupling or unscrewing of the cap 942 from the applicator housing 902.

The engagement portion 946 may comprise threading or another form of engagement portion 946 for engaging a corresponding engagement portion 954 on an exterior surface of the housing 902. The engagement portion 946 may be configured to be rotated relative to the applicator housing 902 to unscrew from the housing 902 and allow for release of the liner removal component 928 from the applicator housing 902.

The applicator 900 may include a liner removal component 928. The liner removal component 928 may be configured to engage a liner 926 positioned on an engaging surface of the patch 922 and remove the liner 926 from the engaging surface of the on-skin wearable medical device upon being withdrawn from the engaging surface of the on-skin wearable medical device. The liner removal component 928 may include an engaging surface 930 for engaging the liner 926. The engaging surface 930 may be a flattened surface that may extend parallel with the liner 926. The engaging surface 930 may include an opening 927 configured to allow the insertion element 915 to pass through. The liner removal component 928 may further include a sheath 939 configured to cover the insertion element 915. The liner removal component 928 may further include a raised portion 936 that may extend from a distal portion 932 of the liner removal component 928. The raised portion 936 may extend axially within the internal cavity 903.

The distal portion 932 of the liner removal component 928 may include a flange 933 for grip by a user to remove the liner removal component 928 from the internal cavity 903 and accordingly remove the liner 926 from the engaging surface of the on-skin wearable medical device. In examples, the flange 933 may be excluded from use.

The liner 926 may be positioned on an engaging surface of the patch in examples. The liner may cover the engaging surface and may protect the engaging surface from damage, deterioration, or other adverse effects. The liner, for example may comprise a sheet of material that covers the engaging surface of the patch. The liner may have a proximal surface contacting the engaging surface of the patch and a distal surface facing opposite the proximal surface. The liner in examples, may be configured to reduce the possibility of an exposed engaging surface from deteriorating or otherwise losing adhesive properties prior to deployment. For example, during a sterilization process using a gas or other sterilizing material, the liner may reduce the possibility of the engaging surface deteriorating. A sterilizing gas may comprise ethylene oxide (EtO) or another form of sterilizing gas as desired. The liner, however, is to be removed from the engaging surface prior to deployment of the on-skin sensor assembly to the skin.

The applicator 900 may be utilized to deploy an on-skin wearable medical device to skin. The on-skin wearable medical device may comprise the on-skin sensor assembly 508 shown in FIG. 6, for example, which may include a housing, an analyte sensor coupled to the housing, an electronics unit, and a patch 922. The on-skin sensor assembly may have forms as shown in FIGS. 2A-4, for example, or other forms as desired.

The cap 942 and the liner removal component 928 may be removed prior to deployment of the on-skin wearable medical device to skin.

Upon activation, an applicator as disclosed herein may insert the analyte sensor into the skin of a host by utilizing an insertion element (such as insertion element 915).

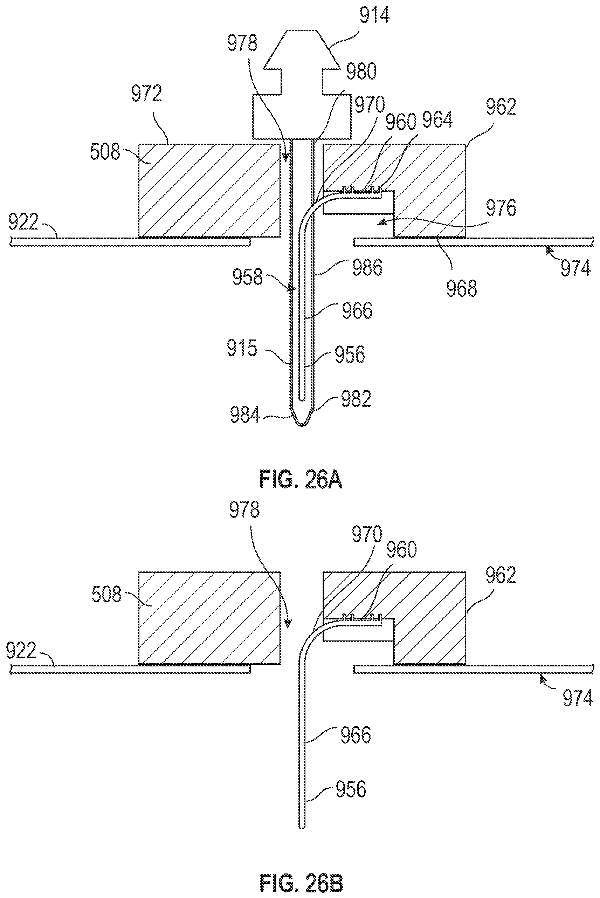

Referring to FIG. 26A, the insertion element 915 may drive the analyte sensor 956 of the on-skin sensor assembly 508 into the host's skin by the analyte sensor 956 extending along a channel 958 of the insertion element 915.

The analyte sensor 956, for example, may include a first portion 960 or contact portion that may be coupled to the housing 962 of the on-skin sensor assembly 508. The first portion 960, for example, may include electrical contacts 964 that may electrically connect to electrical terminals of the on-skin sensor assembly 508 or another component of the on-skin sensor assembly 508. Electrical terminals may be positioned on an interface board or circuit board, or another component of the on-skin sensor assembly 508 as desired. Other methods of coupling between the first portion 960 and the housing 962 may be utilized as desired.

The analyte sensor 956 may include a second portion 966 including a sensing portion that may be configured to be inserted into or through the skin of a host and positioned in or under the skin. The second portion 966, in examples, may extend distally from a distal surface 968 of the housing 962 and may be guided by the insertion element 915 into the skin of the host. The second portion 966 may be straight and may be axially aligned with an opening 978 for the insertion element 915 to pass through, as shown in FIG. 26A.

The analyte sensor 956 may comprise an elongate analyte sensor. The second portion 966 may extend distally to be positioned within the skin layers of the host. In examples, the second portion 966 of the analyte sensor 956 may extend perpendicular with respect to the distal surface 968 of the housing 962. In examples, other angles may be utilized as desired. The second portion 966 may extend perpendicular with respect to the first portion 960 of the analyte sensor 956. In examples, other angles may be utilized as desired.

A bend 970 may angle the second portion 966 of the analyte sensor 956 with respect to the first portion 960 of the analyte sensor 956. The bend 970, for example, may be positioned between the second portion 966 and the first portion 960 and may have a continuous curvature as shown in FIG. 26A or may have another form as desired. The bend 970 may angle the second portion 966 with respect to the first portion 960 at a perpendicular angle or another angle as desired. The bend 970 may be axially aligned with an opening 978 for the insertion element 915 to pass through, as shown in FIG. 26A. Other forms of analyte sensors 956 may be utilized as desired.

The housing 962 of the on-skin sensor assembly 508 may be configured similarly as other forms of housing disclosed herein. The housing 962 may be configured to be worn on the skin of the host. The housing 962 may include the distal surface 968, which may be configured to face towards the host's skin. The patch 922 may be positioned on the distal surface 968 of the housing 962. The patch 922 may include the engaging surface 974 for engaging the skin of the host. The engaging surface 974 may comprise an adhesive surface in examples or another form of a surface.

The housing 962 may include a proximal surface 972 facing opposite the distal surface 968. The proximal surface 972 may extend parallel with the distal surface 968 or may have another configuration as desired.

The housing 962 may include a cavity 976 that may receive the first portion 960 of the analyte sensor 956 in examples. The cavity 976 may have a variety of forms as desired. For example, the cavity 976 may be configured to retain an adhesive (which may comprise a liquid adhesive or curable adhesive) that may couple the first portion 960 of the analyte sensor 956 to the housing 962 in examples. The cavity 976 may include one or more dams or other features that may retain the adhesive and may be utilized to electrically isolate portions of the analyte sensor 956 from each other if desired. In examples, the cavity 976 may comprise a recess for the first portion 960 of the analyte sensor 956 to be inserted into, to otherwise couple with the housing 962. In examples, use of a cavity 976 may be excluded and the first portion 960 of the analyte sensor 956 may otherwise couple to the housing 962.

The housing 962 may include an opening 978 for the insertion element 915 to pass through. The opening 978 may extend through the proximal surface 972 of the housing 962 and may extend to the distal surface 968 of the housing 962. The opening 978 may be configured for the insertion element 915 to be retracted proximally through from the skin. The insertion element 915 may be retracted following penetration of the host's skin. In examples, the insertion element 915 may be positioned within the opening 978 upon insertion into the host's skin or may be passed distally relative to the opening 978 upon insertion into the host's skin. In an example as shown in FIG. 26A, the insertion element 915 may be positioned within the opening 978 and may be static relative to the opening 978 upon insertion into the host's skin. For example, as shown in FIGS. 7-8 and 21-23, the insertion element 915 may move distally along with the housing 962 of the on-skin sensor assembly 508 and may remain static relative to the housing 962 upon insertion into the host's skin. Other forms of insertion may be utilized in examples.

The insertion element 915 may include a proximal end portion 980 and a distal end portion 982 comprising a tip 984 of the insertion element 915. The tip 984 may comprise a sharpened tip in examples, and may be configured to puncture the host's skin and be inserted into the host's skin.

The needle hub 914 may be positioned at the proximal end portion 980 of the insertion element 915. The needle hub 914 may be in contact with the proximal surface 972 of the housing 962 or may be spaced from the proximal surface 972 as desired.

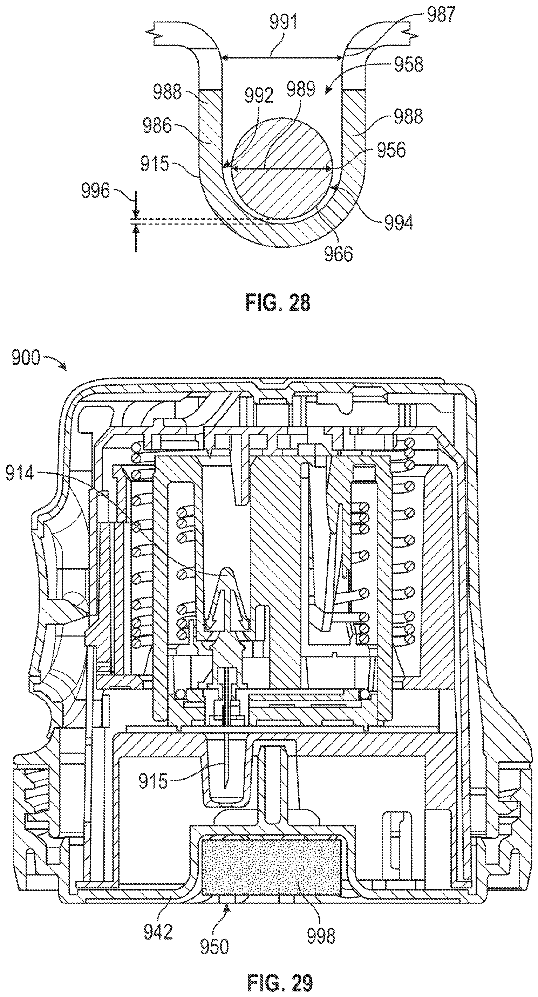

The insertion element 915 may comprise an elongate insertion element 915, and may include a shaft 986 that may extend between the proximal end portion 980 and the distal end portion 982. The shaft 986 may be straight or have a linear shape and may be configured to guide the analyte sensor 956 into the skin of the host. For example, the shaft 986 may have a channel 958 that may receive the analyte sensor 956. Referring to FIG. 28, the shaft 986 has an opening 987 for the channel 958 that the analyte sensor 956 is configured to be positioned in. A portion of the analyte sensor 956 (e.g.. the second portion 966 or sensing portion) may be positioned within the channel 958 and may be bound by side walls 988 (marked in FIG. 28) of the insertion element 915. The side walls 988 may be positioned on the sides of the channel 958. The shaft 986 may extend along the portion (e.g., the second portion 966 or sensing portion) of the analyte sensor 956. The shaft 986 may extend parallel along the portion in examples. The second portion 966 including the sensing portion of the analyte sensor 956 accordingly may extend along the shaft 986.

The analyte sensor 956 may be positioned within the channel 958 such that as the insertion element 915 is inserted into the host's skin the analyte sensor 956 may be inserted along with the insertion element 915 and may be guided into the host's skin. The shaft 986 may be inserted into the skin to guide the portion (e.g., the second portion 966 or sensing portion) into the skin. The channel 958 may create a space within the host's skin for the analyte sensor 956 to be inserted into. Upon retraction of the insertion element 915, the analyte sensor 956 may remain within the host's skin. In examples, other forms of insertion may be provided, for example, the insertion element 915 may lack a channel 958 in examples and the analyte sensor 956 may extend along an outer surface of the insertion element 915 for insertion into the host's skin.

The channel 958 may have a C-shaped cross-section in examples (e.g. FIG. 28) or may have another cross-section as desired.

FIG. 26B illustrates the insertion element 915 having been withdrawn by a retraction assembly of the applicator or by another method. The analyte sensor 956 may remain positioned within the skin of the host and may sense an analyte of the host continuously for a period of days.

As used throughout this specification, and unless specified otherwise, the term friction may comprise a kinetic friction and/or may comprise a static friction or stiction between the insertion element 915 and the analyte sensor 956. Friction may exist between the insertion element 915 and the analyte sensor 956 that may be beneficial. After the analyte sensor 956 is positioned within the channel 958 during manufacture, friction between the insertion element 915 and the analyte sensor 956 can help maintain the positioning of the analyte sensor 956 therein, and reduce the likelihood that the analyte sensor 956 undesirably dislodges from the channel 958 during transportation and/or handling prior to insertion. During insertion, friction may also beneficially maintain the position of the analyte sensor 956 within the channel 958 as the insertion element 915 pierces and guides the analyte sensor 956 into the host's skin.

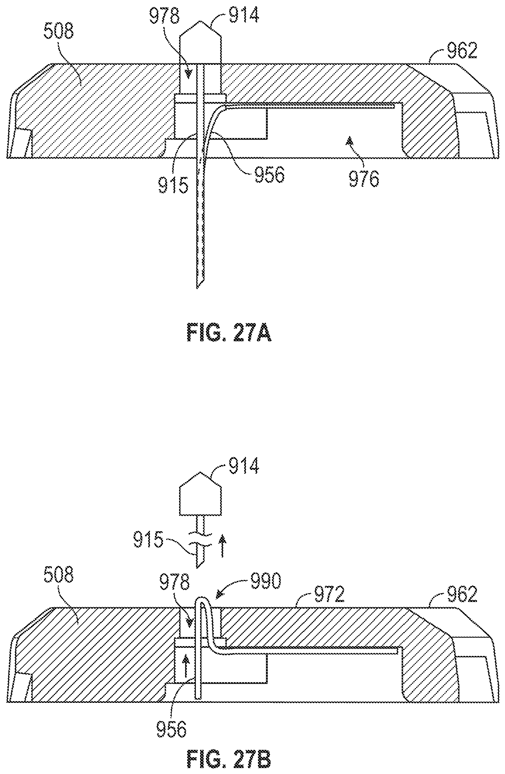

Friction may exist between the insertion element 915 and the analyte sensor 956 that may produce adverse results. Referring to FIG. 27A, for example, the analyte sensor 956 may be positioned within the channel of the insertion element 915 prior to and during insertion into the host's skin. If too high of a level of friction exists between the analyte sensor 956 and the insertion element 915 then upon retraction of the insertion element 915 (as shown in FIG. 27B) the analyte sensor 956 may also be retracted proximally. In particular, if too high of a level of stiction exists between the analyte sensor 956 and the insertion element 915 then upon retraction of the insertion element 915 (as shown in FIG. 27B) the analyte sensor 956 may also be retracted proximally. The analyte sensor 956 may retract proximally to possibly be withdrawn entirely from the host's skin or may be withdrawn partially from the host's skin. The retraction of the analyte sensor 956 may reduce the ability of the analyte sensor 956 to properly sense the analyte within the host's body due to a mispositioning of the analyte sensor 956, or result in a complete withdrawal of the analyte sensor 956 from the host's skin. Further, a bend 990 that may comprise a "U" bend may be formed in the analyte sensor 956 upon retraction of the insertion element 915. The formation of the bend 990 may disrupt the electrical signals provided by the analyte sensor 956 to the electronics of the on-skin sensor assembly 508 and may be undesirable.

The analyte sensor 956, in examples, may retract along the opening 978 of the housing 962 along with the retraction of the insertion element 915 along the opening 978. In examples, the analyte sensor 956 may retract to protrude from the proximal surface 972 of the housing 962 as shown in FIG. 27B, or otherwise undesirably retract without protrusion from the proximal surface 972.

The retraction of the analyte sensor 956 may be caused by friction (e.g., kinetic friction or stiction) between the analyte sensor 956 and an interior surface 992 (marked in FIG. 28) of the insertion element 915. It has been observed that stiction between the analyte sensor 956 and an interior surface 992 tends to be greater than the kinetic friction occurring as the insertion element 915 is retracted. Therefore, in many situations, whether the analyte sensor 956 retracts along with the insertion element 915 is determined by the level of stiction between the analyte sensor 956 and an interior surface 992. The interior surface 992 may comprise an interior surface of the insertion element 915 that defines the channel 958. In other configurations (e.g., in which the insertion element 915 does not include a channel) the interior surface 992 may comprise an exterior surface of the insertion element or another surface as desired. The interior surface 992 may have friction with an exterior surface 994 of the analyte sensor 956 or another surface in examples.

An undesirable level of friction between the analyte sensor 956 and the insertion element 915 may be produced or increased in a sterilization process applied to the analyte sensor 956 and/or the insertion element 915 and/or other components of the on-skin sensor assembly or the applicator. For example, a sterilization process may include heat applied to such components. A sterilization process may include an increased humidity applied to such components. A sterilization process may include a sterilizing gas (e.g., ethylene oxide (EtO), or another form of sterilizing gas) applied to such components. In examples, combinations of sterilizing methods may be utilized in a sterilization process. For example, a sterilization process utilizing ethylene oxide (EtO) may include applying heat, humidity, and the EtO to the analyte sensor 956 and the insertion element 915 for a duration of time.

A sterilization process may include applying the heat, humidity, and the EtO to the applicator 900 with the analyte sensor 956 and insertion element 915 positioned within the applicator housing 902. The heat, humidity, and EtO may pass through the openings 950 shown in FIG. 25 for example and/or may pass through a barrier layer covering the openings 950. The barrier layer may be moisture and/or gas permeable to allow the humidity and EtO to contact the analyte sensor 956 and insertion element 915. Other components of the applicator 900 may be sterilized. Other forms of sterilizing gas and other sterilizing methods may be utilized.

The analyte sensor 956 may be positioned within the channel 958 of the insertion element 915 during a sterilization process. For example, the analyte sensor 956 and insertion element 915 may be in a position as shown in FIG. 27A and/or FIG. 28 during a sterilization process. In examples, other configurations of analyte sensors 956 and insertion elements 915 may be utilized as desired.

A sterilization process applied to the analyte sensor 956 and/or the insertion element 915 may increase a friction between the analyte sensor 956 and the insertion element 915. For example, stiction between the analyte sensor 956 and the insertion element 915 may be increased during the sterilization process. Heat and humidity, for example, may swell a membrane of the analyte sensor 956, which may produce adhesion between the analyte sensor 956 and the insertion element 915. The adhesion may remain after a drying cycle applied to the analyte sensor 956 and the insertion element 915. Without being bound to any particular theory, the stiction may be caused by hydration of the membrane resulting in the formation of hydrogen bonding between the exterior surface 994 of the analyte sensor 956 and the interior surface 992 of the insertion element 915, or by other forms of bonding between the analyte sensor 956 and the insertion element 915 that may be due to electrical charges, chemical interactions, and/or mechanical in nature. For example, a sterilization process involving heat, humidity, and/or EtO may cause or increase hydrogen bonding between the analyte sensor 956 and the insertion element 915. Other forms of bonding (whether electrical, chemical, or mechanical) may be formed or increased as a result of a sterilization process.

An extended or escalated sterilization process may increase the possibility of undesired friction (e.g., stiction) and undesired retraction of the analyte sensor 956 after insertion. The greater the surface area contact with the insertion element 915, and the greater the membrane sensitivity of the analyte sensor 956 to heat and humidity during a sterilization process, may also increase the possibility of undesired friction and undesired retraction of the analyte sensor 956 after insertion. A reduced duration or reduced intensity sterilization process (i.e. lower temperatures and lower humidity) may decrease the possibility of friction and undesired retraction of the analyte sensor 956 after insertion. A relatively low surface area contact with the insertion element 915 and a low membrane sensitivity of the analyte sensor 956 during a sterilization process may also decrease the possibility of undesired friction and undesired retraction of the analyte sensor 956.

In examples, an increased elastic modulus or stiffness of the analyte sensor 956, to resist a buckling force applied proximally to the analyte sensor 956, may reduce the possibility of retraction of the analyte sensor 956. A relatively lower elastic modulus or stiffness of an analyte sensor 956 may increase the possibility of undesired retraction of the analyte sensor 956.

In examples, an increase of the retraction spring (e.g. second spring 526) force increases the instantaneous retraction acceleration of the insertion element 915 at the beginning of the retraction step. The force transmitted through friction (i.e. breakaway stiction) between the analyte sensor 956 and insertion element 915 is insufficient to accelerate the analyte sensor 956 at the same rate as the insertion element 915 given the stiffness and inertial mass of the analyte sensor 956. Therefore, the insertion element 915 retracts while the analyte sensor 956 remains inserted into the skin of the host.

The systems, apparatuses, and methods disclosed herein may be utilized following a sterilization process, however, such systems, apparatuses, and methods may be utilized in the absence of a sterilization process. For example, systems, apparatuses, and methods are not limited to those utilized following a sterilization process or utilized during or prior to a sterilization process.

Systems, apparatuses, and methods disclosed herein may include providing a diametrical clearance 996 (marked in FIG. 28) from the shaft 986 of the insertion element 915 to the analyte sensor 956 (e.g., the second portion 966 or sensing portion). The clearance 996 may reduce the possibility of friction (e.g., kinetic friction or stiction) between the insertion element 915 and the analyte sensor 956. For example, following a sterilization process the analyte sensor 956 may swell (e.g., increase in diameter) and/or hydrogen bonds (or other forms of bonding) may form between the insertion element 915 and the analyte sensor 956. A diametrical clearance 996 at or greater than a threshold may reduce the possibility of such undesired friction.

The diametrical clearance 996, in examples, may be determined prior to a sterilization process being applied to the analyte sensor 956 and/or the insertion element 915. The diametrical clearance 996 may be measured prior to a sterilization process and if the clearance 996 is at or greater than a threshold then the analyte sensor 956 and/or insertion element 915 may continue to be used in a sterilization process. In examples, the diametrical clearance 996 may be measured in the absence of application of a sterilization process or may be measured following a sterilization process. The analyte sensor 956 and/or insertion element 915 may be utilized if the threshold diametrical clearance is met. The threshold diametrical clearance 996 may be set to reduce the possibility of undesired friction between the analyte sensor 956 and/or the insertion element 915.

In examples, the diametrical clearance 996 may be set to be at least 0.07 millimeters. This may be a distance prior to a sterilization process or may be a distance following a sterilization process. The diametrical clearance may be between the shaft 986 of the insertion element 915 to the analyte sensor 956 (e.g., the second portion 966 or sensing portion). The distance may reduce the possibility of undesired friction between the analyte sensor 956 and the insertion element 915. In examples, the diametrical clearance 996 may be set to be at least 0.10 millimeters. In examples, the diametrical clearance 996 may be set to be at least 0.12 millimeters. Other diametrical clearances may be utilized and set to reduce undesired friction between the analyte sensor 956 and the insertion element 915 and reduce the prevalence of the analyte sensor 956 undesirably retracting during retraction of the insertion element 915.

In examples, other features of the analyte sensor 956 and/or the insertion element 915 may be utilized or determined to reduce the possibility of undesirable retraction of the analyte sensor 956 upon retraction of the insertion element 915. The ratio of a diameter 989 (marked in FIG. 28) of the second portion including the sensing portion of the analyte sensor 956 to the width 991 of the opening 987 for the channel 958 for example, may be determined prior to a sterilization process or during or after a sterilization process. In examples, if the ratio is determined to be at or less than a threshold, then the analyte sensor 956 may continue to be used in a sterilization process. In examples, the ratio may be measured in the absence of application of a sterilization process or may be measured following a sterilization process. The analyte sensor 956 may be utilized if the threshold ratio is met.

In examples, the ratio of the diameter 989 to the width 991 may be set to be less than 0.9. This may be a ratio prior to a sterilization process or may be a ratio following a sterilization process. In examples, the ratio may be set to be less than 0.8. In examples, the ratio may be set to be less than 0.7. Other ratios may be utilized and set to reduce the possibility of the analyte sensor 956 retracting during retraction of the insertion element 915.

In examples, other features of the analyte sensor 956 may be utilized or determined to reduce the possibility of undesirable retraction of the analyte sensor 956 upon retraction of the insertion element 915. The flexural modulus of the analyte sensor 956 for example, may be determined prior to a sterilization process or during or after a sterilization process. An analyte sensor 956 with a greater flexural modulus may have a lesser possibility of retracting during retraction of the insertion element 915. In examples, if the flexural modulus is determined to be at or greater than a threshold, then the analyte sensor 956 may continue to be used in a sterilization process. In examples, the flexural modulus may be measured in the absence of application of a sterilization process or may be measured following a sterilization process. The analyte sensor 956 may be utilized if the threshold flexural modulus is met.

In examples, the flexural modulus may be set to be greater than 8 giga Pascals. This may be a flexural modulus prior to a sterilization process or may be a flexural modulus following a sterilization process. The flexural modulus may reduce the possibility of the analyte sensor 956 retracting during retraction of the insertion element 915. In examples, the flexural modulus may be set to be greater than 8.4 giga Pascals. In examples, the flexural modulus may be set to be greater than 8.6 giga Pascals. Other moduli may be utilized and set to reduce the possibility of the analyte sensor 956 retracting during retraction of the insertion element 915. The flexural modulus may be of the second portion 966 including the sensing portion of the analyte sensor 956 in examples.

The configurations of the insertion element 915 and/or analyte sensor 956 may be utilized solely in or combination with other systems, apparatuses, and/or methods disclosed herein.

In examples, systems, apparatuses, and/or methods may include reducing friction between the analyte sensor 956 and the insertion element 915, for example via coating, lubrication, and surface roughness modifications. In examples, the friction may be reduced during or following a sterilization process being performed to the analyte sensor 956 and the insertion element 915 and prior to retraction of the insertion element 915 from the skin of the host. In examples, the friction may be reduced prior to a sterilization process or at another time as desired.

In examples, the friction between the analyte sensor 956 and the insertion element 915 may be reduced by vibrating the analyte sensor 956 and the insertion element 915. In examples, during or following a sterilization process (e.g., a EtO sterilization process) the analyte sensor 956 and the insertion element 915 may be vibrated to reduce a friction (e.g., breaking the stiction) between the analyte sensor 956 and the insertion element 915. A process may include sterilizing one or a plurality of the applicators 900 (e.g., in a configuration as shown in FIG. 25) and vibrating the applicator(s) 900 to reduce the friction. The plurality of applicators 900, for example, may be sterilized on a surface such as a pallet and the entire pallet or other surface may be vibrated to vibrate the analyte sensor 956 and the insertion element 915. In examples, a direct vibration to the analyte sensor 956 and/or insertion element 915 may be provided. Methods of direct vibration may include methods disclosed herein or other methods of directly vibrating the analyte sensor 956 and/or insertion element 915. In other examples, the vibration may occur prior to a sterilization process or in the absence of a sterilization process.

In examples, the friction between the analyte sensor 956 and the insertion element 915 may be reduced by increasing an ambient temperature or decreasing an ambient temperature. For example, during or following a sterilization process the ambient temperature surrounding the analyte sensor 956 and the insertion element 915 may be reduced for a duration of time. The temperature may be reduced to a freezing temperature in examples (e.g., 0 Celsius (C), -18 C, or - 40 C) or to another temperature as desired. The temperature may be maintained at a reduced state for a duration (e.g., 24 hours, or 2-3 hours), as desired. In examples, the temperature may be increased to a high temperature (e.g., 50 C) for a duration (e.g., 24 hours, or 2-3 hours), as desired. The variation in temperature may cause the overall friction (i.e. by breaking the stiction) to be reduced between the analyte sensor 956 and the insertion element 915 due to differences in the coefficients of thermal expansion between the analyte sensor 956 and the insertion element 915 causing movement therebetween. In examples, a combination of an increased temperature and a decreased temperature may reduce the friction between the analyte sensor 956 and the insertion element 915. For example, a cycle of an increased temperature followed by or preceded by a decreased temperature may be utilized to reduce the friction between the analyte sensor 956 and the insertion element 915. An increased temperature may be provided for a duration (e.g., 24 hours, or 2-3 hours) followed by or preceded by a duration of a decreased temperature (e.g., 24 hours, or 2-3 hours). An increased temperature may alternate with a decreased temperature for a desired number of cycles. A thermal shock of increased temperature and decreased temperature may be utilized in examples. In other examples, the variation in temperature may occur prior to a sterilization process or in the absence of a sterilization process.

In examples, the friction between the analyte sensor 956 and the insertion element 915 may be reduced by decreasing an ambient humidity. For example, during or following a sterilization process the ambient humidity surrounding the analyte sensor 956 and the insertion element 915 may be reduced for a duration of time. The humidity may be reduced for a duration of time to reduce moisture present in the membrane of the analyte sensor 956. The humidity may be reduced to dry out the analyte sensor 956 and the insertion element 915 or the space between the analyte sensor 956 and the insertion element 915, and may shrink or de-swell the analyte sensor 956. The humidity may be reduced to produce a dry ambient environment and may be reduced with an increase in temperature to produce a hot dry ambient environment. The environment may be produced following a sterilization process. In other examples, the variation in ambient humidity may occur prior to a sterilization process or in the absence of a sterilization process.

Methods disclosed herein may occur with the on-skin sensor assembly (including the analyte sensor 956) and the insertion element positioned within a housing of an applicator, or may occur outside of the housing of the applicator.

In examples, a desiccant 998 may be packaged with or otherwise provided with the analyte sensor 956 and/or insertion element 915. The desiccant 998 for example may reduce the moisture of the ambient environment surrounding the analyte sensor 956 and/or the insertion element 915 to reduce the friction (e.g., stiction) between the analyte sensor 956 and the insertion element 915. In examples, the desiccant 998 may be packaged or otherwise provided following or during a sterilization process. For example, the desiccant 998 may be inserted in a cavity, such as a cavity of the cap 942 as shown in FIG. 29, with the desiccant 998 positioned to reduce moisture through the openings 950. Other positions of a desiccant 998 may be provided as desired.

Methods disclosed herein may reduce hydrogen bonding between the analyte sensor 956 and the insertion element 915 or may otherwise reduce friction (e.g., kinetic friction or stiction).

Methods as disclosed herein may be utilized solely or in combination with any system, apparatus, or other method disclosed herein.

In examples, a spacer body may be configured to be positioned between a portion of an elongate analyte sensor 956 and the insertion element 915 and may space the portion of the elongate analyte sensor 956 from the insertion element 915. The spacer body may space the portion (e.g., the second portion 966 including the sensing portion of the analyte sensor 956) of the elongate analyte sensor 956 from the shaft 986 of the insertion element 915.

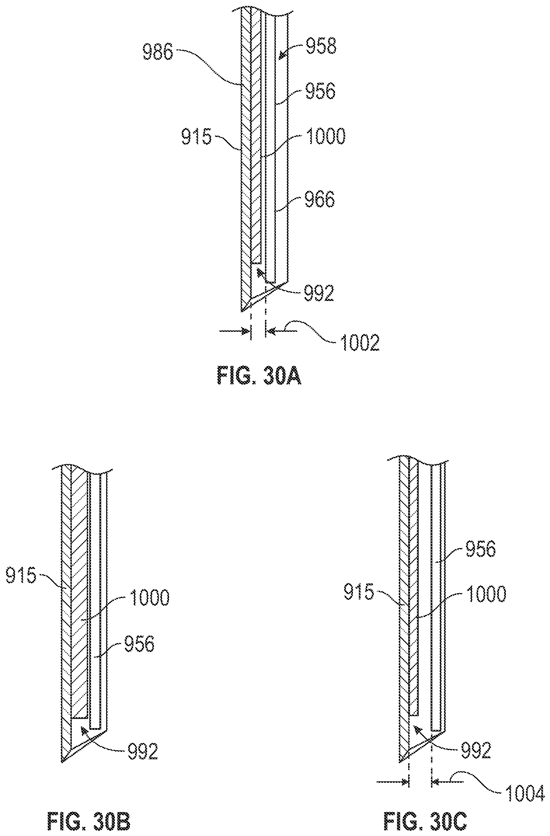

FIG. 30A illustrates an example in which the spacer body 1000 may comprise a thermally expandable body that may be positioned between the analyte sensor 956 and the insertion element 915. The spacer body 1000, for example, may be positioned within the channel 958 between the analyte sensor 956 and the insertion element 915. The spacer body 1000 may be positioned on the interior surface 992 of the insertion elemem 915. The spacer body 1000 may be an elongate body that may extend along the longitudinal axis of the insertion element 915, or may have another form in examples.

In examples, the spacer body 1000 may comprise a thermally expandable metal. In examples, the spacer body 1000 may comprise another form of thermally expandable material (e.g., a polymer or other form of material). The spacer body 1000 in examples, may have a second coefficient of thermal expansion that differs from a first coefficient of thermal expansion of the insertion element 915. The insertion element 915, for example, may be configured to expand at a first rate in response to a variation in temperature and the spacer body 1000 may be configured to expand at a second rate that is different than the first rate. The second rate may be greater than the first rate to allow for greater expansion in response to a variation in temperature.

Referring to FIG. 30A, the analyte sensor 956 may be spaced from the insertion element 915 at a distance 1002. Such a distance 1002 may be prior to a thermal expansion of the spacer body 1000. in examples, the temperature of the spacer body 1000 may be varied. Such variation may comprise an increase in the temperature of the spacer body 1000 (which may include a variation in the temperature of the insertion element 915). The temperature may be varied during a sterilization process (e.g., with heat applied) or following a sterilization process. For example, the variation in temperature may occur during a EtO process and the spacer body 1000 may expand in a thermal increase of a EtO process or following a EtO process. In other examples, the temperature may be varied prior to a sterilization process or in the absence of a sterilization process.

The variation in temperature may increase a size of the spacer body 1000 as shown in FIG. 30B. The spacer body 1000 may expand towards the analyte sensor 956 and may push the analyte sensor 956 away from the interior surface 992 of the insertion element 915. The diametrical clearance of the insertion element 915 from the analyte sensor 956 accordingly may be increased.

The temperature may be further varied (e.g., reduced) to cause the spacer body 1000 to decrease in size. FIG. 30C, for example, illustrates the spacer body 1000 reduced in size yet the analyte sensor 956 remaining at an increased distance 1004 (greater than the distance 1002 shown in FIG. 30A). The increased distance accordingly may reduce possible friction (e.g., kinetic friction or stiction) between the analyte sensor 956 and the insertion element 915 and may reduce the possibility of undesirable retraction of the analyte sensor 956 upon retraction of the insertion element 915.

Other forms of spacer bodies may be utilized in examples.

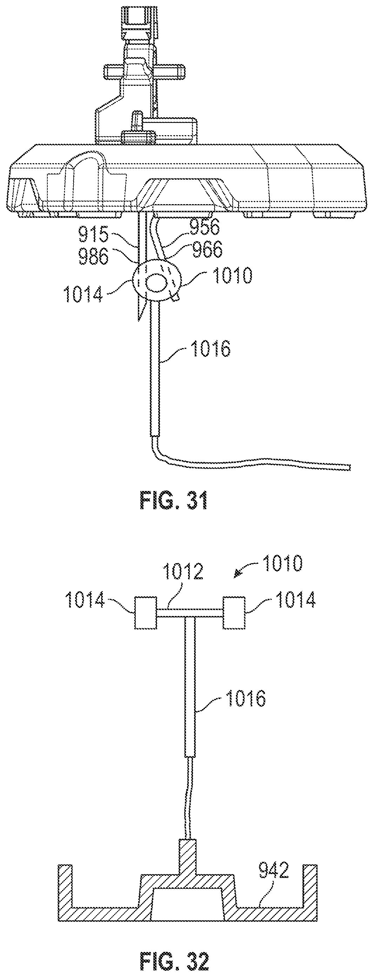

FIG. 31, for example, illustrates an example of a spacer body 1010 configured to be positioned between a portion of the elongate analyte sensor 956 and the insertion element 915 and space the portion of the elongate analyte sensor 956 from the insertion element 915. The spacer body 1010 may be positioned to deflect the analyte sensor 956 away from the insertion element 915 such that the analyte sensor 956 is not initially positioned within the channel 958 of the insertion element 915. In examples, the spacer body 1010 may space the analyte sensor 956 from the insertion element 915 with the analyte sensor 956 positioned within the channel 958, yet spaced from the insertion element 915 due to the presence of the spacer body 1010.

The spacer body 1010 may space the analyte sensor 956 from the insertion element 915 prior to a sterilization process, during a sterilization process, or following a sterilization process. As such, in an example in which the spacer body 1010 is positioned prior to or during a sterilization process, the spacer body 1010 may reduce the possibility of friction (e.g., stiction) forming between the analyte sensor 956 and the insertion element 915 during such a sterilization process. For example, the distance between the analyte sensor 956 and the insertion element 915 may reduce the possibility of hydrogen bonding or other forms of bonding occurring during a sterilization process. Accordingly, the analyte sensor 956 and the insertion element 915 may be sterilized in a configuration as shown in FIG. 31. In examples, the spacer body 1010 may be inserted in a loading process for the analyte sensor 956 and insertion element 915, in which the insertion element 915 is inserted to extend along the analyte sensor 956, yet the spacer body 1010 spaces the analyte sensor 956 from the insertion element 915.

The spacer body 1010 may have a variety of forms and may comprise a bar as shown in FIGS. 31 and 32. The bar may include a cross bar 1012 (marked in FIG. 32) and may include larger diameter end portions 1014 of the cross bar 1012 that may prevent the cross bar 1012 from sliding laterally out from the analyte sensor 956.

The spacer body 1010 may be removable from between a portion of the analyte sensor 956 and the insertion element 915 (e.g., the shaft 986 of the insertion element 915). The spacer body 1010 may be configured to be removed to seat the portion of the analyte sensor (e.g., the second portion 966 or sensing portion) into the channel 958 (shown in FIG. 28).

In examples, the spacer body 1010 may be manually removable from between the analyte sensor 956 and the insertion element 915. For example, the spacer body 1010 may be coupled to a tether 1016 that may be configured to be pulled to remove the spacer body 1010 from between the portion of the analyte sensor 956 and the insertion element 915. The tether 1016 may be coupled to the cross bar 1012 or another portion of the spacer body 1010.

In examples, the tether 1016 may comprise a pull tab for a user to pull. For example, prior to insertion of the insertion element 915 and the analyte sensor 956 into the host's skin, the tether 1016 may be pulled by a user. The spacer body 1010 may be removed to allow the analyte sensor 956 to seat into the channel 958 of the insertion element 915. The insertion element 915 may then be utilized to insert the analyte sensor 956 into the host's skin. By placing the analyte sensor 956 into the channel 958 of the insertion clement 915 immediately prior to application by a user, any friction due to the sterilization process and/or that increases over time is eliminated. This benefit also applies to other embodiments where the analyte sensor 956 is spaced apart from the channel 958 until just prior to application by a user.

The tether 1016 may be covered by a cover in the form of a cap (for example cap 942 shown in FIG. 25) in examples. The cap 942 may cover the distal surface of the housing of the on-skin sensor assembly. The cap 942 may be positioned at a distal opening of an applicator housing (as shown in FIG. 25 for example). Upon removal of the cap 942, the tether 1016 may be accessible to a user that may pull the tether 1016 to displace the spacer body 1010 away from its position between the analyte sensor 956 and insertion element 915.

The tether 1016 and spacer body 1010 may be formed from a single piece of material, although other multi-material configurations may be utilized as desired.

In examples, the spacer body 1010 may be coupled to the cap 942 via the tether 1016 or in another manner. For example, referring to FIG. 32, the tether 1016 may be coupled to the cap 942 to thereby couple the spacer body 1010 to the cap 942. Removal of the cap 942 may pull the tether 1016 coupled to the spacer body 1010 to remove the spacer body 1010 from between the portion of the analyte sensor 956 and the insertion element 915.

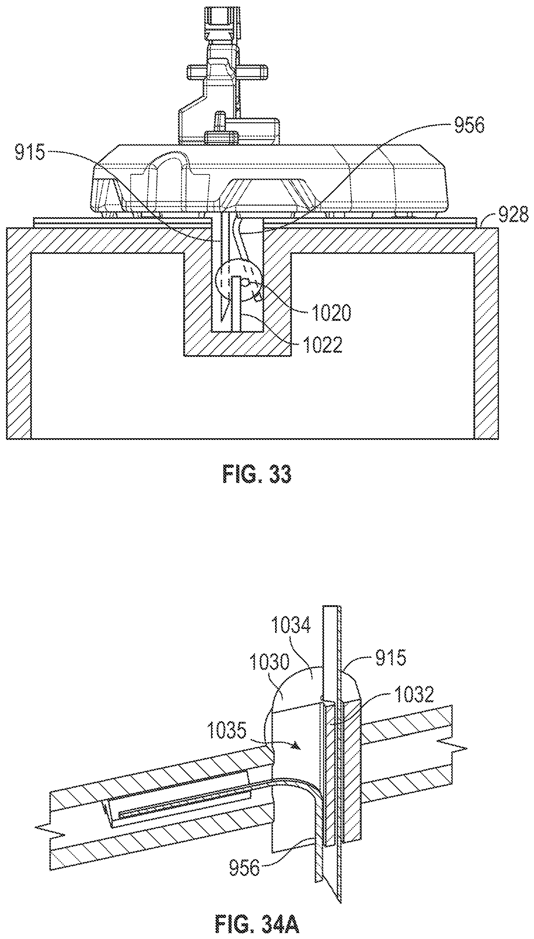

In examples, a spacer body 1020 may be configured as a pin that may be positioned between the insertion element 915 and the analyte sensor 956. FIG. 33, for example, illustrates such a configuration of a spacer body 1020. The spacer body 1020 may operate in a similar manner as the spacer body 1010 and may space the insertion element 915 from the analyte sensor 956.

As shown in FIG. 33, the spacer body 1020 may be coupled to a post 1022 that is coupled to a liner removal component 928 or other removable component. The liner removal component 928 may comprise a cover (e.g., a liner cover) that may cover the distal surface of the housing of the on-skin sensor assembly. The liner removal component 928 may comprise a liner cover for the patch of the on-skin sensor assembly. The spacer body 1020 may be removed from between the insertion element 915 and the analyte sensor 956 upon removal of the liner removal component 928. In examples, the spacer body 1020 may be coupled to a tether in the form of a pull tab or a tether coupled to the cap 942 as shown in FIG. 32. In examples, a spacer body 1010 as shown in FIGS. 31 and 32 may be coupled to a liner removal component 928 as desired.

In examples, a spacer body 1030 may comprise a sheath. For example, referring to FIG. 34A, the spacer body 1030 in the form of a sheath may space the analyte sensor 956 from the insertion element 915. The sheath may surround the insertion element 915 and may include a portion 1032 positioned between the analyte sensor 956 and the insertion element 915 and a portion 1034 that extends around an exterior surface of the insertion element 915. The sheath may include a channel 1035 that the analyte sensor 956 is positioned within.

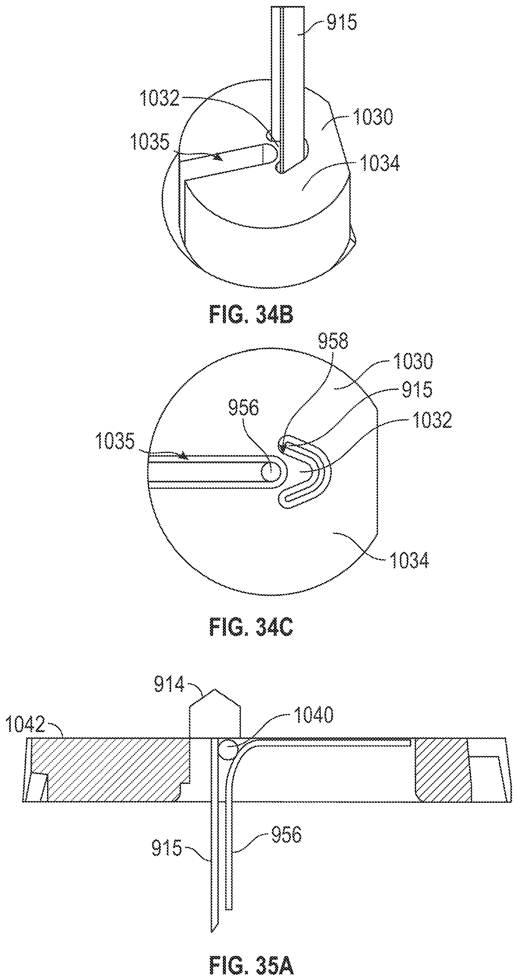

FIG. 34B, for example, illustrates a perspective view of the spacer body 1030 surrounding the insertion element 915. FIG. 34C illustrates an end view of the analyte sensor 956 and the insertion element 915 spaced by the spacer body 1030.

The spacer body 1030 may be positioned between the analyte sensor 956 and the insertion element 915 prior to a sterilization process. In other examples, a spacer body 1030 is placed in position following a sterilization process.

At a desired time prior to application, the spacer body 1030 may be withdrawn from between the analyte sensor 956 and the insertion element 915 to allow the analyte sensor to seat into the channel 958 of the insertion element 915. A user, for example, may manually remove the spacer body 1030. The spacer body 1030 may be coupled to a cap 942 or a liner removal component 928 or to another device for removal in examples as desired. The spacer body 1030, for example, may be coupled to a tether for removal if desired.

In examples, a spacer body 1040 may be positioned between a portion of the elongate analyte sensor 956 and the insertion element 915 and space the portion of the elongate analyte sensor 956 from the insertion element 915. The spacer body 1040 may be compressible and configured to compress to allow the analyte sensor 956 to seat into the insertion element 915. The spacer body 1040, for example, may comprise a compressible body that is configured to compress over a duration of time. The spacer body 1040, for example, may comprise a collapsible body that is configured to collapse after exposure to the sterilization process. For example, FIG. 35A illustrates a configuration of the spacer body 1040 in an uncompressed or expanded configuration and spacing the analyte sensor 956 from the insertion element 915.

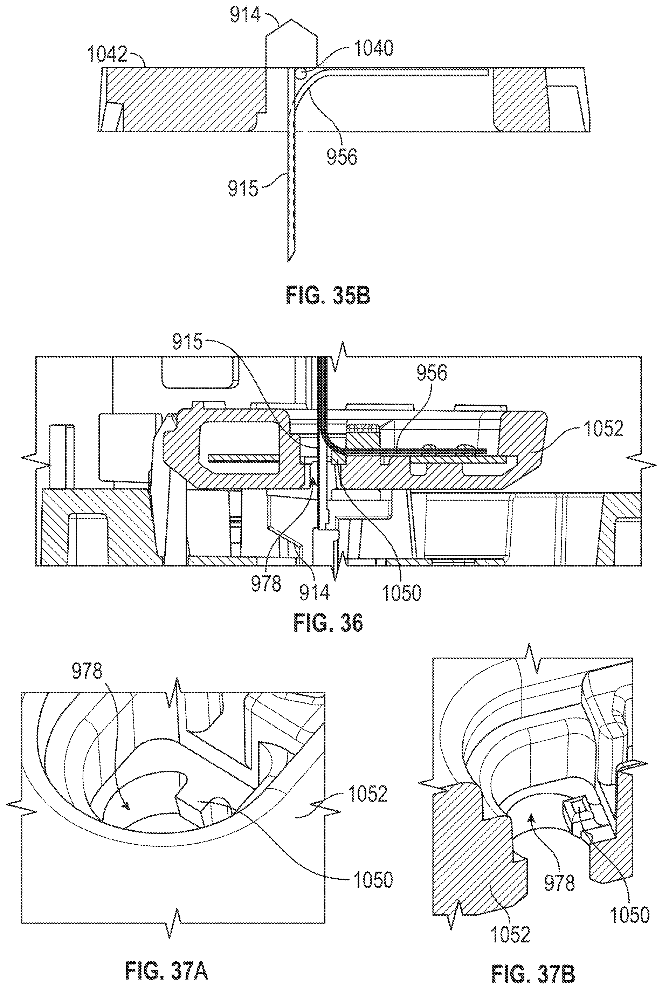

In examples, the configuration shown in FIG. 35A may comprise a configuration of the analyte sensor 956 and the insertion element 915 positioning during sterilization, or prior to sterilization. The configuration of FIG. 35B may comprise a configuration following sterilization in examples.

The spacer body 1040 may be positioned on the needle hub 914 or may be positioned on another component of the applicator or the on-skin sensor assembly. For example, the spacer body 1040 may be positioned on the housing 1042 of the on-skin sensor assembly or on another component as desired.

The spacer body 1040 may be configured to compress, collapse, and/or reduce in size over time. The spacer body 1040 may compress to allow the analyte sensor 956 to seat into the insertion element 915 as shown in FIG. 35B for example. The reduced size of the spacer body 1040 may reduce the spacing between the analyte sensor 956 and the insertion element 915 and allow the analyte sensor 956 to move into the channel 958 (shown in FIG. 28 for example).

In examples, the spacer body 1040 may compress due solely to a span of time, or may collapse based on a sterilization process. For example, a heat cycle, humidity cycle, and/or exposure to sterilizing gas (e.g., EtO), may initiate the collapse of the spacer body 1040. As such, following sterilization the spacer body 1040 may gradually collapse and seat the analyte sensor 956 into the insertion element 915. In examples. the compression of the spacer body 1040 may occur over a span of days or for a greater or lesser duration.

Features of spacer bodies disclosed herein may reduce friction between the analyte sensor and the insertion element and may reduce the possibility of undesirable retraction of the analyte sensor 956 upon retraction of the insertion element 915. In examples, the spacer bodies may prevent friction (e.g., stiction) from occurring between the analyte sensor 956 and the insertion element 915. The spacer bodies may be utilized prior to or during a sterilization process and/or may be utilized following a sterilization process. In examples, the spacer bodies may be utilized during packaging and/or distribution of the applicators and on-skin sensor assemblies as desired. The use of a spacer body may be utilized solely or in combination with any system, apparatus, or method disclosed herein.

In examples, a stopper body may be configured to impede the analyte sensor 956 from retracting proximally through the opening 978 that the insertion element 915 extends through, upon the insertion element 915 retracting proximally through the opening 978.

FIG. 36, for example, illustrates a stopper body 1050 positioned proximate the opening 978. The stopper body 1050 may be positioned within the opening 978 and may protrude into the opening 978. The stopper body 1050 may comprise a tab that extends into the opening 978. The stopper body 1050, in examples, may be positioned proximal of the analyte sensor 956. The stopper body 1050 may be positioned within the opening 978 or may be positioned proximal of the opening 978 (e.g., on or above a proximal surface of the housing 1052, among other locations).

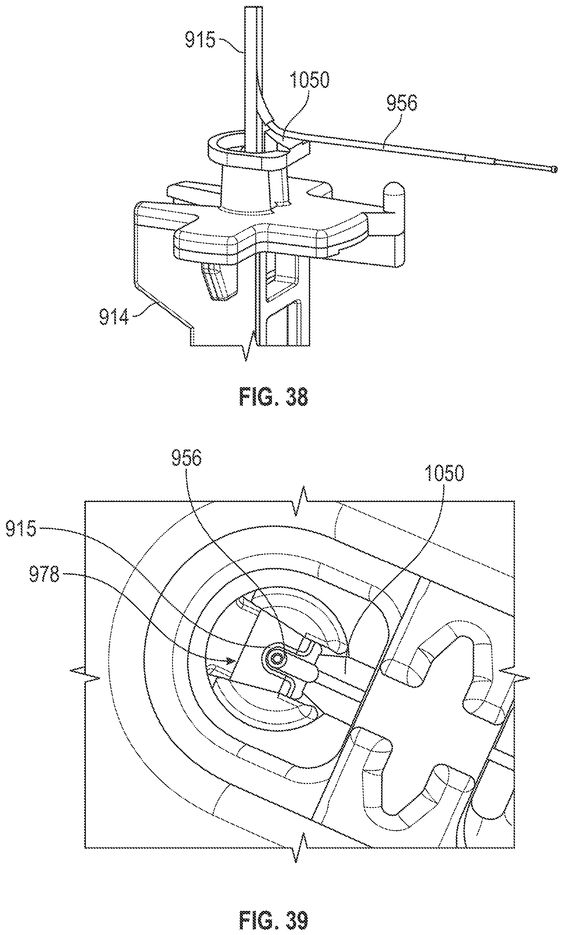

FIGS. 36 and 38 illustrate the stopper body 1050 having an angled face and extending into the opening 978. FIG. 37A illustrates a close up perspective view of the stopper body 1050 having a flat face and extending into the opening 978. FIG. 37B illustrates a close up perspective view of the stopper body 1050 having a projected face and extending into the opening 978. The face portion of the stopper body 1050 may be spaced apart front or initially in contact with, the analyte sensor 956 prior to insertion. The face portion of the stopper body 1050 is designed to contact the analyte sensor 956 in the event the friction between the analyte sensor 956 and the insertion element 915 begins to remove the analyte sensor 956 from the host's skin upon retraction of the insertion element 915. The stopper body 1050 reduces the bending moment applied to the analyte sensor 956 by the insertion element 915 upon retraction by supporting the analyte sensor 956 at a point closer to the insertion element 915.

In examples, the stopper body 1050 may comprise an insert into the housing 1052 of the on-skin sensor assembly or may comprise a molded portion of the housing 1052. For example, FIG. 38 illustrates a perspective view of the stopper body 1050 comprising an insert in the form of a ring that may be inserted into the opening 978. The ring may be positioned distal of the needle hub 914 yet proximal of the analyte sensor 956. The stopper body 1050 accordingly may be positioned between the needle hub 914 and the analyte sensor 956.

FIG. 39 illustrates a distal view of the stopper body 1050 protruding into the opening 978.

Referring to FIG. 36, upon retraction of the insertion element 915 from the host's skin and the opening 978, the stopper body 1050 may contact the analyte sensor 956 to impede the analyte sensor 956 from retracting proximally upon the insertion element 915 retracting proximally through the opening 978. As such, a reduced possibility of retraction of the analyte sensor 956 from the skin and a reduced possibility of a bend 990 as shown in FIG. 27B may result.

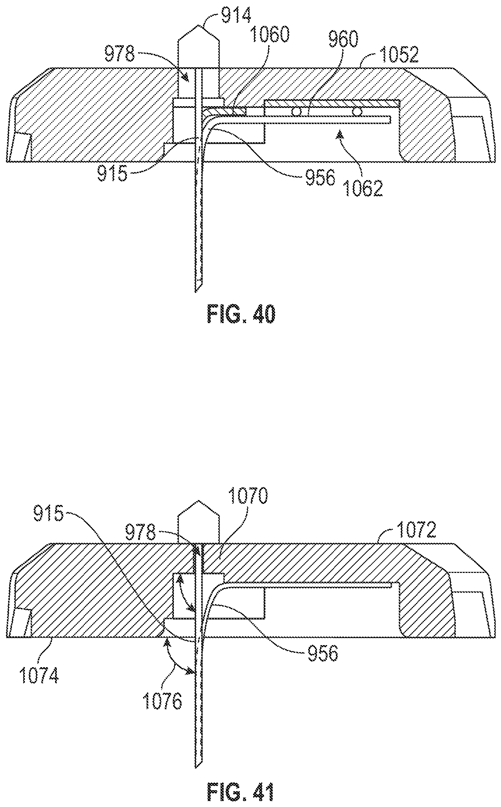

In examples, a stopper body may have other forms. FIG. 40 illustrates an example in which a stopper body 1060 may protrude from a cavity 1062 configured to receive the first portion 960 of the analyte sensor 956. The cavity 1062 may be configured similarly as the cavity 976 shown in FIG. 26A. The cavity 1062, for example, may be configured to receive an adhesive in a liquid form (which may be curable). The cavity 1062 may include one or more tacking dams for retaining the adhesive or other form of curable liquid. The stopper body 1060 may comprise a tab that extends from the cavity 1062 into the opening 978.

In examples, a stopper body 1070 may be integral with the housing 1072. The stopper body 1070 may comprise a portion of the housing 1072.

The stopper body, in examples, may surround the insertion element 915 and may tightly fit the insertion element 915. FIG. 41, for example, illustrates the stopper body 1070 comprising a surface of the housing 1072, with a fit about the insertion element 915 that impedes the analyte sensor 956 from retracting proximally through the opening 978. Further, the fit of the stopper body 1070 to the insertion element 915 may allow the insertion element 915 to extend perpendicular with respect to the distal surface 1074 of the housing 1072 and with respect to the host's skin. A perpendicular angle 1076 of insertion into the host's skin and of retraction from the host's skin may further reduce the possibility of retraction of the analyte sensor 956 from the host's skin.

Systems, methods, and apparatuses disclosed herein may comprise providing a perpendicular insertion angle into the host's skin, and a perpendicular angle of the insertion element from the distal surface of the housing of the on-skin sensor assembly. A size of the opening 978 in the housing that the insertion element 915 passes through may be determined and set to provide such perpendicularity.

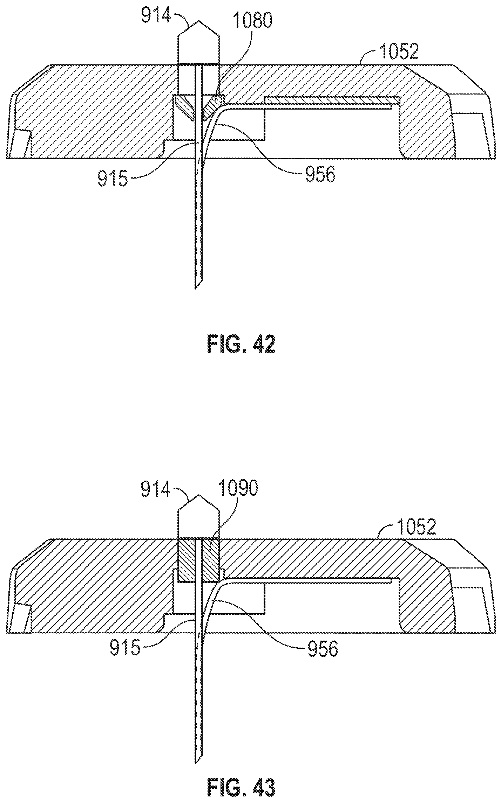

In examples, the stopper body may comprise a plug that is positioned within the opening 978. Referring to FIG. 42, the plug 1080 may have a chamfer that may angle to contact the analyte sensor 956. The plug 1080, for example, may comprise an annular shape (e.g. a washer) positioned within the opening 978 and having a chamfer. The plug 1080 may be inserted into the opening 978 prior to or following assembly of the analyte sensor 956 to the housing 1052. The angled surface of the plug 1080 may contact the analyte sensor 956 upon retraction of the insertion element 915 to impede retraction of the analyte sensor 956.

In examples, the plug may compri se a gasket 1090. Referring to FIG. 43, the gasket 1090 may be inserted into the opening 978 and may fit the insertion element 915. In examples, the gasket 1090 may comprise a self-healing gasket and may conform to a shape of the insertion element 915. The gasket 1090 may contact the analyte sensor 956 to impede retraction of the analyte sensor 956 upon retraction of the insertion element 915. In examples, the gasket 1090 may be overmolded as part of the housing 1052.

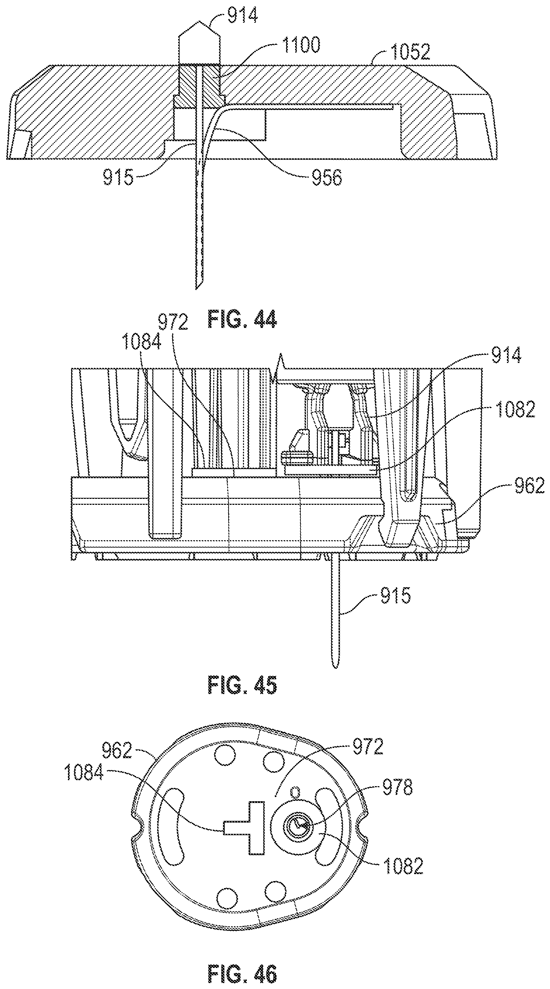

In examples, the plug 1100 may be pierceable by the insertion element 915. Referring to FIG. 44, the plug 1100 may be pierceable such that during assembly of the on-skin sensor assembly the insertion element 915 may pierce the plug 1100 such that the plug 1100 conforms to the shape of the insertion element 915. The stopper body in the form of a plug 1100 accordingly may contact the analyte sensor 956 and impede the analyte sensor 956 from retracting upon retraction of the insertion element 915. The stopper body may be selected to comprise a biocompatible and/or compliant material, in examples.

The use of a stopper body may be utilized solely or in combination with any system, apparatus, or method disclosed herein. The stopper body may be provided during, following, or prior to a sterilization process, or in the absence of a sterilization process. The stopper body may be packaged with the insertion element, the analyte sensor, and/or components of the applicator system during distribution. The stopper body may be utilized upon deployment of the insertion element and the analyte sensor. The stopper body may increase a resistance of the analyte sensor to a buckling force applied proximally to the analyte sensor upon insertion into the host's skin and upon retraction of the insertion element.

In examples, a stopper body may be added following a loading process of an analyte sensor to insertion element. For examples, a stopper body may be added proximal of the analyte sensor. A plug, for example, may be inserted into the opening of the housing or another method of providing a stopper body may be utilized. A stopper body may be press-fit, or snap fit, or may use another form of insertion to stay within the housing when the insertion element 915 retracts. In examples, a stopper body may be injection molded to extend into the opening of the housing. Other forms of formation or insertion of the stopper body may be utilized as desired.

In examples, a displacement mechanism may be utilized that may be configured to displace a portion of the analyte sensor 956 relative to the insertion element 915 prior to retraction of the insenion element 915 from the skin of the host. The displacement mechanism may displace the analyte sensor 956 relative to the insertion element 915 to reduce stiction between the analyte sensor 956 and the insertion element 915.

In examples, the displacement mechanism may be configured to slide the portion of the analyte sensor 956 (e.g., the second portion 966 or sensing portion) relative to the shaft 986 of the insertion element 915. The displacement mechanism may slide the portion of the analyte sensor 956 prior to retraction of the shaft 986 from the skin to reduce friction (e.g., break the stiction) between the analyte sensor 956 and the shaft 986.

Referring to FIG. 45, the displacement mechanism may include a compressible body 1082 that may be positioned between the needle hub 914 and the proximal surface 972 of the housing 962 of the on-skin sensor assembly. FIG. 46 illustrates a top view of the housing 962 with the compressible body 1082 positioned upon the proximal surface 972. The compressible body 1082 may surround the opening 978 in the housing 962.

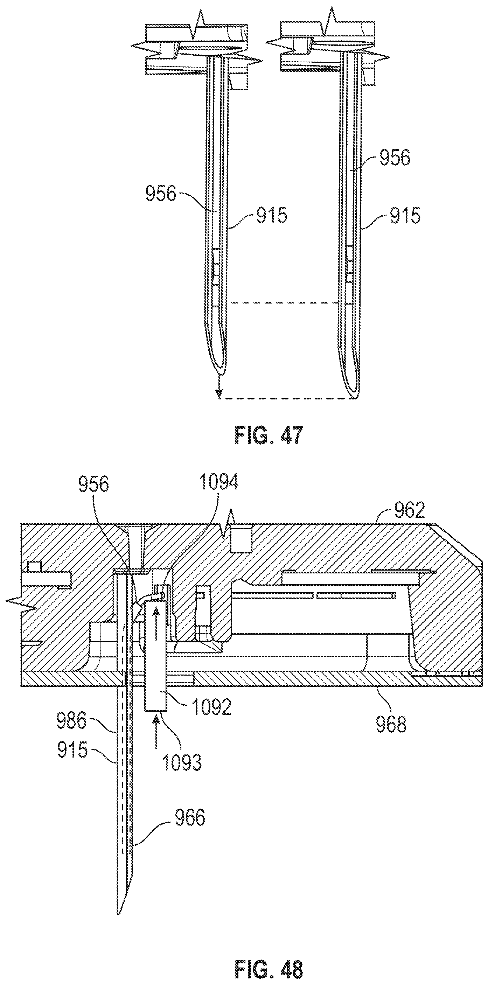

The compressible body 1082 may be configured to compress upon a distal pressure from the needle hub 914 being applied to the compressible body 1082. As such, referring to FIG. 25, the needle carrier assembly 910 or other component of the insertion assembly may apply a distal force to the needle hub 914 upon deployment. Thus, upon insertion of the insertion element 915 and the analyte sensor 956 into the host's skin, the compressible body 1082 may compress. The compression of the compressible body 1082 may allow the needle hub 914 to move distally relative to the housing 962 and the analyte sensor 956 and continue to travel distally with respect to the housing 962 and analyte sensor 956. The insertion element 915 may insert further than the analyte sensor 956. FIG. 47, for example, illustrates the continued travel of the insertion element 915 in the rightmost portion of FIG. 47 relative to the leftmost portion of the FIG. 47. The insertion element 915 may travel independently of the analyte sensor 956 after reaching a full or maximum depth. The displacement of the insertion element 915 relative to the analyte sensor 956 may reduce friction (e.g., break the stiction) between the insertion element 915 and the analyte sensor 956. Thus, upon retraction of the insertion element 915 a reduced possibility of retraction of the analyte sensor 956 from the skin and a reduced possibility of a bend 990 as shown in FIG. 27B may result.

Further, the displacement mechanism may include a second compressible body 1084 (shown in FIG. 46) that the needle carrier assembly 910 may further apply a force to. The second compressible body 1084 may operate in a similar manner as the first compressible body 1082.

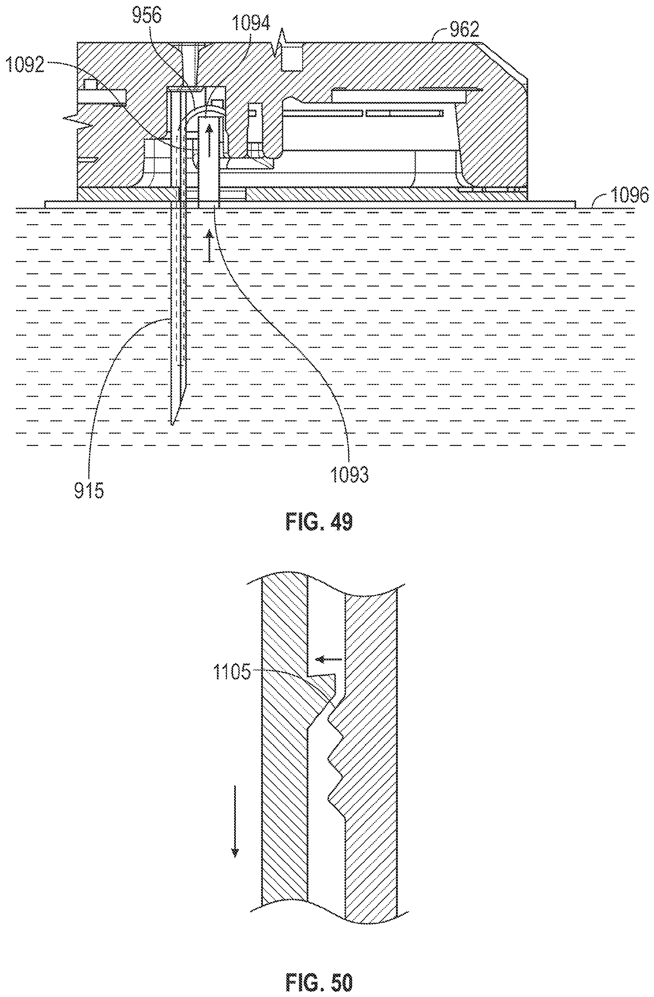

FIGS. 48 and 49 illustrate another form of displacement mechanism in which the displacement mechanism may be configured to slide the portion of the analyte sensor 956 (e.g., the second portion 966 including the sensing portion) relative to the shaft 986 of the insertion element 915. The analyte sensor 956 may be displaced relative to the housing 962. The displacement mechanism may comprise a compressible body 1092 that may protrude distally from the distal surface 968 of the housing 962 and be configured to apply a proximal force upon contact with the host's skin. The compressible body 1092 may have a distal end 1093 configured for contact with the host's skin and a proximal end 1094 configured to contact the analyte sensor 956.

Referring to FIG. 49, as the insertion element 915 and the housing 962 are advanced distally to contact the host's skin 1096 during a deployment or insertion process, the distal end 1093 of the compressible body 1092 may contact the skin 1096 thus driving the proximal end 1094 to contact the analyte sensor 956. The analyte sensor 956 may travel proximally independent of the insertion element 915 after the insertion element 915 reaches a full or maximum depth. The analyte sensor 956 may be displaced proximally relative to the housing 962 and the insertion element 915, thus reducing friction (e.g., stiction) between the insertion element 915 and the analyte sensor 956. Upon retraction of the insertion element 915 a reduced possibility of retraction of the analyte sensor 956 from the skin and a reduced possibility of a bend 990 as shown in FIG. 27B may result.

FIG. 50 illustrates an example of a displacement mechanism configured to vibrate one or more of the insertion element 915 (e.g., the shaft 986) or the analyte sensor 956 (e.g., the second portion 966 or sensing portion) prior to retraction of the insertion element 915 from the skin to break the stiction between the analyte sensor 956 and the insertion element 915. The displacement mechanism may be configured to produce the vibrations upon deployment of the analyte sensor 956 distally into the host's skin. An insertion assembly may include the displacement mechanism, in examples.

The displacement mechanism, for example, may comprise one or more ridges 1105 for a portion of the insertion assembly to contact, to vibrate the insertion assembly upon deployment. The ridges 1105, for example, may be positioned on an inner housing 906 as shown in FIG. 25 and/or the ridges 1105 may be positioned on a holder 912, the needle carrier assembly 910, and/or other component of the applicator as desired. The ridges 1105 may be utilized to vibrate the on-skin sensor assembly and/or the needle hub 914 or other component upon insertion to reduce friction (e.g., break the stiction) between the insertion element 915 and the analyte sensor 956. Upon retraction of the insertion element 915 a reduced possibility of retraction of the analyte sensor 956 from the skin and a reduced possibility of a bend 990 as shown in FIG. 27B may result.

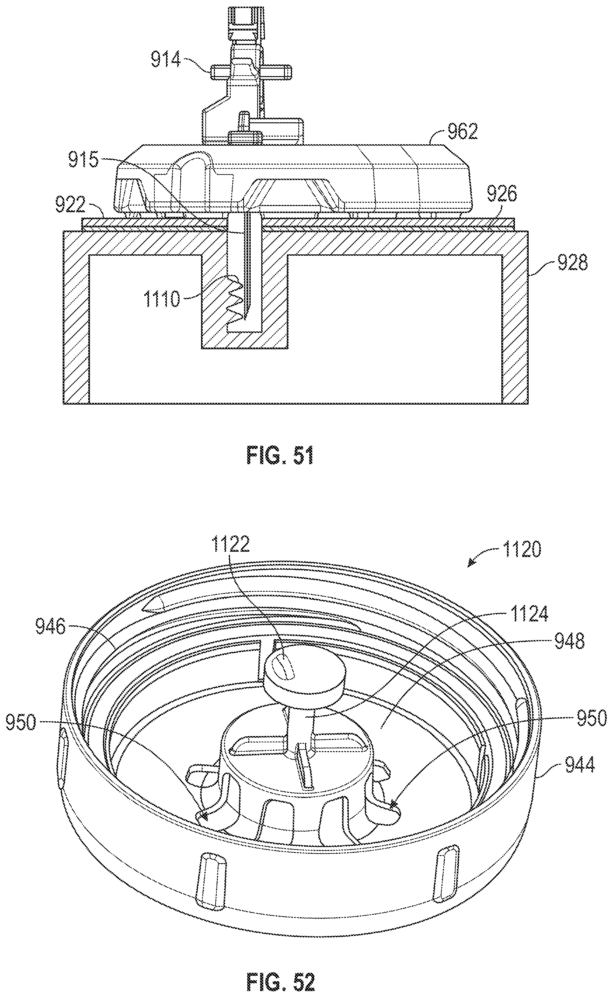

FIG. 51 illustrates an example including a displacement mechanism positioned on a cover covering the distal surface of the housing 962. The cover may comprise an optional liner removal component 928 that may comprise a liner cover for the patch of the on-skin sensor assembly. The displacement mechanism may be configured to vibrate one or more of the insertion element 915 or the analyte sensor 956 upon withdrawal of the liner removal component 928 from the housing 962 and patch 922. The liner removal component 928 may include one or more ridges 1110 that may contact the insertion element 915 and/or the analyte sensor 956 upon removal of the liner removal component 928. The vibration caused by the ridges 1110 may reduce friction (e.g., break the stiction) between the insertion element 915 and the analyte sensor 956. Upon retraction of the insertion element 915 a reduced possibility of retraction of the analyte sensor 956 from the skin and a reduced possibility of a bend 990 as shown in FIG. 27B may result.

FIGS. 52-54 illustrate an example of a displacement mechanism positioned on a cover covering the distal surface of the housing 962. The cover may comprise a cap 1120 comprising a displacement mechanism. The cap 1120 may be configured similarly as the cap 942 shown in FIG. 25 that may be positioned at a distal opening of the applicator housing, yet may include the displacement mechanism in the form of a cam surface 1122. The cam surface 1122 may be configured to apply a force to the housing 962 to displace a portion (e.g., a second portion 966 or sensing portion) of the analyte sensor 956 relative to the insertion element 915. The cam surface 1122 may vibrate one or more of the analyte sensor 956 or the insertion element 915.

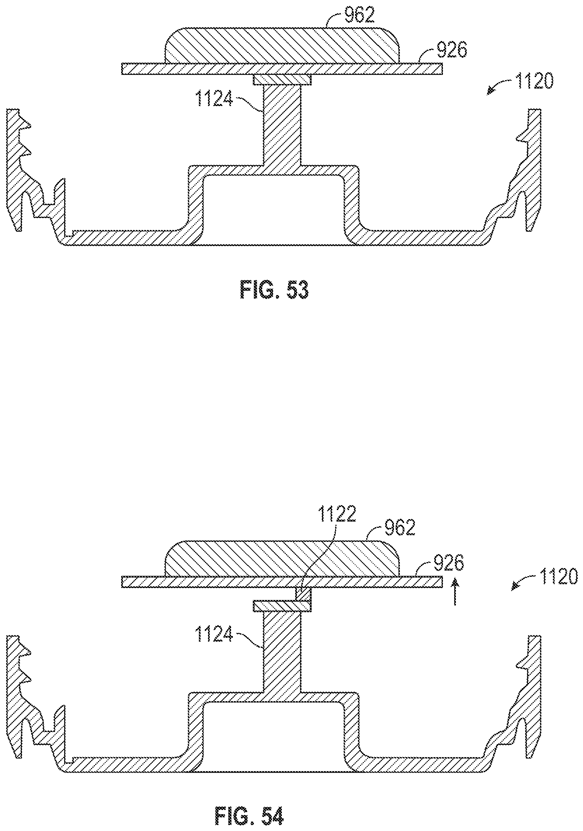

The cam surface 1122 may be positioned on a central support 1124 of the cap 1120 that may extend proximally from the central portion 948 of the cap 942. The cam surface 1122 may be configured to be positioned within a cavity of the housing 962 that may receive the cam surface 1122. Upon rotation of the cap 1120 (in an uncoupling, unscrewing, or uncapping motion) the cam surface 1122 may rotate its position to contact a distal surface of the housing 962 or the patch 922. For example, FIG. 53 illustrates a schematic cross-sectional view in which the cam surface 1122 is positioned within a cavity of the housing 962. FIG. 54 illustrates the cap 1120 rotated such that the cam surface 1122 exits the cavity of the housing 962 and contacts the distal surface of the housing 962 or the patch 922. The housing 962 accordingly displaces proximally in FIG. 54. The displacement of the housing 962 due to the cam surface 1122 may vibrate and displace the analyte sensor 956 relative to the insertion element 915 and may reduce friction (e.g., break the stiction) between the insertion element 915 and the analyte sensor 956. As such, during an uncapping operation the friction between the insertion element 915 and the analyte sensor 956 may be reduced. The continued rotation of the cap 1120 may cause the housing 962 to continue to be vibrated by the cam surface 1122 to continue to reduce friction between the insertion element 915 and the analyte sensor 956. Upon retraction of the insertion element 915, a reduced possibility of retraction of the analyte sensor 956 from the skin and a reduced possibility of a bend 990 as shown in FIG. 27B may result.

The use of a displacement mechanism may be utilized solely or in combination with any system, apparatus, or method disclosed herein. The use of a displacement mechanism may occur following a sterilization process, or in examples, may occur prior to or during a sterilization process. In examples, the use of a displacement mechanism may occur without a prior sterilization process.

In examples, a force channeling component may be utilized that may be configured to channel a force from the insertion assembly proximate the insertion element 915. The force channeling component may reduce a friction (e.g., stiction) between the analyte sensor 956 and the insertion element 915.

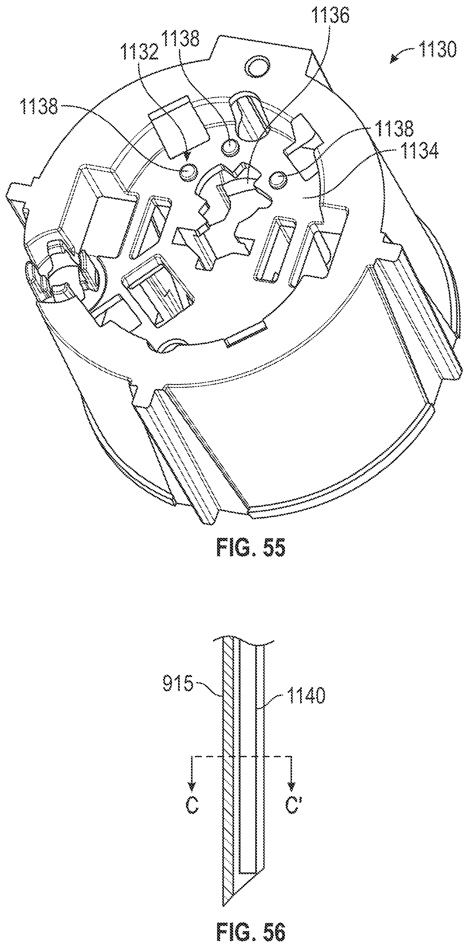

Referring to FIG. 55, a component of the insertion assembly such as a holder 1130 may include the force channeling component 1132. The holder 1130 may be configured similarly as the holder 912 shown in FIG. 25. A portion of the insertion assembly, such as the holder 1130, may include a plate 1134 that may be configured to be positioned proximal of the proximal surface 972 of the housing 962. The plate 1134 may include an opening 1136 for the needle hub 914 to pass through.

The force channeling component 1132 may comprise one or more protrusions 1138 that may be configured to channel a force of the insertion assembly proximate the needle hub 914 and the insertion element 915. The one or more protrusions 1138 may be positioned on the plate 1134. The one or more protrusions 1138 may be configured to contact the proximal surface 972 of the housing 962 and apply the force to the proximal surface 972 proximate the insertion element 915. The one or more protrusions 1138 may contact the proximal surface 972 proximate the opening 978 of the housing 962 for the insertion element 915 to pass through.

The one or more protrusions 1138 may direct the force of deployment proximate the needle hub 914 and the insertion element 915. The force from the force channeling component 1132 may displace the analyte sensor 956 relative to the insertion element 915 and may reduce friction (e.g., break the stiction) between the insertion element 915 and the analyte sensor 956. The force from the force channeling component 1132 may vibrate or transmit a vibration to the analyte sensor 956 relative to the insertion element 915. Upon retraction of the insertion element 915 a reduced possibility of retraction of the analyte sensor 956 from the skin and a reduced possibility of a bend 990 as shown in FIG. 27B may result. In examples, the force channeling component may contact the proximal surface 972 of the housing 962 after the insertion element 915 penetrates the skin.

The use of a force channeling component may be utilized solely or in combination with any system, apparatus, or method disclosed herein.

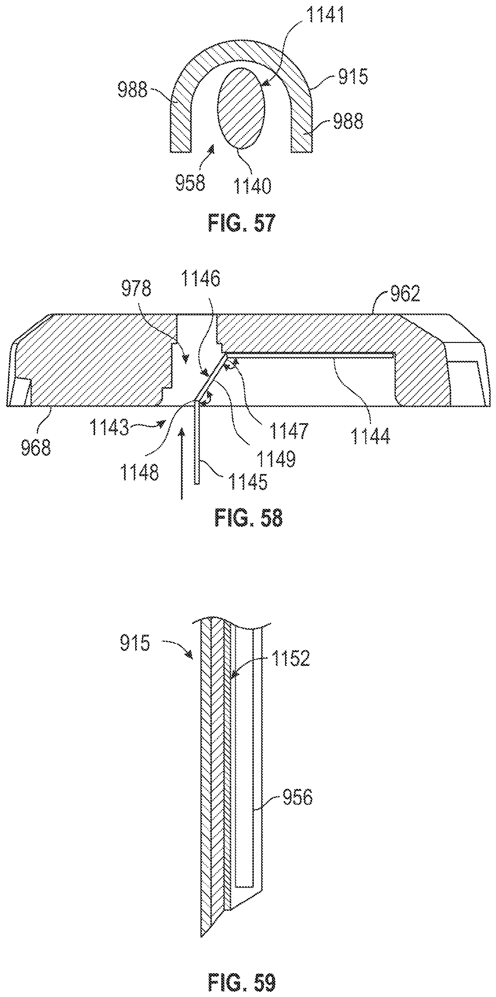

In examples, an analyte sensor may be configured to reduce friction (e.g., stiction or kinetic friction) with an insertion element 915. FIG. 56 illustrates an example of an analyte sensor 1140 having a cross section with an oval shape. FIG. 57 illustrates a cross-sectional view of the analyte sensor 1140 and the insertion element 915 along line C-C' in FIG. 56. The outer surface 1141 of the analyte sensor 1140 may be configured to reduce friction (e.g., stiction) with the insertion element 915. The oval shape of the cross section may reduce the distance or clearance of the analyte sensor 1140 from the side walls 988 of the insertion element 915 and may thus reduce friction with the insertion element 915. For example, the channel 958 of the insertion element 915 may have a C-shaped cross section that may increase the diametrical clearance from the side walls 988 to the oval shaped analyte sensor 1140. Further, reduced contact points or contact surface area between the oval shaped analyte sensor 1140 and the insertion element 915 may result.

The features of FIGS. 56 and 57 may be utilized solely or in combination with any system, apparatus, or method disclosed herein.

In examples, an analyte sensor may have a surface that is configured to reduce friction with an insertion element 915. For example, the surface of the analyte sensor may comprise an outer surface that may be configured to reduce stiction with the insertion element 915. The surface, for example, may be configured to reduce hydrogen bonding with the insertion element 915. In examples, a membrane comprising the outer surface of the analyte sensor may have properties that may prevent hydrophilic permeability and/or membrane swelling. For example, a ratio of polyvinylpyrrolidone (PVP) may be varied (e.g., reduced) to prevent hydrophilic permeability and/or membrane swelling. Features of surfaces of an insertion element disclosed herein may also be utilized with surfaces of an analyte sensor.

In examples, an analyte sensor may be oriented to increase a resistance to a buckling force for the analyte sensor and thus provide a reduced possibility of retraction of the analyte sensor from the skin and a reduced possibility of a bend 990 as shown in FIG. 27B. Referring to FIG. 58, for example, an analyte sensor 1143 may include a first portion 1144 or contact portion and a second portion 1145 or sensing portion. The first portion 1144 may be coupled to the housing 962 and the second portion 1145 may be configured to extend distally from the housing 962 and be inserted into the skin of the host.

The bend 1146 of the analyte sensor 1143 may have at least two kinks 1147, 1148 that may angle the second portion 1145 from the first portion 1144. A first kink 1147 may be positioned between the first portion 1144 and an intermediate portion 1149 of the analyte sensor 1143. A second kink 1148 may be positioned between the intermediate portion 1149 and the second portion 1145.

The first kink 1147 may have an angle that is less than ninety degrees. For example, the first kink 1147 may have an angle between thirty degrees and sixty degrees, which may be an angle of forty-five degrees, or another angle may be utilized as desired. Similarly, the second kink 1148 may have an angle between thirty degrees and sixty degrees, which may be an angle of forty-five degrees, or another angle may be utilized as desired. In examples, the at least two kinks 1147, 1148 may angle the second portion 1145 to be perpendicular from the first portion 1144. The second portion 1145 may extend perpendicular from the distal surface 968 of the housing 962 or at another angle as desired. In examples, the first portion 1144 may extend parallel with the distal surface 968 of the housing 962. Varied other angles may be utilized as desired.

The intermediate portion 1149 of the analyte sensor 1143 may be straight or linear. In examples, the intermediate portion 1149 may have a curvature as desired.

The use of the kinks 1147, 1148 may enhance the strength of the analyte sensor 1143 in response to a buckling force or force of retraction applied to the analyte sensor (in a direction marked by the arrow in FIG. 58). The increased strength of the analyte sensor 1143 in response to the buckling force may reduce the possibility of retraction of the analyte sensor 956 from the skin and a reduced possibility of a bend 990 as shown in FIG. 27B may result.

The features of FIG. 58 may be utilized solely or in combination with any system, apparatus, or method disclosed herein.

In examples, other configurations of an analyte sensor may be utilized to increase the strength of an analyte sensor in response to a buckling force. For example, a stiffness of the analyte sensor may be increased. A stiffer alloy of the sensor may be utilized as desired.

In examples, an insertion element 915 may be configured to reduce friction (e.g., stiction or kinetic friction) with an analyte sensor 956. Referring to FIG. 59, for example, a surface 1152 of the insertion element 915 (e.g., a surface 1152 of the shaft of the insertion element 915) may be configured to reduce friction with a portion of the analyte sensor (e.g., a second portion 966 or sensing portion). In examples, metals or alloys may be selected for the insertion element 915 that may reduce friction with the analyte sensor 956.

According to **the present invention, the** surface **1152** comprises a coating configured to reduce friction with the portion of the analyte sensor 956. The coating, for example, may be a lubricant that may be positioned on the portion of the insertion element. The lubricant may comprise a biocompatible lubricant. Petrolatum (petroleum jelly) may be utilized in examples, among other forms of lubricant. In examples, the coating may comprise a polymer. For example a coating of a plastic, such as a thin plastic coating may be utilized. A polymer such as polytetrafluoroethylene (PTFE), parylene, or another form of polymer may be coated on the insertion element. Vapor deposition may be utilized to apply a polymer to the insertion element 915.

In examples, the coating may comprise a thermal oxide. A thermal oxide may be formed on an insertion element 915 comprising aluminum or titanium, for example.

In examples, the coating of the insertion element 915 may comprise an inert material. For example, an inert material having low surface energy may be utilized, to reduce the possibility of hydrogen bonding or other forms of electrical bonding with the analyte sensor. An inert material such as silane (SiH₄) may be utilized for example.

In examples, the coating of the insertion element 915 may comprise one or more of a spray coating, a brush coating, an electrostatically applied coating, anodization, or more preferably a plating, a dip coating, or a deposition. Vapor deposition including chemical vapor deposition and physical vapor deposition may be utilized in examples. For example, vapor deposition of chromium nitride, titanium nitride, and titanium carbonitride can be applied to the insertion element 915. In certain embodiments, the thickness of the nitride coating is between 10 - 5000 nanometers (nm) and, preferably, between 100 - 1000 nm. A plating may include a plating such as titanium, nickel, gold, other forms of plating, and alloys thereof.

In examples, a coating may be provided that may be bonded to the shaft of the insertion element 915. The coating may be chemically bonded in examples. The coating may be provided as a plating, a dip coating, or a deposition (which may be a spray coating, a chemical vapor deposition, or a physical vapor deposition), among other processes. The coating material may be silicone based, silicone based, fluoro compound based, or parylene based, among other materials.

A coating may be cured in examples. The coating may be cured via addition curing, condensation curing, thermal curing, as well as other curing methods. A coating that is cured may be applied in a variety of the manners disclosed herein. The curing may produce cross-linking that may improve adhesion and cohesion of the coating and the robustness of the coating upon the insertion element 915. Examples of coatings herein may be heated, evaporated, or have other processes performed to them to facilitate a curing. Curing may be performed at room temperature in examples.

In examples, the coating may include silicone. The coating may be derived from a silane (SiH₄) compound. In examples, the silicone may comprise an aminofunctional dimethylsiloxane copolymer. The material may be provided as a compound of about 50% active silicone ingredients (e.g., the aminofunctional dimethylsiloxane copolymer) mixed with one or more solvents. The solvents may comprise aliphatic hydrocarbon and isopropanol solvents in examples. Other proportions of components comprising the material may be provided in examples (e.g., about 45% to 55% active silicone ingredients, about 40% to 60% active silicone ingredients, etc.). Other forms of solvents may be utilized in examples. Other forms of materials for coatings may be utilized in examples.

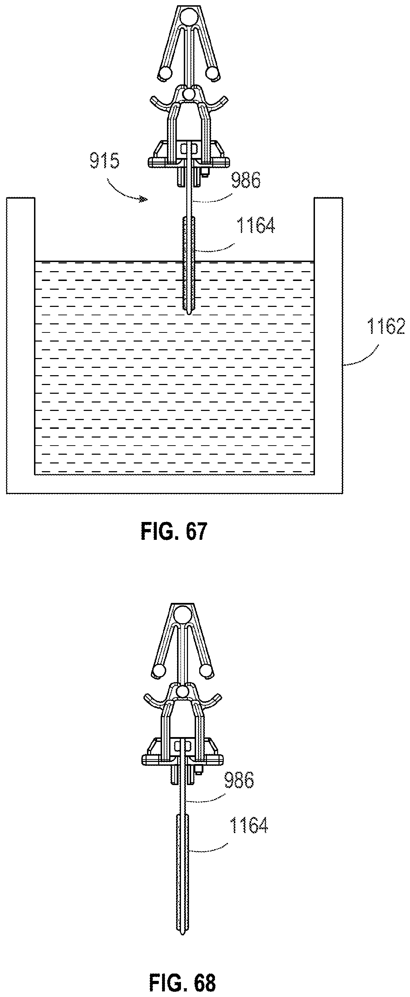

FIG. 67 illustrates an exemplary step in a method of coating at least a portion of a shaft 986 of the elongate insertion element 915. The coating may be of a material configured to reduce stiction and friction between the elongate insertion element 915 and the elongate analyte sensor 956. In examples, other forms of application of the material may be utilized as disclosed herein (e.g., a spray coating or other form of deposition).

FIG. 67 illustrates the shaft 986 of the elongate insertion element 915 having been positioned within a bath 1162. The bath 1162 may include the material therein. A solution of the material may be provided. For example, in an example in which the material comprises the compound of active silicone ingredients (e.g., the aminofunctional dimethylsiloxane copolymer) mixed with one or more solvents, this material may be combined or diluted with additional solvents. The material may be added to additional solvents, producing a mixture of about 0.1 % of the material (e.g., the aminofunctional dimethylsiloxane copolymer mixed with the solvents) in the bath of the additional solvents. The proportion of the material to the additional solvents may be varied as desired. For example, about 0.05%, 0.2%, 0.3%, 1.0%, or a greater concentration of the material may be provided as desired. The additional solvents may comprise hexane or other forms of solvents.

The shaft 986 of the elongate insertion element 915 may be positioned within the bath 1162 for a desired duration (e.g., less than 30 minutes, or another duration as desired). A thickness of the material upon the shaft 986 of the elongate insertion element 915 may be determined by the duration within the bath 1162. Loose silicone molecules may chemically bond to the surface of the shaft 986 of the elongate insertion element 915. The polar ends of any aminofunctional groups may adhere to a metallic shaft 986 to form a densely packed layer on the surface. The shaft 986 may be withdrawn from the bath 1162 (partial withdrawal is shown in FIG. 67) to produce a layer 1164 of the material upon the shaft 986 as represented in FIG. 67.

The layer 1164 may be cured upon the shaft 986. FIG. 68, for example, illustrates the shaft 986 external of the bath 1162. The material may cure at room temperature or other methods of curing may be utilized (e.g., heating or evaporation, among others). Air or other gas may be blown over the material in a curing process. Ambient (room) relative humidity may be utilized. The curing may occur for a desired duration (e.g., about 1 week, less than 24 hours, less than 30 minutes, or greater or lesser durations, as desired). The curing may produce cross-linking that may improve adhesion and cohesion of the coating and the robustness of the coating upon the insertion element 915. A permanent chemical bond may result.

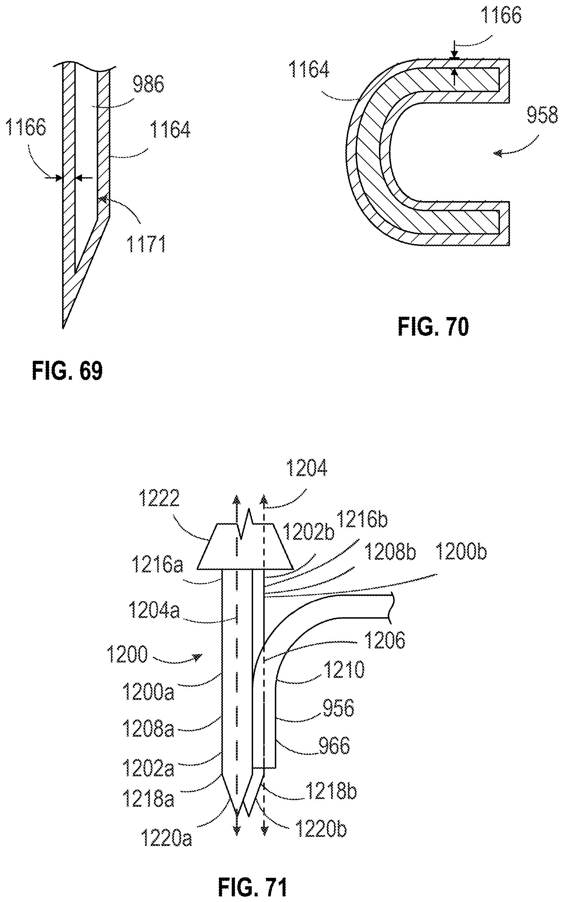

The resulting layer 1164 upon the outer surface of the shaft 986 of the insertion element 915 is represented in FIG. 69. The layer 1164 may have a thickness as desired based on the selected coating material, duration within the bath, and the curing process and duration utilized. The layer 1164 may have a thickness 1166 upon the shaft 986 that is less than 1 micrometer in examples. The layer 1164 may have a thickness 1166 upon the shaft 986 that is less than 1.5 micrometers in examples. The layer 1164 may have a thickness 1166 upon the shaft 986 that is less than 2 micrometers in examples. The layer 1164 may have a thickness 1166 in a range between 0.1 micrometers and 1, 1.5, or 2 micrometers in examples. The layer 1164 may have a thickness 1166 in a range between 0.5 micrometers and 1, 1.5, or 2 micrometers in examples. Greater or lesser thicknesses may be provided as desired.

In examples, a channel 958 of the insertion element 915 may include a layer of the material. For example, FIG. 70 illustrates an interior surface of the insertion element 915 forming the channel 958 being provided with the layer of the material. The layer may have a thickness in an amount as disclosed herein.

The layer of material may be beneficially stable and durable. The layer of material may be very low in extractables and leachables, and thus safe to use for a medical insertion application. The layer of material may reduce stiction and friction forces of the insertion element 915 with the analyte sensor 956 and local tissue. The material may reduce friction (e.g., stiction or kinetic friction) with the elongate analyte sensor 956. Improved deployment reliability and accuracy may be produced. Reduced insertion tissue damage may be provided.

In examples, the material may produce a friction coefficient for the shaft 986 that is more than ten times lower than a friction coefficient of the surface 1171 (marked in FIG. 69) of the portion of the shaft 986 coated with the material. In examples, a greater or lesser variation in the friction coefficient may be provided.

Following a coating process, additional assembly steps may be provided utilizing the insertion element 915. For example, the elongate insertion element 915 may be positioned adjacent to the elongate analyte sensor 956. The elongate analyte sensor 956 may be positioned within a channel 958 of the elongate insertion element 915 as disclosed herein. The elongate analyte sensor 956 may be configured similarly as other forms of analyte sensors disclosed herein. For example, the elongate analyte sensor 956 may extend distally from a housing 962 configured to be worn on the skin of the host. Other assembly steps may be provided following or prior to a coating process.

The methods disclosed herein may be utilized with other forms of materials. Steps of the methods may be substituted, excluded, added to, or modified as desired.

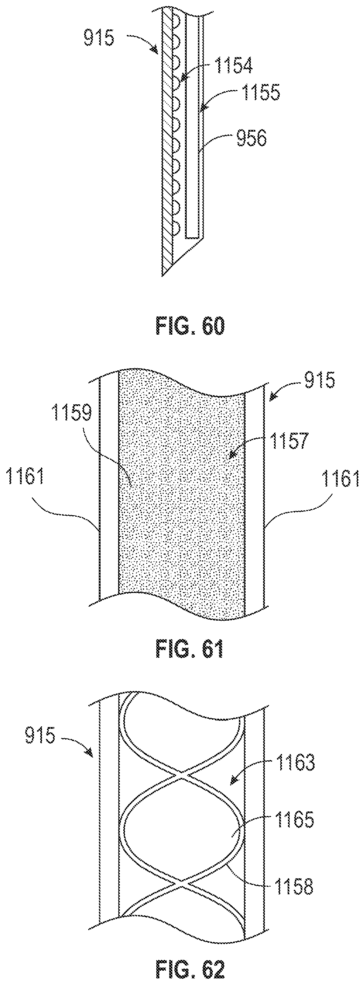

In examples, a surface of the insertion element 915 may include a surface roughness. For example, referring to FIG. 60, a surface 1154 may include a plurality of bumps. The raised portions of the bumps may contact the analyte sensor 956 and may reduce the contact surface area and thus friction with the analyte sensor 956. In examples, the height of the bumps may be varied as desired. For example, a surface roughness may be 35 root mean square (RMS) microinches or greater in examples. In examples, a surface roughness may be 40 RMS microinches or greater. In examples, a surface roughness may be 45 RMS microinches or greater. A greater or lesser surface roughness may be provided as desired.

The surface 1154 of the insertion element 915 may be an interior surface that faces the analyte sensor 956. The interior surface may be positioned within a channel 1155 of the insertion element 915.

The surface of the insertion element may have a surface texture. The texture may comprise one or more patterns of raised portions of the surface. FIG. 61, for example, illustrates a front view of a channel 1157 of an insertion element showing an interior surface 1159 having a surface texture. The insertion element 915 may have side walls 1161 that bound the channel 1157. The texture reduces the overall surface area contact between the analyte sensor 956 and the insertion element 915 thus lowering the friction.

In examples, the surface of the insertion element 915 may include grooves. FIG. 62, for example, illustrates a front view of a channel 1163 of an insertion element 915 showing an interior surface 1165 having grooves 1158. The grooves 1158 may extend along the longitudinal axis of the channel 1163 and may be straight or may be angled or curved as desired. For example, as shown in FIG. 62, the grooves 1158 may intersect in a repeating curved pattern. The grooves 1158 may be configured in a helix or spiral or other configurations as desired. The grooves may be laser, mechanically, chemically etched or may be produced in another manner. The grooves 1158 reduce the overall surface area contact between the analyte sensor 956 and the insertion element 915 thus lowering the friction.

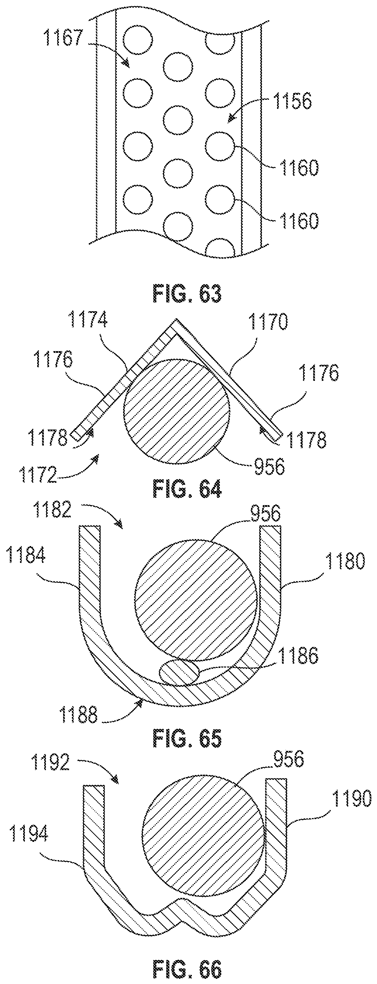

In examples, the surface of the insertion element 915 may include holes. FIG. 63, for example, illustrates a front view of a channel 1167 of an insertion element 915 showing an interior surface 1156 having holes 1160. The holes 1160 may be arranged in a pattern and the pattern may extend along the longitudinal axis of the channel 1167. For example, a repeating pattern of holes 1160 longitudinally aligned may be utilized or another pattern may be provided as desired. The holes 1160 reduce the overall surface area contact between the analyte sensor 956 and the insertion element 915 thus lowering the friction.

In examples, a surface of the insertion element may include one or more of bumps, holes, or grooves. The surfaces may be configured to reduce friction (e.g., stiction or kinetic friction) with a portion of the analyte sensor 956. For example, the surfaces may reduce the hydrogen bonding sites between the analyte sensor 956 and the insertion element 915 among other forms of reduced friction (e.g., electrical or mechanical).

The channels of insertion element 915 shown in FIGS. 59-63 may have a C-shaped cross-section. In examples, other cross-sectional shapes may be provided.

FIG. 64, for example, illustrates a top cross-sectional view showing an insertion element 1170 having a V-shaped cross-sectional channel 1172 for receiving a portion of the analyte sensor 956. The shaft 1174 of the insertion element 1170 may include the V-shaped cross-sectional channel 1172.

The V shape of the channel 1172 may be formed by side walls 1176 of the insertion element 1170 being angled relative to each other. Accordingly, the interior surfaces 1178 of the side walls 1176 may be angled relative to each other. In examples, the V-shaped cross-sectional channel 1172 may have an angle of between 60 degrees and 120 degrees. In examples, the V-shaped cross-sectional channel 1172 may have an angle of 90 degrees. In examples, greater or lesser angles may be provided as desired. The interior surfaces 1178 may comprise flattened walls that may be positioned to contact a circular analyte sensor 956 at only two contact points. A reduced surface area may result, and as such, reduced friction (e.g., stiction or kinetic friction) may result.

FIG. 65 illustrates a top cross-sectional view showing an insertion element 1180 having a W-shaped cross-sectional channel 1182 for receiving a portion of the analyte sensor 956. The shaft 1184 of the insertion element 1180 may include the W-shaped cross-sectional channel 1182.

The W shape of the channel 1182 may be formed by an elongate protrusion 1186 added to a central portion 1188 of an elongate insertion element 1180 having a C-shaped cross-sectional channel. As such, a C-shaped cross-sectional channel may be modified to produce a W-shaped cross-sectional channel 1182 by the addition of the protrusion 1186. A reduced number of possible contact points between the insertion element 1180 and the analyte sensor 956 may result. For example, the outer surface of the analyte sensor 956 may have a circular-shaped cross-section. As such, reduced friction (e.g., stiction or kinetic friction) may result.

FIG. 66 illustrates a top cross-sectional view showing an insertion element 1190 having a W-shaped cross-sectional channel 1192 for receiving a portion of the analyte sensor 956. The shaft 1194 of the insertion element 1190 may include the W-shaped cross-sectional channel 1192.

The W shape of the channel 1192 may be formed by the shaft 1194 being shaped (e.g., stamped) into a W shape. A reduced number of contact points or surface area between the insertion element 1190 and the analyte sensor 956 may result, and as such, reduced friction (e.g., stiction or kinetic friction) may result.

FIG. 71 illustrates a configuration in which one or more elongate insertion elements 1200 are utilized (elongate insertion elements 1200a and 1200b are marked in FIG. 71). Each of the elongate insertion elements 1200 may include a respective shaft 1202a, b that may be configured to extend along a portion of the elongate analyte sensor 956.

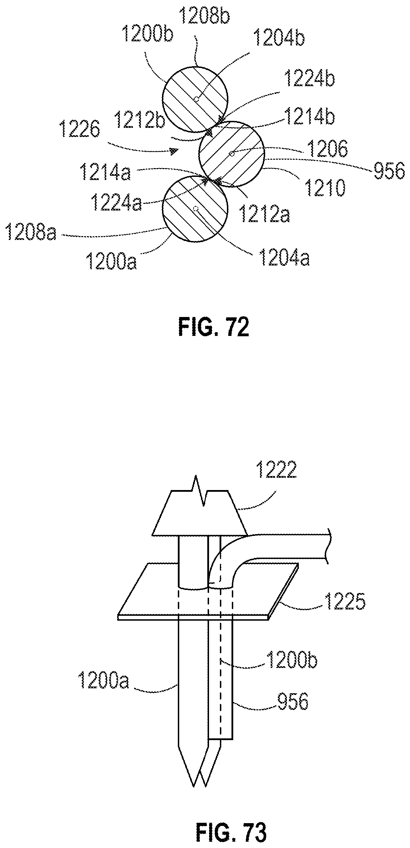

Each of the elongate insertion elements 1200 may be configured to guide the elongate analyte sensor 956 into skin of a host with the elongate analyte sensor 956 positioned external to the shaft 1202a, b of the respective elongate insertion element 1200a, b. As such, the elongate insertion elements 1200a, b may lack a channel that retains the elongate analyte sensor 956 as shown in FIG. 28, for example. Rather, the elongate analyte sensor 956 may be positioned external to the shaft 1202a, b in an arrangement as shown in the cross sectional view of FIG. 72. In examples, the elongate insertion elements 1200 may have a solid center or interior. In examples, the elongate insertion elements 1200 may comprise pins or pin-shaped needles. In examples, the elongate insertion elements 1200 may be hollow or have a channel, yet with the elongate analyte sensor 956 positioned external of the shaft of such elongate insertion elements.

Each of the elongate insertion elements 1200 may extend along a respective central axis 1204a, b. The elongate analyte sensor 956 may include a central axis 1206. The central axis 1206 of the elongate analyte sensor 956 may be configured to be positioned parallel and laterally spaced apart from the respective central axes 1204a, b of the elongate insertion elements 1200 (as shown in FIG. 72 for example). In examples, a second portion 966, sensing portion, or distal portion of the elongate analyte sensor 956 may include the central axis 1206 that extends parallel and laterally spaced apart from the respective central axes 1204a, b of the elongate insertion elements 1200.

In examples, each of the elongate insertion elements 1200 may include a respective outer surface 1208a, b. The outer surfaces 1208a, b of the elongate insertion elements 1200a, b may extend parallel with each other. The elongate analyte sensor 956 may be positioned exterior of the respective outer surfaces 1208a, b. The elongate analyte sensor 956 may include an outer surface 1210.

FIG. 72 illustrates a cross sectional view of the arrangement of FIG. 71 at a view perpendicular to the central axes 1204a, b, 1206. The outer surface 1208a of the elongate insertion element 1200a may include a longitudinally extending segment 1212a that is configured to extend parallel and adjacent to the outer surface 1210 of the elongate analyte sensor 956. The longitudinally extending segment 1212a may comprise only a portion of the entire circumference or outer perimeter of the outer surface 1208a. The longitudinally extending segment 1212a may extend for the entirety of the length of the insertion element 1200a or only a portion of the length in examples. The outer surface 1208b of the elongate insertion element 1200b may include a similarly configured longitudinally extending segment 1212b. The longitudinally extending segments 1212a, b may face towards each other in examples.

The longitudinally extending segments 1212a, b comprising only a portion of the entire circumference or outer perimeter of the respective outer surfaces 1208a, b may produce longitudinally extending contact surfaces 1214a, b for the respective elongate insertion elements 1200a, b. The longitudinally extending contact surfaces 1214a, b may comprise only a portion of the entire circumference or outer perimeter of the respective outer surfaces 1208a, b, thus producing relatively narrow or thin line contact regions between the outer surfaces 1208a, b and the elongate analyte sensor 956. As such, reduced stiction, friction, and contact surface areas between the elongate insertion elements 1200a, b and the elongate analyte sensor 956 may result.

The respective outer surfaces 1208a, b and longitudinally extending segments 1212a, b may comprise a convex outer surface in examples. The convex outer surfaces may bow radially outward and may further reduce the size of the longitudinally extending contact surfaces 1214a, b that may contact the outer surface 1210 of the elongate analyte sensor 956. In examples, other shapes of outer surfaces 1208a, b or longitudinally extending segments 1212a, b may be utilized (e.g., flat, triangular, rectangular, pentagonal, hexagonal, among others).

FIG. 71 illustrates a configuration in which a plurality of the insertion elements 1200a, b may be utilized. Each insertion element 1200a, b may include a respective proximal end portion 1216a, b and a distal end portion 1218a, b. The respective shafts 1202a, b may extend between the proximal end portion 1216a, b and the distal end portion 1218a, b. The distal end portions 1218a, b may comprise respective tips 1220a, b of the insertion elements 1200a, b.

A needle hub 1222 may couple both proximal end portions 1216a, b to each other. The shafts 1202a, b of the respective insertion elements 1200a, b may extend from the proximal end portions 1216a, b to the respective tip 1220a, b. The shafts 1202a, b may be separate from each other along the length of the shafts 1202a, b and unconnected to each other by the material comprising the shafts 1202a, b. The shafts 1202a, b accordingly may comprise independent columns or pillars extending parallel to each other and joined at the needle hub 1222. The shafts 1202a, b may be overmolded at the needle hub 1222. The respective tips 1220a, b may be unconnected to each other. The shafts 1202a, b may comprise free shafts that are free to deflect independent of each other.

The shafts 1202a, b of the respective insertion elements 1200a, b may be positioned to bound sides of the elongate analyte sensor 956. For example, referring to FIG. 72, each shaft 1202a, b may be positioned on a respective opposite side 1224a, b of the elongate analyte sensor 956. The shafts 1202a, b and elongate analyte sensor 956 may be arranged in a triangular configuration, although other configurations may be utilized as desired. The elongate analyte sensor 956 may be positioned between the shafts 1202a, b of the respective insertion elements 1200a, b as shown in FIG. 72, with the central axis 1206 offset laterally from the plane or line extending between the central axes 1204a, b. Other configurations may be utilized as desired (e.g., the central axis 1206 may be aligned with the plane or line extending between the central axes 1204a, b in a collinear arrangement, among other configurations).

The shafts 1202a, b of the respective insertion elements 1200a, b may be configured to support the elongate analyte sensor 956 upon insertion into the skin of a host. The shafts 1202a, b of the respective insertion elements 1200a, b may be configured to support the elongate analyte sensor 956 in a deployment configuration as represented in FIG. 71 for example. The deployment configuration may comprise a configuration that the elongate analyte sensor 956 is positioned in for deployment to the skin of the host. The elongate analyte sensor 956 may be positioned in a deployment configuration immediately prior to insertion into the skin of the host and/or at another time prior to insertion (e.g., at a time of sterilization of the insertion elements 1200a, b and/or the elongate analyte sensor 956 as desired).

The outer surfaces 1208a. b of the shafts 1202a, b may be in contact with the outer surface 1210 of the elongate analyte sensor 956 in the deployment configuration. For example, during a sterilization procedure or prior to insertion, the outer surfaces 1208a, b may be in contact with the outer surface 1210 of the elongate analyte sensor 956 in an arrangement as shown in FIGS. 71 and 72 for example. In examples, the outer surfaces 1208a, b may be spaced from the outer surface 1210 of the elongate analyte sensor 956 in a deployment configuration. In examples, one or more of the insertion elements 1200a, b or the elongate analyte sensor 956 may pass through a septum 1225 that may support the position of the elongate analyte sensor 956 relative to the insertion elements 1200a, b. FIG. 73, for example, illustrates a configuration in which the insertion elements 1200a, b and the elongate analyte sensor 956 pass axially through a septum 1225 that laterally stabilizes the elongate analyte sensor 956 relative to the insertion elements 1200a, b. The septum 1225 may be coupled to a housing for the elongate analyte sensor 956 to extend from, or may have another position (e.g., positioned on the patch for the housing or a liner for the patch as desired). In examples, the use of the septum 1225 may be excluded.

The position of the elongate analyte sensor 956 external to the shafts 1202a, b of the respective insertion elements 1200a, b may provide a variety of benefits. For example, in a sterilization procedure, the reduced contact surface areas between the elongate analyte sensor 956 and the shafts 1202a, b may reduce the strength of any stiction that may be formed between the elongate analyte sensor 956 and the shafts 1202a, b. This may be stiction formed due to swelling of the elongate analyte sensor 956 during sterilization facilitating hydrogen bonding or formed by other causes. Additionally, swelling of an elongate analyte sensor 956 internal to an insertion element (e.g., within a channel as represented in FIG. 28) may produce a large contact surface area between the outer surface of the elongate analyte sensor 956 and the interior surface of an insertion element due to the position of the elongate analyte sensor 956 within the insertion element. This large contact surface area may produce greater friction (both stiction and kinetic friction) between the insertion element and the analyte sensor 956. Positioning the analyte sensor 956 external to the shaft may reduce the surface area and the friction (stiction and kinetic friction). For example, the longitudinally extending segments 1212a, b may comprise a lesser surface area for contact than an interior surface of a channel of an insertion element. The reduced friction may result regardless of whether a sterilization process occurs.

The insertion elements 1200a, b may also provide a lesser penetration profile for insertion into the skin than an insertion element that retains the analyte sensor 956 therein. Reduced wound size and impact may result.

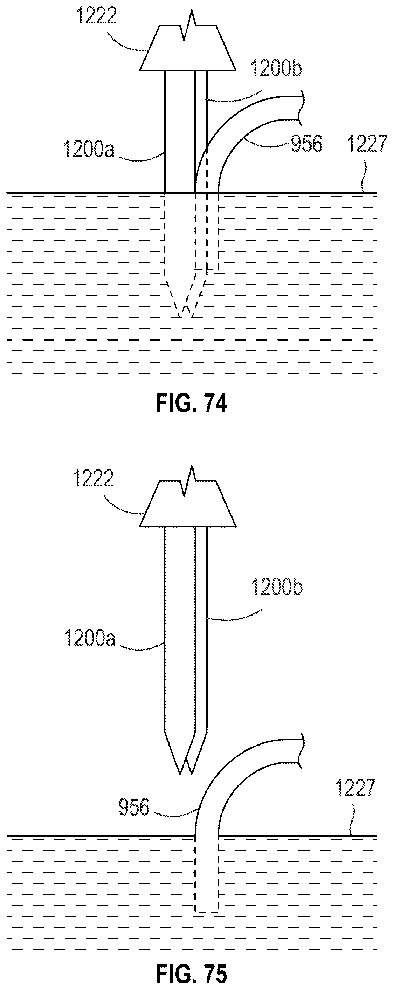

The elongate insertion elements 1200a, b may guide the elongate analyte sensor 956 into the skin of the host in a configuration as shown in FIG. 71. The insertion elements 1200a, b and the elongate analyte sensor 956 together may be inserted distally into the skin. FIG. 74, for example, illustrates the insertion elements 1200a, b and the elongate analyte sensor 956 together penetrating axially into the skin 1227. The insertion elements 1200a, b may retract from the insertion site to leave the elongate analyte sensor 956 inserted into the skin 1227 as represented in FIG. 75 for example. The scales and size of relative components may vary from the representations of FIG. 74 and 75.

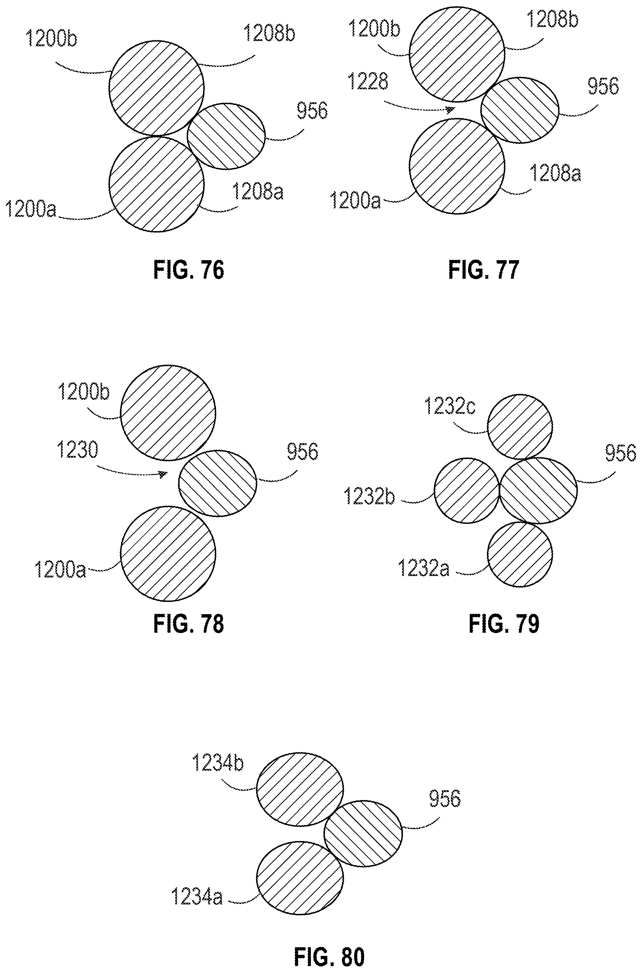

In examples, a space 1226 (marked in FIG. 72) between the elongate insertion elements 1200a, b may comprise a tear region in which the skin 1227 is torn by the insertion elements 1200a, b for the elongate analyte sensor 956 to insert into. The outer surfaces 1208a, b of the shafts 1202a, b may be laterally spaced from each other to produce the space 1226 as desired. In examples, variations may be provided. FIG. 76, for example, illustrates a variation in which the outer surfaces 1208a, b are in contact with each other. The elongate insertion elements 1200a, b may produce a tear that the elongate analyte sensor 956 slides into in examples.

Various other spacings may be utilized. FIG. 77, for example, illustrates an example in which a spacing 1228 smaller than the spacing shown in FIG. 72 may be utilized. FIG. 78 illustrates an example in which a greater spacing 1230 than shown in FIG. 77 may be utilized. The spacing may be set to produce a desired tear region size in examples.

In examples, the number of insertion elements utilized may be varied. One or more insertion elements may be utilized in examples. FIG. 79, for example, illustrates a configuration in which three elongate insertion elements 1232a, b, c may be utilized. The insertion elements 1232a, b, c may be positioned in a triangular arrangement with the elongate analyte sensor 956 bound by the insertion elements 1232a, b, c. In examples, only one insertion element may be utilized. In examples, two or more insertion elements may be utilized.

In examples, the size or diameter of the insertion elements utilized may be varied. The insertion elements 1232a, b, c, for example, may each have a lesser diameter than a respective one of the insertion elements 1200a, b. The insertion elements 1232a, b, c, may each have a lesser diameter than the elongate analyte sensor 956. The insertion elements 1232a, b, c may otherwise be configured similarly as the insertion elements 1200a, b.

In examples, the shape of the insertion elements may be varied. FIG. 80, for example, illustrates a configuration of insertion elements 1234a, b each having an oval cross section. The oval cross section may vary a size or shape of a respective longitudinally extending segment of the respective insertion element 1234a, b that may face the elongate analyte sensor 956. The insertion elements 1234a, b may otherwise be configured similarly as the insertion elements 1200a, b. The insertion elements may have a circular cross section (as shown in FIG. 72) in examples. Other shapes may be utilized in examples. The shape of the elongate sensor 956 may be varied to produce an oval shape as shown in FIGS. 76-80 for example. In examples, a circular shape (as shown in FIG. 72) may be utilized, among other shapes.

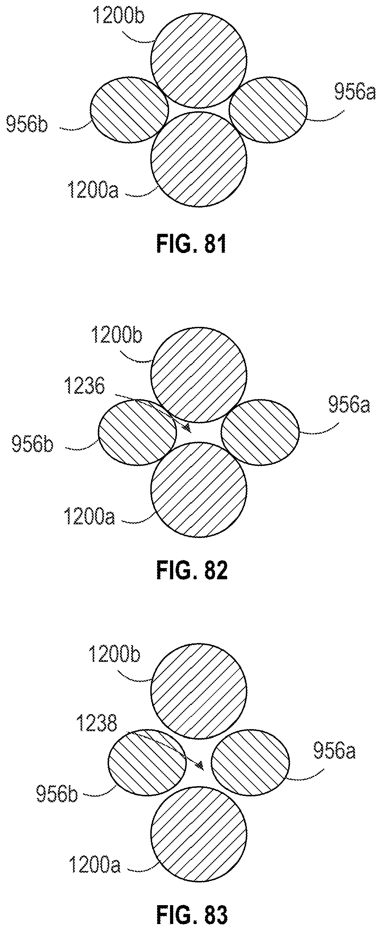

In examples, more than one analyte sensor may be utilized. FIGS. 81-85 illustrate examples in which a plurality of analyte sensors may be utilized. The features of FIGS. 71-80 or any other example herein may be utilized with the examples of FIGS. 81-85.

FIG. 81 illustrates an example in which a second analyte sensor 956b may be utilized in combination with a first analyte sensor 956a. The second analyte sensor 956b may be positioned on an opposite side of the elongate insertion elements 1200a, b than the first analyte sensor 956a. The analyte sensors 956a, b and the elongate insertion elements 1200a, b may be positioned in a diamond configuration. Other configurations (e.g., rectangular or collinear) may be utilized in examples.

The elongate insertion elements 1200a, b may divide and separate the first analyte sensor 956a from the second analyte sensor 956b. As such, during a sterilization process a reduced possibility of the first analyte sensor 956a contacting and having stiction with the second analyte sensor 956b may result. Such a configuration differs from a configuration in which multiple analyte sensors may be inserted into a single channel (with a representative channel shown in FIG. 28). A sterilization process may result in stiction between multiple analyte sensors in a single channel. The elongate insertion elements 1200a, b may divide and separate the first analyte sensor 956a from the second analyte sensor 956b to reduce the possibility of such stiction. The elongate insertion elements 1200a, b may serve to guide the analyte sensors 956a, b into the skin of the host in a similar manner as discussed in regard to FIGS. 71-80.

In examples, a lateral spacing between the elongate insertion elements 1200a, b may be varied to vary the size or shape of the space 1236 between the elongate insertion elements 1200a, b. As represented in FIG. 83, a greater lateral spacing 1238 between the elongate insertion elements 1200a, b than the space 1236 shown in FIG. 82 may reduce the lateral distance between the analyte sensors 956a, b. The size or shape of a space 1236 may be varied as desired.

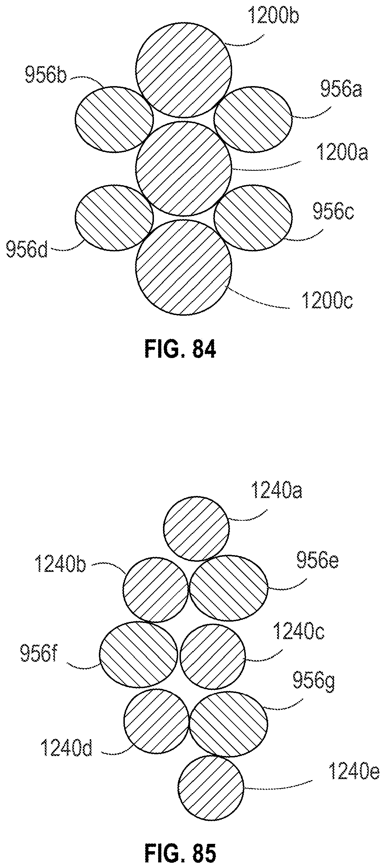

The number of analyte sensors may be varied as desired. FIG. 84, for example, illustrates four analyte sensors 956a, b, c, d being utilized. The elongate insertion element 1200a may comprise a central elongate insertion element 1200a between the elongate insertion elements 1200b, c and the four analyte sensors 956a, b, c, d. The elongate insertion element 1200a may divide and separate the four analyte sensors 956a, b, c, d from each other.

The elongate insertion elements 1200a, b, c may be positioned collinear with each other, with each analyte sensor 956a, b, c, d bound by two of the elongate insertion elements (analyte sensor 956a is bound by elongate insertion elements 1200a, b; analyte sensor 956b is bound by elongate insertion elements 1200a, b on an opposite side than analyte sensor 956a; analyte sensor 956c is bound by elongate insertion elements 1200a, c; and analyte sensor 956d is bound by elongate insertion elements 1200a, c on an opposite side than analyte sensor 956c). The insertion element 1200c may otherwise be configured similarly as the insertion elements 1200a, b, and the analyte sensors 956a, b, c, d may otherwise be configured similarly as the analyte sensor 956.

In examples, a greater or lesser number of analyte sensors and elongate insertion elements may be utilized. In examples, a shape or size of the analyte sensors and elongate insertion elements may be varied. A position of one or more analyte sensors or elongate insertion elements may be varied relative to each other.

FIG. 85, for example, illustrates five elongate insertion elements 1240a, b, c, d, e utilized to divide and separate three analyte sensors 956e, f, g. Each insertion element 1240a, b, c, d, e may have a smaller diameter than each of the analyte sensors 956e, f, g. The insertion elements 1240a, b, c, d, e may be arranged in a staggered orientation. The analyte sensors 956e, f, g may be arranged in a staggered orientation. The analyte sensor 956e may be bound by the insertion elements 1240a, b, c. The analyte sensor 956f may be bound by the insertion elements 1240b, c, d. The analyte sensor 956g may be bound by the insertion elements 1240c, d, e. Various other configurations may be utilized as desired. The insertion elements 1240a, b, c, d, e may otherwise be configured similarly as the insertion elements 1200a, b, and the analyte sensors 956e, f, g may otherwise be configured similarly as the analyte sensor 956.

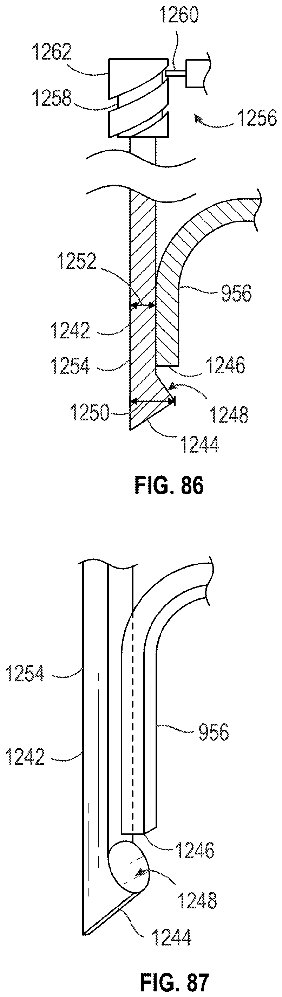

FIG. 86 illustrates an example in which the elongate insertion element 1242 includes a distal tip 1244 configured to extend radially (e.g., in a lateral direction as shown in FIG. 86) over at least a portion of the distal tip 1246 of the elongate analyte sensor 956. FIG. 87 illustrates a perspective view of the distal tip 1244 or a proximal surface 1248 of the distal tip 1244 extending over the distal tip 1246 of the elongate analyte sensor 956.

The distal tip 1244 may protrude radially outward to have a greater diameter 1250 than a diameter 1252 of the shaft 1254 of the elongate insertion element 1242. The distal tip 1244 may extend radially over at least a portion of the distal tip 1246 of the elongate analyte sensor 956 to shield the distal tip 1246 of the elongate analyte sensor 956 upon penetration into the skin of the host. As such, the distal tip 1244 may produce a tear region distal of the elongate analyte sensor 956 that the elongate analyte sensor 956 may insert into.

The elongate insertion element 1242 may be configured to be rotated to displace the distal tip 1244 of the elongate insertion element 1242 from the distal tip 1246 of the elongate analyte sensor 956 upon retraction of the elongate insertion element 1242. For example, a rotation mechanism 1256 may be provided that may rotate the elongate insertion element 1242 and the distal tip 1244 to uncover the portion of the distal tip 1246 of the elongate analyte sensor 956.

The rotation mechanism 1256 may have a variety of configurations in examples. For example, FIG. 86 illustrates a spiral threading 1258 that may be engaged by a protrusion 1260. The spiral threading 1258 may be positioned on the needle hub 1262 or in another location as desired. The protrusion 1260 may be positioned on an applicator system for the on-skin wearable medical device or on another position as desired. Upon retraction of the elongate insertion element 1242, the rotation mechanism 1256 may produce rotation of the distal tip 1244 of the elongate insertion element 1242 to uncover the portion of the distal tip 1246. Other configurations of rotation mechanisms 1256 may be utilized as desired (e.g., gears, cams, levers, electrical actuation, among others).

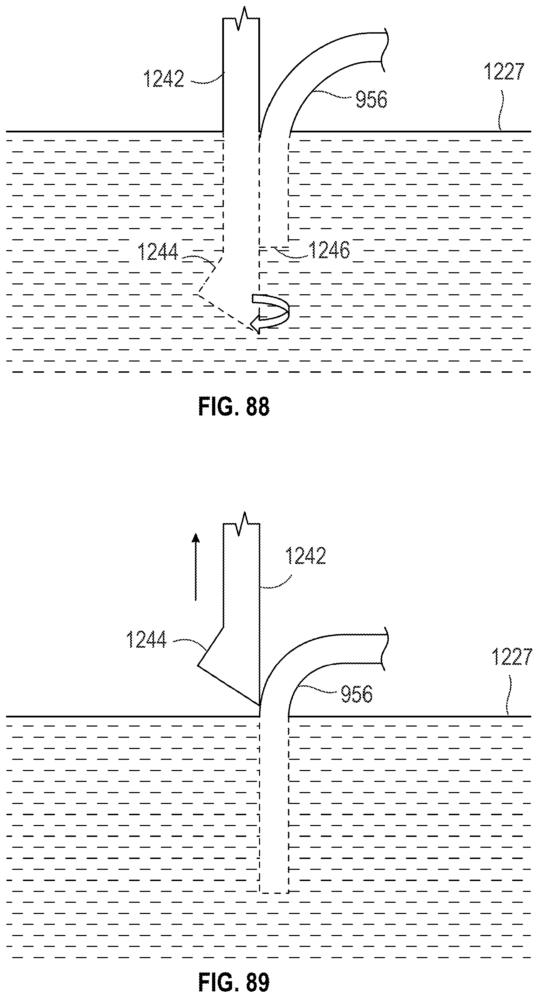

The rotation of the distal tip 1244 may occur within the skin 1227. For example, FIG. 88 illustrates the elongate insertion element 1242 having penetrated the skin 1227 and rotated within the skin 1227. The distal tip 1244 rotates (e.g., by 180 degrees or another amount) to uncover the distal tip 1246 of the elongate analyte sensor 956. The distal tip 1244 and elongate insertion element 1242 accordingly may be retracted from the skin 1227 with the elongate analyte sensor 956 remaining in position.

The elongate insertion element 1242 may otherwise be configured similarly as the insertion element 1200a or any other form of insertion element disclosed herein.

The elongate insertion elements may comprise needles or may have any other form as desired.

Features of examples disclosed herein may be utilized solely or in combination with any other system, apparatus, or method disclosed herein.

The above description presents the best mode contemplated for carrying out the present invention, and of the manner and process of making and using it, in such full, clear, concise, and exact terms as to enable any person skilled in the art to which it pertains to make and use this invention. This invention is, however, susceptible to modifications and alternate constructions from that discussed above that are fully equivalent. Consequently, this invention is not limited to the particular examples disclosed. On the contrary, this invention covers all modifications and alternate constructions coming within the scope of the invention as generally expressed by the following claims, which particularly point out and distinctly claim the subject matter of the invention. While the disclosure has been illustrated and described in detail in the drawings and foregoing description, such illustration and description are to be considered illustrative or exemplary and not restrictive.

To the extent publications and patents or patent applications referenced herein contradict the disclosure contained in the specification, the specification is intended to supersede and/or take precedence over any such contradictory material.

Unless otherwise defined, all terms (including technical and scientific terms) are to be given their ordinary and customary meaning to a person of ordinary skill in the art, and are not to be limited to a special or customized meaning unless expressly so defined herein. It should be noted that the use of particular terminology when describing certain features or aspects of the disclosure should not be taken to imply that the terminology is being re-defined herein to be restricted to include any specific characteristics of the features or aspects of the disclosure with which that terminology is associated. Terms and phrases used in this application, and variations thereof, especially in the appended claims, unless otherwise expressly stated, should be construed as open ended as opposed to limiting. As examples of the foregoing, the term 'including' should be read to mean 'including, without limitation,' 'including but not limited to,' or the like; the term 'comprising' as used herein is synonymous with 'including,' 'containing,' or 'characterized by,' and is inclusive or open-ended and does not exclude additional, unrecited elements or method steps; the term 'having' should be interpreted as 'having at least;' the term 'includes' should be interpreted as 'includes but is not limited to;' the term 'example' is used to provide exemplary instances of the item in discussion, not an exhaustive or limiting list thereof; adjectives such as 'known', 'normal', 'standard', and terms of similar meaning should not be construed as limiting the item described to a given time period or to an item available as of a given time, but instead should be read to encompass known, normal, or standard technologies that may be available or known now or at any time in the future; and use of terms like 'preferably,' 'preferred,' 'desired,' or 'desirable,' and words of similar meaning should not be understood as implying that certain features are critical, essential, or even important to the structure or function of the invention, but instead as merely intended to highlight alternative or additional features that may or may not be utilized in a particular example. Likewise, a group of items linked with the conjunction 'and' should not be read as requiring that each and every one of those items be present in the grouping, but rather should be read as 'and/or' unless expressly stated otherwise. Similarly, a group of items linked with the conjunction 'or' should not be read as requiring mutual exclusivity among that group, but rather should be read as 'and/or' unless expressly stated otherwise.

Where a range of values is provided, it is understood that the upper and lower limit, and each intervening value between the upper and lower limit of the range is encompassed within the examples.

With respect to the use of substantially any plural and/or singular terms herein, those having skill in the art can translate from the plural to the singular and/or from the singular to the plural as is appropriate to the context and/or application. The various singular/plural permutations may be expressly set forth herein for sake of clarity. The indefinite article 'a' or 'an' does not exclude a plurality. A single processor or other unit may fulfill the functions of several items recited in the claims. The mere fact that certain measures are recited in mutually different dependent claims does not indicate that a combination of these measures cannot be used to advantage. Any reference signs in the claims should not be construed as limiting the scope.

It will be further understood by those within the art that if a specific number of an introduced claim recitation is intended, such an intent will be explicitly recited in the claim, and in the absence of such recitation no such intent is present. For example, as an aid to understanding, the following appended claims may contain usage of the introductory phrases 'at least one' and 'one or more' to introduce claim recitations. However, the use of such phrases should not be construed to imply that the introduction of a claim recitation by the indefinite articles 'a' or 'an' limits any particular claim containing such introduced claim recitation to examples containing only one such recitation, even when the same claim includes the introductory phrases 'one or more' or 'at least one' and indefinite articles such as 'a' or 'an' (e.g., 'a' and/or 'an' should typically be interpreted to mean 'at least one' or 'one or more'); the same holds true for the use of definite articles used to introduce claim recitations. In addition, even if a specific number of an introduced claim recitation is explicitly recited, those skilled in the art will recognize that such recitation should typically be interpreted to mean at least the recited number (e.g., the bare recitation of 'two recitations,' without other modifiers, typically means at least two recitations, or two or more recitations). Furthermore, in those instances where a convention analogous to 'at least one of A, B, and C, etc.' is used, in general such a construction is intended in the sense one having skill in the art would understand the convention (e.g., 'a system having at least one of A, B, and C' would include but not be limited to systems that have A alone, B alone, C alone, A and B together, A and C together, B and C together, and/or A, B, and C together, etc.). In those instances where a convention analogous to 'at least one of A, B, or C, etc.' is used, in general such a construction is intended in the sense one having skill in the art would understand the convention (e.g., 'a system having at least one of A, B, or C' would include but not be limited to systems that have A alone, B alone, C alone, A and B together, A and C together, B and C together, and/or A, B, and C together, etc.). It will be further understood by those within the art that virtually any disjunctive word and/or phrase presenting two or more alternative terms, whether in the description, claims, or drawings, should be understood to contemplate the possibilities of including one of the terms, either of the terms, or both terms. For example, the phrase 'A or B' will be understood to include the possibilities of 'A' or 'B' or 'A and B.'

All numbers expressing quantities of ingredients, reaction conditions, and so forth used in the specification are to be understood as being modified in all instances by the term 'about.' Accordingly, unless indicated to the contrary, the numerical parameters set forth herein are approximations that may vary depending upon the desired properties sought to be obtained. At the very least, and not as an attempt to limit the application of the doctrine of equivalents to the scope of any claims in any application claiming priority to the present application, each numerical parameter should be construed in light of the number of significant digits and ordinary rounding approaches.

Furthermore, although the foregoing has been described in some detail by way of illustrations and examples for purposes of clarity and understanding, it is apparent to those skilled in the art that certain changes and modifications may be practiced. Therefore, the description and examples should not be construed as limiting the scope of the invention to the specific examples described herein, but rather to also cover all modification and alternatives coming with the scope of the invention as defined by the claims.

## Claims

1. A medical device system comprising:
a housing (962) configured to be worn on skin of a host and including a distal surface (968) for facing towards the skin and a proximal surface (980) facing opposite the distal surface (968);
an elongate analyte sensor (956) coupled to the housing (962) and configured to extend distally from the housing (962) and be positioned in the skin of the host; and
an elongate insertion element (915) including a shaft (986) configured to extend along a portion of the elongate analyte sensor (956) and be inserted into the skin to guide the elongate analyte sensor (956) into the skin of the host, **characterised in** the shaft (986) including a surface (992, 1152, 1154, 1156, 1159, 1165, 1171, 1178) configured to reduce friction with the portion of the elongate analyte sensor,
wherein the surface includes a coating configured to reduce friction with the portion of the elongate analyte sensor (956).

2. The medical device system of claim 1, wherein the surface (1152) is configured to reduce stiction with the portion of the elongate analyte sensor (956).

3. The medical device system of claim 1 or claim 2, wherein the surface (1159) includes a surface texture.

4. The medical device system of any of claims 1-3, wherein the surface (1156, 1165) includes a surface roughness of 35 root mean square, RMS, microinches or greater.

5. The medical device system of any of claims 1-4, wherein the surface (1154) includes one or more of bumps, holes, or grooves.

6. The medical device system of any one of the preceding claims, wherein the coating comprises a lubricant, and/or wherein the coating comprises one or more of a plating, a dip coating, or a deposition.

7. The medical device system of any of the preceding claims, wherein the coating comprises a polymer, and/or wherein the coating comprises a thermal oxide, and/or wherein the coating comprises an inert material.

8. The medical device system of any of the preceding claims, wherein the coating is bonded to the shaft (986).

9. The medical device system of any of the preceding claims, wherein the coating has a thickness upon the shaft (986) of less than 1.5 micrometers.

10. The medical device system of any of the preceding claims, wherein the coating is cured.

11. The medical device system of any of the preceding claims, wherein the coating includes silicone, optionally, wherein the silicone comprises an aminofunctional dimethylsiloxane copolymer.

12. The medical device system of any of the preceding claims, wherein the surface (992, 1152) is configured to reduce hydrogen bonding with the portion of the elongate analyte sensor (956).

13. The medical device system of any of the preceding claims, wherein the elongate insertion element (915) comprises a needle.

14. The medical device system of any of the preceding claims, wherein the elongate insertion element (915) includes a channel (958, 1155, 1157, 1163, 1167, 1172, 1182, 1192) for receiving the portion of the elongate analyte sensor (956), optionally, wherein the channel (958, 1155, 1157, 1163, 1167) has a C-shaped cross-section.

## Patentansprüche

1. Medizinisches Vorrichtungssystem, umfassend:
ein Gehäuse (962), das so ausgebildet ist, dass es auf der Haut eines Wirts getragen werden kann, und eine distale Oberfläche (968), die zur Haut hin ausgerichtet ist, und eine proximale Oberfläche (980) einschließt, die der distalen Oberfläche (968) gegenüberliegt;
einen länglichen Analytsensor (956), der mit dem Gehäuse (962) gekoppelt und so ausgebildet ist, dass er sich distal vom Gehäuse (962) aus erstreckt und in der Haut des Wirts positioniert werden kann; und
ein längliches Einführelement (915), das einen Schaft (986) einschließt, der so ausgebildet ist, dass er sich entlang eines Abschnitts des länglichen Analytsensors (956) erstreckt und in die Haut eingeführt werden kann, um den länglichen Analytsensor (956) in die Haut des Wirts zu führen, **gekennzeichnet dadurch,**
**dass** der Schaft (986) eine Oberfläche (992, 1152, 1154, 1156, 1159, 1165, 1171, 1178) einschließt, die so ausgebildet ist, dass sie die Reibung mit dem Abschnitt des länglichen Analytsensors reduziert,
wobei die Oberfläche eine Beschichtung einschließt, die so ausgebildet ist, dass sie die Reibung mit dem Abschnitt des länglichen Analytsensors (956) reduziert.

2. Medizinisches Vorrichtungssystem nach Anspruch 1, wobei die Oberfläche (1152) so ausgebildet ist, dass sie die Haftreibung mit dem Abschnitt des länglichen Analytsensors (956) reduziert.

3. Medizinisches Vorrichtungssystem nach Anspruch 1 oder Anspruch 2, wobei die Oberfläche (1159) eine Oberflächenstruktur einschließt.

4. Medizinisches Vorrichtungssystem nach einem der Ansprüche 1-3, wobei die Oberfläche (1156, 1165) eine Oberflächenrauheit von 35 Mikrozoll im quadratischen Mittel, RMS, oder mehr einschließt.

5. Medizinisches Vorrichtungssystem nach einem der Ansprüche 1-4, wobei die Oberfläche (1154) eine oder mehrere Erhebungen, Löcher oder Rillen einschließt.

6. Medizinisches Vorrichtungssystem nach einem der vorstehenden Ansprüche, wobei die Beschichtung ein Schmiermittel umfasst und/oder wobei die Beschichtung eine oder mehrere von einer Plattierung, einer Tauchbeschichtung oder einer Abscheidung umfasst.

7. Medizinisches Vorrichtungssystem nach einem der vorstehenden Ansprüche, wobei die Beschichtung ein Polymer umfasst und/oder wobei die Beschichtung ein thermisches Oxid umfasst und/oder wobei die Beschichtung ein inertes Material umfasst.

8. Medizinisches Vorrichtungssystem nach einem der vorstehenden Ansprüche, wobei die Beschichtung an den Schaft (986) gebunden ist.

9. Medizinisches Vorrichtungssystem nach einem der vorstehenden Ansprüche, wobei die Beschichtung auf dem Schaft (986) eine Dicke von weniger als 1,5 Mikrometern aufweist.

10. Medizinisches Vorrichtungssystem nach einem der vorstehenden Ansprüche, wobei die Beschichtung ausgehärtet ist.

11. Medizinisches Vorrichtungssystem nach einem der vorstehenden Ansprüche, wobei die Beschichtung Silikon einschließt, wobei das Silikon wahlweise ein aminofunktionelles Dimethylsiloxan-Copolymer umfasst.

12. Medizinisches Vorrichtungssystem nach einem der vorstehenden Ansprüche, wobei die Oberfläche (992, 1152) so ausgebildet ist, dass sie die Wasserstoffbrückenbindung mit dem Abschnitt des länglichen Analytsensors (956) reduziert.

13. Medizinisches Vorrichtungssystem nach einem der vorstehenden Ansprüche, wobei das längliche Einführelement (915) eine Nadel umfasst.

14. Medizinisches Vorrichtungssystem nach einem der vorstehenden Ansprüche, wobei das längliche Einführelement (915) einen Kanal (958, 1155, 1157, 1163, 1167, 1172, 1182, 1192) zum Aufnehmen des Abschnitts des länglichen Analytsensors (956) einschließt, wobei der Kanal (958, 1155, 1157, 1163, 1167) wahlweise einen c-förmigen Querschnitt aufweist.

## Revendications

1. Système de dispositif médical comprenant :
un boîtier (962) configuré pour être porté sur la peau d'un hôte et incluant une surface distale (968) à orienter vers la peau et une surface proximale (980) orientée en regard de la surface distale (968) ;
un capteur (956) d'analyte allongé couplé au boîtier (962) et configuré pour s'étendre distalement à partir du boîtier (962) et être positionné dans la peau de l'hôte ; et
un élément d'insertion allongé (915) incluant un arbre (986) configuré pour s'étendre le long d'une portion du capteur (956) d'analyte allongé et être inséré dans la peau pour guider le capteur (956) d'analyte allongé dans la peau de l'hôte,
**caractérisé en ce que** l'arbre (986) inclut une surface (992, 1152, 1154, 1156, 1159, 1165, 1171, 1178) configurée pour réduire la friction avec la portion du capteur d'analyte allongé,
dans lequel la surface inclut un revêtement configuré pour réduire la friction avec la portion du capteur (956) d'analyte allongé.

2. Système de dispositif médical selon la revendication 1, dans lequel la surface (1152) est configurée pour réduire la friction avec la portion du capteur (956) d'analyte allongé.

3. Système de dispositif médical selon la revendication 1 ou la revendication 2, dans lequel la surface (1159) inclut une texture de surface.

4. Système de dispositif médical selon l'une quelconque des revendications 1 à 3, dans lequel la surface (1156, 1165) inclut une rugosité de surface moyenne quadratique, RMS, de 35 micropouces ou plus.

5. Système de dispositif médical selon l'une quelconque des revendications 1 à 4, dans lequel la surface (1154) inclut un(e) ou plusieurs bosses, trous ou rainures.

6. Système de dispositif médical selon l'une quelconque des revendications précédentes, dans lequel le revêtement comprend un lubrifiant, et/ou dans lequel le revêtement comprend un ou plusieurs d'un placage, d'un revêtement par immersion ou d'un dépôt.

7. Système de dispositif médical selon l'une quelconque des revendications précédentes, dans lequel le revêtement comprend un polymère, et/ou dans lequel le revêtement comprend un oxyde thermique, et/ou dans lequel le revêtement comprend un matériau inerte.

8. Système de dispositif médical selon l'une quelconque des revendications précédentes, dans lequel le revêtement est lié à l'arbre (986).

9. Système de dispositif médical selon l'une quelconque des revendications précédentes, dans lequel le revêtement présente une épaisseur sur l'arbre (986) inférieure à 1,5 micromètres.

10. Système de dispositif médical selon l'une quelconque des revendications précédentes, dans lequel le revêtement est durci.

11. Système de dispositif médical selon l'une quelconque des revendications précédentes, dans lequel le revêtement inclut du silicone, facultativement, dans lequel le silicone comprend un copolymère de diméthylsiloxane aminofonctionnel.

12. Système de dispositif médical selon l'une quelconque des revendications précédentes, dans lequel la surface (992, 1152) est configurée pour réduire la liaison hydrogène avec la portion du capteur (956) d'analyte allongé.

13. Système de dispositif médical selon l'une quelconque des revendications précédentes, dans lequel l'élément d'insertion allongé (915) comprend une aiguille.

14. Système de dispositif médical selon l'une quelconque des revendications précédentes, dans lequel l'élément d'insertion allongé (915) inclut un canal (958, 1155, 1157, 1163, 1167, 1172, 1182, 1192) pour recevoir la portion du capteur (956) d'analyte allongé, facultativement, dans lequel le canal (958, 1155, 1157, 1163, 1167) présente une section transversale en forme de C.
